# EUROPEAN PATENT SPECIFICATION

(11) **EP 2 363 472 B1**
(45) Date of publication and mention of the grant of the patent: **10.12.2014**
(21) Application number: 09823696.1
(22) Date of filing: 30.10.2009
(51) Int. Cl.: C12N 15/09, A61K 35/76, A61K 48/00, A61P 43/00, C12N 1/15, C12N 1/19, C12N 1/21, C12N 5/10, C12P 21/02, C12N 15/62, C12N 15/67

(54) **METHOD FOR ENHANCING EXPRESSION OF RECOMBINANT PROTEIN**
VERFAHREN ZUR VERBESSERUNG DER EXPRESSION REKOMBINANTEN PROTEINS
PROCÉDÉ POUR L'AMPLIFICATION DE L'EXPRESSION D'UNE PROTÉINE RECOMBINÉE

(30) Priority: 31.10.2008 JP 2008282151
(43) Date of publication of application: 07.09.2011
(73) Proprietor: Dnavec Corporation, Ibaraki 3002611 (JP)
(72) Inventor: INOUE, Makoto, Tsukuba-shi Ibaraki 3002611 (JP); SAEKI, Koichi, Tsukuba-shi Ibaraki 3002611 (JP); YOU, Jun, Tsukuba-shi Ibaraki 3002611 (JP); TABATA, Toshiaki, Tsukuba-shi Ibaraki 3002611 (JP); IWASAKI, Hitoshi, Tsukuba-shi Ibaraki 3002611 (JP); SHU, Tsugumine, Tsukuba-shi Ibaraki 3002611 (JP); HASEGAWA, Mamoru, Tsukuba-shi Ibaraki 3002611 (JP)
(74) Representative: Vossius & Partner
(86) International application number: PCT/JP2009/068682
(87) International publication number: WO 2010/050586

(56) References cited:
- WO-A1-01/72340
- NEMCHINOV L G ET AL: "Development of a plant-derived subunit vaccine candidate against hepatitis C virus", 1 January 2000 (2000-01-01), ARCHIVES OF VIROLOGY, SPRINGER-VERLAG, PAGE(S) 2557 - 2573, XP002473291, ISSN: 0304-8608 * page 2558, paragraph 4 - page 2559, paragraph 1 * * page 2562, paragraph 2 - page 2563, column 2 * * page 2569, paragraph 4 *
- GONG Z. ET AL.: 'Oral administration of a cholera toxin B subunit-insulin fusion protein produced in silkworm protects against autoimmune diabetes' J. BIOTECHNOL. vol. 119, 2005, pages 93 - 105, XP027663346
- GONG Z. ET AL.: 'Incorporation of partial polyhedrin homology sequences (PPHS) enhances the production of cloned foreign genes in a baculovirus expression system' BIOTECHNOL. APPL. BIOCHEM. vol. 43, 2006, pages 165 - 170, XP008165301
- HE D. M. ET AL.: 'Stable expression of foot-and- mouth disease virus protein VP1 fused with cholera toxin B subunit in the potato (Solanum tuberosum)' COLLOIDS SURF. B: BIOINTERFACES vol. 55, 2007, pages 159 - 163, XP005895528

### SEQUENCE LISTING

<110> DNAVEC CORPORATION
<120> Methods for enhancing expression of recombinant proteins
<130> D4-A0803P
<150> JP 2008-282151
   <151> 2008-10-31
<160> 122
<170> PatentIn version 3.4
<210> 1
   <211> 530
   <212> DNA
   <213> Artificial
<220>
   <223> an artificially synthesized sequence
<220>
   <221> CDS
   <222> (18)..(203)
<400> 1
   gcggccgcca aggttca atg agc gtg ccc acc cag gtg ctg ggc ctg ctg        50
                      Met Ser Val Pro Thr Gln Val Leu Gly Leu Leu
                      1               5                   10
   ctg ctg tgg ctg acc gac gcc aga tgc gac gcc gag ttc aga cac gac        98
   Leu Leu Trp Leu Thr Asp Ala Arg Cys Asp Ala Glu Phe Arg His Asp
               15                  20                  25
   agc ggc tac gag gtg cac cac cag aag ctg gtg ttc ttc gcc gag gac       146
   Ser Gly Tyr Glu Val His His Gln Lys Leu Val Phe Phe Ala Glu Asp
           30                  35                  40
   gtg ggc agc aac aag ggc gcc atc atc ggc ctg atg gtg ggc ggc gtg       194
   Val Gly Ser Asn Lys Gly Ala Ile Ile Gly Leu Met Val Gly Gly Val
       45                  50                  55
   gtg atc gcc taagaattca cgcgtggtac ctctagagtc gacccgggcc               243
   Val Ile Ala
   60
   cgcttcgagc agacatgata agatacattg atgagtttgg acaaaccaca actagaatgc     303
   agtgaaaaaa atgctttatt tgtgaaattt gtgatgctat tgctttattt gtaaccatta     363
   taagctgcaa taaacaagtt aacaacaaca attgcattca ttttatgttt caggttcagg     423
   gggagatgtg ggaggttttt taaagcaagt aaaacctcta caaatgtggt aaaatcgata     483
   aggatccacc gtagtaagaa aaacttaggg tgaaagttcg cggccgc                   530
<210> 2
   <211> 62
   <212> PRT
   <213> Artificial
<220>
   <223> Synthetic Construct
<400> 2
   Met Ser Val Pro Thr Gln Val Leu Gly Leu Leu Leu Leu Trp Leu Thr
   1               5                   10                  15
   Asp Ala Arg Cys Asp Ala Glu Phe Arg His Asp Ser Gly Tyr Glu Val
               20                  25                  30
   His His Gln Lys Leu Val Phe Phe Ala Glu Asp Val Gly Ser Asn Lys
           35                  40                  45
   Gly Ala Ile Ile Gly Leu Met Val Gly Gly Val Val Ile Ala
       50                  55                  60
<210> 3
   <211> 228
   <212> DNA
   <213> Artificial
<220>
   <223> an artificially synthesized sequence
<400> 3
   atatctcgag gcggccgctg gctagccacc atgagcgtgc ccacccaggt gctgggcctg 60
   ctgctgctgt ggctgaccga cgccaggtgc tcggaggagc cgcccatctc tctggatctc 120
   accttccacc ttctgcggga agtcttggaa atggcccggg cagagcagtt agctcagcaa 180
   gctcacagca acaggaaact gatggagatt atcgggtaag atatccag 228
<210> 4
   <211> 80
   <212> DNA
   <213> Artificial
<220>
   <223> an artificially synthesized sequence
<400> 4
   aactgctctg cccgggccat ttccaagact tcccgcagaa ggtggaaggt gagatccaga 60
   gagatgggcg gctcctccga 80
<210> 5
   <211> 78
   <212> DNA
   <213> Artificial
<220>
   <223> an artificially synthesized sequence
<400> 5
   ctggatatct tacccgataa tctccatcag tttcctgttg ctgtgagctt gctgagctaa 60
   ctgctctgcc cgggccat 78
<210> 6
   <211> 18
   <212> DNA
   <213> Artificial
<220>
   <223> an artificially synthesized sequence
<400> 6
   ctggatatct tacccgat 18
<210> 7
   <211> 40
   <212> DNA
   <213> Artificial
<220>
   <223> an artificially synthesized sequence
<400> 7
   atatctcgag gcggccgctg gctagccacc atgagcgtgc 40
<210> 8
   <211> 36
   <212> DNA
   <213> Artificial
<220>
   <223> an artificially synthesized sequence
<400> 8
   agatgggcgg ctcctccgag cacctggcgt cggtca 36
<210> 9
   <211> 280
   <212> DNA
   <213> Artificial
<220>
   <223> an artificially synthesized sequence
<400> 9
   taagatatcc agacatgata agatacattg atgagtttgg acaaaccaca actagaatgc 60
   agtgaaaaaa atgctttatt tgtgaaattt gtgatgctat tgctttattt gtaaccatta 120
   taagctgcaa taaacaagtt aacaacaaca attgcattca ttttatgttt caggttcagg 180
   gggagatgtg ggaggttttt taaagcaagt aaaacctcta caaatgtggt agatccgtag 240
   taagaaaaac ttagggtgaa agttcatcgc ggccgcatat 280
<210> 10
   <211> 25
   <212> DNA
   <213> Artificial
<220>
   <223> an artificially synthesized sequence
<400> 10
   taagatatcc agacatgata agata 25
<210> 11
   <211> 22
   <212> DNA
   <213> Artificial
<220>
   <223> an artificially synthesized sequence
<400> 11
   atatgcggcc gcgatgaact tt 22
<210> 12
   <211> 482
   <212> DNA
   <213> Artificial
<220>
   <223> an artificially synthesized sequence
<220>
   <221> CDS
   <222> (21)..(206)
<400> 12
   gcggccgctg gctagccacc atg agc gtg ccc acc cag gtg ctg ggc ctg ctg      53
                         Met Ser Val Pro Thr Gln Val Leu Gly Leu Leu
                         1               5                   10
   ctg ctg tgg ctg acc gac gcc agg tgc tcg gag gag ccg ccc atc tct       101
   Leu Leu Trp Leu Thr Asp Ala Arg Cys Ser Glu Glu Pro Pro Ile Ser
           15                  20                  25
   ctg gat ctc acc ttc cac ctt ctg cgg gaa gtc ttg gaa atg gcc cgg       149
   Leu Asp Leu Thr Phe His Leu Leu Arg Glu Val Leu Glu Met Ala Arg
       30                  35                  40
   gca gag cag tta gct cag caa gct cac agc aac agg aaa ctg atg gag       197
   Ala Glu Gln Leu Ala Gln Gln Ala His Ser Asn Arg Lys Leu Met Glu
   45                  50                  55
   att atc ggg taagatatcc agacatgata agatacattg atgagtttgg               246
   Ile Ile Gly
   60
   acaaaccaca actagaatgc agtgaaaaaa atgctttatt tgtgaaattt gtgatgctat     306
   tgctttattt gtaaccatta taagctgcaa taaacaagtt aacaacaaca attgcattca     366
   ttttatgttt caggttcagg gggagatgtg ggaggttttt taaagcaagt aaaacctcta     426
   caaatgtggt agatccgtag taagaaaaac ttagggtgaa agttcatcgc ggccgc         482
<210> 13
   <211> 62
   <212> PRT
   <213> Artificial
<220>
   <223> Synthetic Construct
<400> 13
   Met Ser Val Pro Thr Gln Val Leu Gly Leu Leu Leu Leu Trp Leu Thr
   1               5                   10                  15
   Asp Ala Arg Cys Ser Glu Glu Pro Pro Ile Ser Leu Asp Leu Thr Phe
               20                  25                  30
   His Leu Leu Arg Glu Val Leu Glu Met Ala Arg Ala Glu Gln Leu Ala
           35                  40                  45
   Gln Gln Ala His Ser Asn Arg Lys Leu Met Glu Ile Ile Gly
       50                  55                  60
<210> 14
   <211> 800
   <212> DNA
   <213> Artificial
<220>
   <223> an artificially synthesized sequence
<220>
   <221> CDS
   <222> (21)..(530)
<400> 14
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc       101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac       149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc       197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc       245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac      293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc      341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc      389
   Val Trp Asn Asn Lys Thr Pro His Ala Ile Ala Ala Ile Ser Met Ala
           110                 115                 120
   aac ggc ccc ggc ccc gac gcc gag ttc aga cac gac agc ggc tac gag      437
   Asn Gly Pro Gly Pro Asp Ala Glu Phe Arg His Asp Ser Gly Tyr Glu
       125                 130                 135
   gtg cac cac cag aag ctg gtg ttc ttc gcc gag gac gtg ggc agc aac      485
   Val His His Gln Lys Leu Val Phe Phe Ala Glu Asp Val Gly Ser Asn
   140                 145                 150                 155
   aag ggc gcc atc atc ggc ctg atg gtg ggc ggc gtg gtg atc gcc          530
   Lys Gly Ala Ile Ile Gly Leu Met Val Gly Gly Val Val Ile Ala
                   160                 165                 170
   taacagacat gataagatac attgatgagt ttggacaaac cacaactaga atgcagtgaa    590
   aaaaatgctt tatttgtgaa atttgtgatg ctattgcttt atttgtaacc attataagct    650
   gcaataaaca agttaacaac aacaattgca ttcattttat gtttcaggtt cagggggaga   710
   tgtgggaggt tttttaaagc aagtaaaacc tctacaaatg tggtagatcc gtagtaagaa    770
   aaacttaggg tgaaagttca tcgcggccgc                                     800
<210> 15
   <211> 170
   <212> PRT
   <213> Artificial
<220>
   <223> Synthetic Construct
<400> 15
   Met Ile Lys Leu Lys Phe Gly Val Phe Phe Thr Val Leu Leu Ser Ser
   1 5 10 15
   Ala Tyr Ala His Gly Thr Pro Gln Asn Ile Thr Asp Leu Cys Ala Glu
   20 25 30
   Tyr His Asn Thr Gln Ile His Thr Leu Asn Asp Lys Ile Phe Ser Tyr
   35 40 45
   Thr Glu Ser Leu Ala Gly Lys Arg Glu Met Ala Ile Ile Thr Phe Lys
   50 55 60
   Asn Gly Ala Thr Phe Gln Val Glu Val Pro Gly Ser Gln His Ile Asp
   65 70 75 80
   Ser Gln Lys Lys Ala Ile Glu Arg Met Lys Asp Thr Leu Arg Ile Ala
   85 90 95
   Tyr Leu Thr Glu Ala Lys Val Glu Lys Leu Cys Val Trp Asn Asn Lys
   100 105 110
   Thr Pro His Ala Ile Ala Ala Ile Ser Met Ala Asn Gly Pro Gly Pro
   115 120 125
   Asp Ala Glu Phe Arg His Asp Ser Gly Tyr Glu Val His His Gln Lys
   130 135 140
   Leu Val Phe Phe Ala Glu Asp Val Gly Ser Asn Lys Gly Ala Ile Ile
   145 150 155 160
   Gly Leu Met Val Gly Gly Val Val Ile Ala
   165 170
<210> 16
   <211> 552
   <212> DNA
   <213> Artificial
<220>
   <223> an artificially synthesized sequence
<400> 16
   tatactcgag gcggccgctg gctagccacc atgatcaagc tgaagttcgg cgtgttcttc 60
   accgtgctgc tgagcagcgc ctacgcccac ggcacccccc agaacatcac cgacctgtgc 120
   gccgagtacc acaacaccca gatccacacc ctgaacgaca agatcttcag ctacaccgag 180
   agcctggccg gcaagagaga gatggccatc atcaccttca agaacggcgc caccttccag 240
   gtggaggtgc ccggctccca gcacatcgac agccagaaga aggccatcga gagaatgaag 300
   gacaccctgc ggatcgccta cctgaccgag gccaaggtgg agaagctgtg cgtgtggaac 360
   aacaagaccc cccacgccat cgccgccatc agcatggcca acggccccgg cccctcggag 420
   gagccgccca tctctctgga tctcaccttc caccttctgc gggaagtctt ggaaatggcc 480
   cgggcagagc agttagctca gcaagctcac agcaacagga aactgatgga gattatcggg 540
   taagatatcc ag 552
<210> 17
   <211> 35
   <212> DNA
   <213> Artificial
<220>
   <223> an artificially synthesized sequence
<400> 17
   tatactcgag gcggccgctg gctagccacc atgat 35
<210> 18
   <211> 50
   <212> DNA
   <213> Artificial
<220>
   <223> an artificially synthesized sequence
<400> 18
   tccagagaga tgggcggctc ctccgagggg ccggggccgt tggccatgct 50
<210> 19
   <211> 806
   <212> DNA
   <213> Artificial
<220>
   <223> an artificially synthesized sequence
<220>
   <221> CDS
   <222> (21)..(530)
<400> 19
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc      101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac      149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc      197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc      245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac      293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc      341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc      389
   Val Trp Asn Asn Lys Thr Pro His Ala Ile Ala Ala Ile Ser Met Ala
           110                 115                 120
   aac ggc ccc ggc ccc tcg gag gag ccg ccc atc tct ctg gat ctc acc      437
   Asn Gly Pro Gly Pro Ser Glu Glu Pro Pro Ile Ser Leu Asp Leu Thr
       125                 130                 135
   ttc cac ctt ctg cgg gaa gtc ttg gaa atg gcc cgg gca gag cag tta      485
   Phe His Leu Leu Arg Glu Val Leu Glu Met Ala Arg Ala Glu Gln Leu
   140                 145                 150                 155
   gct cag caa gct cac agc aac agg aaa ctg atg gag att atc ggg          530
   Ala Gln Gln Ala His Ser Asn Arg Lys Leu Met Glu Ile Ile Gly
                   160                 165                 170
   taagatatcc agacatgata agatacattg atgagtttgg acaaaccaca actagaatgc    590
   agtgaaaaaa atgctttatt tgtgaaattt gtgatgctat tgctttattt gtaaccatta    650
   taagctgcaa taaacaagtt aacaacaaca attgcattca ttttatgttt caggttcagg    710
   gggagatgtg ggaggttttt taaagcaagt aaaacctcta caaatgtggt agatccgtag    770
   taagaaaaac ttagggtgaa agttcatcgc ggccgc                              806
<210> 20
   <211> 170
   <212> PRT
   <213> Artificial
<220>
   <223> Synthetic Construct
<400> 20
   Met Ile Lys Leu Lys Phe Gly Val Phe Phe Thr Val Leu Leu Ser Ser
   1               5                   10                  15
   Ala Tyr Ala His Gly Thr Pro Gln Asn Ile Thr Asp Leu Cys Ala Glu
               20                  25                  30
   Tyr His Asn Thr Gln Ile His Thr Leu Asn Asp Lys Ile Phe Ser Tyr
           35                  40                  45
   Thr Glu Ser Leu Ala Gly Lys Arg Glu Met Ala Ile Ile Thr Phe Lys
       50                  55                  60
   Asn Gly Ala Thr Phe Gln Val Glu Val Pro Gly Ser Gln His Ile Asp
   65                  70                  75                  80
   Ser Gln Lys Lys Ala Ile Glu Arg Met Lys Asp Thr Leu Arg Ile Ala
                   85                  90                  95
   Tyr Leu Thr Glu Ala Lys Val Glu Lys Leu Cys Val Trp Asn Asn Lys
               100                 105                 110
   Thr Pro His Ala Ile Ala Ala Ile Ser Met Ala Asn Gly Pro Gly Pro
           115                 120                 125
   Ser Glu Glu Pro Pro Ile Ser Leu Asp Leu Thr Phe His Leu Leu Arg
       130                 135                 140
   Glu Val Leu Glu Met Ala Arg Ala Glu Gln Leu Ala Gln Gln Ala His
   145                 150                 155                 160
   Ser Asn Arg Lys Leu Met Glu Ile Ile Gly
                   165                 170
<210> 21
   <211> 166
   <212> DNA
   <213> Artificial
<220>
   <223> an artificially synthesized sequence
<400> 21
   atatctcgag gcggccgctg gctagccacc atgagcgtgc ccacccaggt gctgggcctg     60
   ctgctgctgt ggctgaccga cgccaggtgc ttgatggaca aggagtgtgt ctacttctgc    120
   cacctggaca tcatctgggt caacactccc gagtaagata tcctgc                   166
<210> 22
   <211> 76
   <212> DNA
   <213> Artificial
<220>
   <223> an artificially synthesized sequence
<400> 22
   gcaggatatc ttactcggga gtgttgaccc agatgatgtc caggtggcag aagtagacac 60
   actccttgtc catcaa 76
<210> 23
   <211> 25
   <212> DNA
   <213> Artificial
<220>
   <223> an artificially synthesized sequence
<400> 23
   gcaggatatc ttactcggga gtgtt 25
<210> 24
   <211> 37
   <212> DNA
   <213> Artificial
<220>
   <223> an artificially synthesized sequence
<400> 24
   acacactcct tgtccatcaa gcacctggcg tcggtca 37
<210> 25
   <211> 279
   <212> DNA
   <213> Artificial
<220>
   <223> an artificially synthesized sequence
<400> 25
   taagatatcc tgcagacatg ataagataca ttgatgagtt tggacaaacc acaactagaa 60
   tgcagtgaaa aaaatgcttt atttgtgaaa tttgtgatgc tattgcttta tttgtaacca 120
   ttataagctg caataaacaa gttaacaaca acaattgcat tcattttatg tttcaggttc 180
   agggggagat gtgggaggtt ttttaaagca agtaaaacct ctacaaatgt ggtagatccg 240
   tagtaagaaa aacttagggt gaaagttcat cgcggccgc 279
<210> 26
   <211> 30
   <212> DNA
   <213> Artificial
<220>
   <223> an artificially synthesized sequence
<400> 26
   taagatatcc tgcagacatg ataagataca 30
<210> 27
   <211> 422
   <212> DNA
   <213> Artificial
<220>
   <223> an artificially synthesized sequence
<220>
   <221> CDS
   <222> (21)..(143)
<400> 27
   gcggccgctg gctagccacc atg agc gtg ccc acc cag gtg ctg ggc ctg ctg      53
                         Met Ser Val Pro Thr Gln Val Leu Gly Leu Leu
                         1               5                   10
   ctg ctg tgg ctg acc gac gcc agg tgc ttg atg gac aag gag tgt gtc       101
   Leu Leu Trp Leu Thr Asp Ala Arg Cys Leu Met Asp Lys Glu Cys Val
               15                  20                  25
   tac ttc tgc cac ctg gac atc atc tgg gtc aac act ccc gag               143
   Tyr Phe Cys His Leu Asp Ile Ile Trp Val Asn Thr Pro Glu
           30                  35                  40
   taagatatcc tgcagacatg ataagataca ttgatgagtt tggacaaacc acaactagaa     203
   tgcagtgaaa aaaatgcttt atttgtgaaa tttgtgatgc tattgcttta tttgtaacca     263
   ttataagctg caataaacaa gttaacaaca acaattgcat tcattttatg tttcaggttc     323
   agggggagat gtgggaggtt ttttaaagca agtaaaacct ctacaaatgt ggtagatccg     383
   tagtaagaaa aacttagggt gaaagttcat cgcggccgc                             422
<210> 28
   <211> 41
   <212> PRT
   <213> Artificial
<220>
   <223> Synthetic Construct
<400> 28
   Met Ser Val Pro Thr Gln Val Leu Gly Leu Leu Leu Leu Trp Leu Thr
   1               5                   10                  15
   Asp Ala Arg Cys Leu Met Asp Lys Glu Cys Val Tyr Phe Cys His Leu
               20                  25                  30
   Asp Ile Ile Trp Val Asn Thr Pro Glu
           35                  40
<210> 29
   <211> 490
   <212> DNA
   <213> Artificial
<220>
   <223> an artificially synthesized sequence
<400> 29
   tatactcgag gcggccgctg gctagccacc atgatcaagc tgaagttcgg cgtgttcttc 60
   accgtgctgc tgagcagcgc ctacgcccac ggcacccccc agaacatcac cgacctgtgc 120
   gccgagtacc acaacaccca gatccacacc ctgaacgaca agatcttcag ctacaccgag 180
   agcctggccg gcaagagaga gatggccatc atcaccttca agaacggcgc caccttccag 240
   gtggaggtgc ccggctccca gcacatcgac agccagaaga aggccatcga gagaatgaag 300
   gacaccctgc ggatcgccta cctgaccgag gccaaggtgg agaagctgtg cgtgtggaac 360
   aacaagaccc cccacgccat cgccgccatc agcatggcca acggccccgg ccccttgatg 420
   gacaaggagt gtgtctactt ctgccacctg gacatcatct gggtcaacac tcccgagtaa 480
   gatatcctgc 490
<210> 30
   <211> 40
   <212> DNA
   <213> Artificial
<220>
   <223> an artificially synthesized sequence
<400> 30
   actccttgtc catcaagggg ccggggccgt tggccatgct 40
<210> 31
   <211> 746
   <212> DNA
   <213> Artificial
<220>
   <223> an artificially synthesized sequence
<220>
   <221> CDS
   <222> (21)..(467)
<400> 31
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc       101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac       149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc       197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc       245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac       293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc       341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc       389
   Val Trp Asn Asn Lys Thr Pro His Ala Ile Ala Ala Ile Ser Met Ala
           110                 115                 120
   aac ggc ccc ggc ccc ttg atg gac aag gag tgt gtc tac ttc tgc cac       437
   Asn Gly Pro Gly Pro Leu Met Asp Lys Glu Cys Val Tyr Phe Cys His
       125                 130                 135
   ctg gac atc atc tgg gtc aac act ccc gag taagatatcc tgcagacatg         487
   Leu Asp Ile Ile Trp Val Asn Thr Pro Glu
   140                 145
   ataagataca ttgatgagtt tggacaaacc acaactagaa tgcagtgaaa aaaatgcttt     547
   atttgtgaaa tttgtgatgc tattgcttta tttgtaacca ttataagctg caataaacaa     607
   gttaacaaca acaattgcat tcattttatg tttcaggttc agggggagat gtgggaggtt     667
   ttttaaagca agtaaaacct ctacaaatgt ggtagatccg tagtaagaaa aacttagggt     727
   gaaagttcat cgcggccgc                                                  746
<210> 32
   <211> 149
   <212> PRT
   <213> Artificial
<220>
   <223> Synthetic Construct
<400> 32
   Met Ile Lys Leu Lys Phe Gly Val Phe Phe Thr Val Leu Leu Ser Ser
   1               5                   10                  15
   Ala Tyr Ala His Gly Thr Pro Gln Asn Ile Thr Asp Leu Cys Ala Glu
               20                  25                  30
   Tyr His Asn Thr Gln Ile His Thr Leu Asn Asp Lys Ile Phe Ser Tyr
           35                  40                  45
   Thr Glu Ser Leu Ala Gly Lys Arg Glu Met Ala Ile Ile Thr Phe Lys
       50                  55                  60
   Asn Gly Ala Thr Phe Gln Val Glu Val Pro Gly Ser Gln His Ile Asp
   65                  70                  75                  80
   Ser Gln Lys Lys Ala Ile Glu Arg Met Lys Asp Thr Leu Arg Ile Ala
                   85                  90                  95
   Tyr Leu Thr Glu Ala Lys Val Glu Lys Leu Cys Val Trp Asn Asn Lys
               100                 105                 110
   Thr Pro His Ala Ile Ala Ala Ile Ser Met Ala Asn Gly Pro Gly Pro
           115                 120                 125
   Leu Met Asp Lys Glu Cys Val Tyr Phe Cys His Leu Asp Ile Ile Trp
       130                 135                 140
   Val Asn Thr Pro Glu
   145
<210> 33
   <211> 214
   <212> DNA
   <213> Artificial
<220>
   <223> an artificially synthesized sequence
<400> 33
   atatctcgag gcggccgctg gctagccacc atgagcgtgc ccacccaggt gctgggcctg 60
   ctgctgctgt ggctgaccga cgccaggtgc taccctgcca aaccagaggc tcccggcgaa 120
   gacgcctccc cggaggagct gagccgctac tacgcctccc tgcgccacta cctcaacctg 180
   gtcacccggc agcggtatta agatatccag acat 214
<210> 34
   <211> 24
   <212> DNA
   <213> Artificial
<220>
   <223> an artificially synthesized sequence
<400> 34
   atgtctggat atcttaatac cgct 24
<210> 35
   <211> 66
   <212> DNA
   <213> Artificial
<220>
   <223> an artificially synthesized sequence
<400> 35
   ggcgtagtag cggctcagct cctccgggga ggcgtcttcg ccgggagcct ctggtttggc 60
   agggta 66
<210> 36
   <211> 66
   <212> DNA
   <213> Artificial
<220>
   <223> an artificially synthesized sequence
<400> 36
   gatatcttaa taccgctgcc gggtgaccag gttgaggtag tggcgcaggg aggcgtagta 60
   gcggct 66
<210> 37
   <211> 37
   <212> DNA
   <213> Artificial
<220>
   <223> an artificially synthesized sequence
<400> 37
   gcctctggtt tggcagggta gcacctggcg tcggtca 37
<210> 38
   <211> 464
   <212> DNA
   <213> Artificial
<220>
   <223> an artificially synthesized sequence
<220>
   <221> CDS
   <222> (21)..(188)
<400> 38
   gcggccgctg gctagccacc atg agc gtg ccc acc cag gtg ctg ggc ctg ctg      53
                         Met Ser Val Pro Thr Gln Val Leu Gly Leu Leu
                         1               5                   10
   ctg ctg tgg ctg acc gac gcc agg tgc tac cct gcc aaa cca gag gct       101
   Leu Leu Trp Leu Thr Asp Ala Arg Cys Tyr Pro Ala Lys Pro Glu Ala
           15                  20                  25
   ccc ggc gaa gac gcc tcc ccg gag gag ctg agc cgc tac tac gcc tcc       149
   Pro Gly Glu Asp Ala Ser Pro Glu Glu Leu Ser Arg Tyr Tyr Ala Ser
       30                  35                  40
   ctg cgc cac tac ctc aac ctg gtc acc cgg cag cgg tat taagatatcc        198
   Leu Arg His Tyr Leu Asn Leu Val Thr Arg Gln Arg Tyr
       45                  50                  55
   agacatgata agatacattg atgagtttgg acaaaccaca actagaatgc agtgaaaaaa     258
   atgctttatt tgtgaaattt gtgatgctat tgctttattt gtaaccatta taagctgcaa     318
   taaacaagtt aacaacaaca attgcattca ttttatgttt caggttcagg gggagatgtg     378
   ggaggttttt taaagcaagt aaaacctcta caaatgtggt agatccgtag taagaaaaac     438
   ttagggtgaa agttcatcgc ggccgc                                          464
<210> 39
   <211> 56
   <212> PRT
   <213> Artificial
<220>
   <223> Synthetic Construct
<400> 39
   Met Ser Val Pro Thr Gln Val Leu Gly Leu Leu Leu Leu Trp Leu Thr
   1               5                   10                  15
   Asp Ala Arg Cys Tyr Pro Ala Lys Pro Glu Ala Pro Gly Glu Asp Ala
               20                  25                  30
   Ser Pro Glu Glu Leu Ser Arg Tyr Tyr Ala Ser Leu Arg His Tyr Leu
   35 40 45
   Asn Leu Val Thr Arg Gln Arg Tyr
   50 55
<210> 40
   <211> 538
   <212> DNA
   <213> Artificial
<220>
   <223> an artificially synthesized sequence
<400> 40
   atatctcgag gcggccgctg gctagccacc atgatcaagc tgaagttcgg cgtgttcttc 60
   accgtgctgc tgagcagcgc ctacgcccac ggcacccccc agaacatcac cgacctgtgc 120
   gccgagtacc acaacaccca gatccacacc ctgaacgaca agatcttcag ctacaccgag 180
   agcctggccg gcaagagaga gatggccatc atcaccttca agaacggcgc caccttccag 240
   gtggaggtgc ccggctccca gcacatcgac agccagaaga aggccatcga gagaatgaag 300
   gacaccctgc ggatcgccta cctgaccgag gccaaggtgg agaagctgtg cgtgtggaac 360
   aacaagaccc cccacgccat cgccgccatc agcatggcca acggccccgg cccctaccct 420
   gccaaaccag aggctcccgg cgaagacgcc tccccggagg agctgagccg ctactacgcc 480
   tccctgcgcc actacctcaa cctggtcacc cggcagcggt attaagatat ccagacat 538
<210> 41
   <211> 40
   <212> DNA
   <213> Artificial
<220>
   <223> an artificially synthesized sequence
<400> 41
   ctggtttggc agggtagggg ccggggccgt tggccatgct 40
<210> 42
   <211> 788
   <212> DNA
   <213> Artificial
<220>
   <223> an artificially synthesized sequence
<220>
   <221> CDS
   <222> (21)..(512)
<400> 42
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc       101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac       149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc       197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc       245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac       293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc       341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc       389
   Val Trp Asn Asn Lys Thr Pro His Ala Ile Ala Ala Ile Ser Met Ala
           110                 115                 120
   aac ggc ccc ggc ccc tac cct gcc aaa cca gag gct ccc ggc gaa gac       437
   Asn Gly Pro Gly Pro Tyr Pro Ala Lys Pro Glu Ala Pro Gly Glu Asp
       125                 130                 135
   gcc tcc ccg gag gag ctg agc cgc tac tac gcc tcc ctg cgc cac tac       485
   Ala Ser Pro Glu Glu Leu Ser Arg Tyr Tyr Ala Ser Leu Arg His Tyr
   140                 145                 150                 155
   ctc aac ctg gtc acc cgg cag cgg tat taagatatcc agacatgata             532
   Leu Asn Leu Val Thr Arg Gln Arg Tyr
                   160
   agatacattg atgagtttgg acaaaccaca actagaatgc agtgaaaaaa atgctttatt     592
   tgtgaaattt gtgatgctat tgctttattt gtaaccatta taagctgcaa taaacaagtt     652
   aacaacaaca attgcattca ttttatgttt caggttcagg gggagatgtg ggaggttttt     712
   taaagcaagt aaaacctcta caaatgtggt agatccgtag taagaaaaac ttagggtgaa     772
   agttcatcgc ggccgc                                                     788
<210> 43
   <211> 164
   <212> PRT
   <213> Artificial
<220>
   <223> Synthetic Construct
<400> 43
   Met Ile Lys Leu Lys Phe Gly Val Phe Phe Thr Val Leu Leu Ser Ser
   1               5                   10                  15
   Ala Tyr Ala His Gly Thr Pro Gln Asn Ile Thr Asp Leu Cys Ala Glu
               20                  25                  30
   Tyr His Asn Thr Gln Ile His Thr Leu Asn Asp Lys Ile Phe Ser Tyr
           35                  40                  45
   Thr Glu Ser Leu Ala Gly Lys Arg Glu Met Ala Ile Ile Thr Phe Lys
       50                  55                  60
   Asn Gly Ala Thr Phe Gln Val Glu Val Pro Gly Ser Gln His Ile Asp
   65                  70                  75                  80
   Ser Gln Lys Lys Ala Ile Glu Arg Met Lys Asp Thr Leu Arg Ile Ala
                   85                  90                  95
   Tyr Leu Thr Glu Ala Lys Val Glu Lys Leu Cys Val Trp Asn Asn Lys
               100                 105                 110
   Thr Pro His Ala Ile Ala Ala Ile Ser Met Ala Asn Gly Pro Gly Pro
           115                 120                 125
   Tyr Pro Ala Lys Pro Glu Ala Pro Gly Glu Asp Ala Ser Pro Glu Glu
       130                 135                 140
   Leu Ser Arg Tyr Tyr Ala Ser Leu Arg His Tyr Leu Asn Leu Val Thr
   145                 150                 155                 160
   Arg Gln Arg Tyr
<210> 44
   <211> 211
   <212> DNA
   <213> Artificial
<220>
   <223> an artificially synthesized sequence
<400> 44
   atatctcgag gcggccgctg gctagccacc atgagcgtgc ccacccaggt gctgggcctg 60
   ctgctgctgt ggctgaccga cgccaggtgc cacgctgatg gctccttctc tgatgagatg 120
   agcaccattc tggacaatct tgccaccagg gacttcatca actggctgat tcaaaccaag 180
   atcactgact aagatatcct gcagacatga t 211
<210> 45
   <211> 25
   <212> DNA
   <213> Artificial
<220>
   <223> an artificially synthesized sequence
<400> 45
   atcatgtctg caggatatct tagtc 25
<210> 46
   <211> 66
   <212> DNA
   <213> Artificial
<220>
   <223> an artificially synthesized sequence
<400> 46
   gaagtccctg gtggcaagat tgtccagaat ggtgctcatc tcatcagaga aggagccatc 60
   agcgtg 66
<210> 47
   <211> 59
   <212> DNA
   <213> Artificial
<220>
   <223> an artificially synthesized sequence
<400> 47
   gatatcttag tcagtgatct tggtttgaat cagccagttg atgaagtccc tggtggcaa 59
<210> 48
   <211> 36
   <212> DNA
   <213> Artificial
<220>
   <223> an artificially synthesized sequence
<400> 48
   agaaggagcc atcagcgtgg cacctggcgt cggtca 36
<210> 49
   <211> 458
   <212> DNA
   <213> Artificial
<220>
   <223> an artificially synthesized sequence
<220>
   <221> CDS
   <222> (21)..(179)
<400> 49
   gcggccgctg gctagccacc atg agc gtg ccc acc cag gtg ctg ggc ctg ctg      53
                         Met Ser Val Pro Thr Gln Val Leu Gly Leu Leu
                         1               5                   10
   ctg ctg tgg ctg acc gac gcc agg tgc cac gct gat ggc tcc ttc tct       101
   Leu Leu Trp Leu Thr Asp Ala Arg Cys His Ala Asp Gly Ser Phe Ser
               15                  20                  25
   gat gag atg agc acc att ctg gac aat ctt gcc acc agg gac ttc atc       149
   Asp Glu Met Ser Thr Ile Leu Asp Asn Leu Ala Thr Arg Asp Phe Ile
           30                  35                  40
   aac tgg ctg att caa acc aag atc act gac taagatatcc tgcagacatg         199
   Asn Trp Leu Ile Gln Thr Lys Ile Thr Asp
       45                  50
   ataagataca ttgatgagtt tggacaaacc acaactagaa tgcagtgaaa aaaatgcttt     259
   atttgtgaaa tttgtgatgc tattgcttta tttgtaacca ttataagctg caataaacaa     319
   gttaacaaca acaattgcat tcattttatg tttcaggttc agggggagat gtgggaggtt     379
   ttttaaagca agtaaaacct ctacaaatgt ggtagatccg tagtaagaaa aacttagggt     439
   gaaagttcat cgcggccgc                                                  458
<210> 50
   <211> 53
   <212> PRT
   <213> Artificial
<220>
   <223> Synthetic Construct
<400> 50
   Met Ser Val Pro Thr Gln Val Leu Gly Leu Leu Leu Leu Trp Leu Thr
   1               5                   10                  15
   Asp Ala Arg Cys His Ala Asp Gly Ser Phe Ser Asp Glu Met Ser Thr
               20                  25                  30
   Ile Leu Asp Asn Leu Ala Thr Arg Asp Phe Ile Asn Trp Leu Ile Gln
           35                  40                  45
   Thr Lys Ile Thr Asp
       50
<210> 51
   <211> 535
   <212> DNA
   <213> Artificial
<220>
   <223> an artificially synthesized sequence
<400> 51
   atatctcgag gcggccgctg gctagccacc atgatcaagc tgaagttcgg cgtgttcttc      60
   accgtgctgc tgagcagcgc ctacgcccac ggcacccccc agaacatcac cgacctgtgc     120
   gccgagtacc acaacaccca gatccacacc ctgaacgaca agatcttcag ctacaccgag     180
   agcctggccg gcaagagaga gatggccatc atcaccttca agaacggcgc caccttccag     240
   gtggaggtgc ccggctccca gcacatcgac agccagaaga aggccatcga gagaatgaag     300
   gacaccctgc ggatcgccta cctgaccgag gccaaggtgg agaagctgtg cgtgtggaac     360
   aacaagaccc cccacgccat cgccgccatc agcatggcca acggccccgg cccccacgct     420
   gatggctcct tctctgatga gatgagcacc attctggaca atcttgccac cagggacttc     480
   atcaactggc tgattcaaac caagatcact gactaagata tcctgcagac atgat          535
<210> 52
   <211> 40
   <212> DNA
   <213> Artificial
<220>
   <223> an artificially synthesized sequence
<400> 52
   aggagccatc agcgtggggg ccggggccgt tggccatgct 40
<210> 53
   <211> 782
   <212> DNA
   <213> Artificial
<220>
   <223> an artificially synthesized sequence
<220>
   <221> CDS
   <222> (21)..(503)
<400> 53
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc       101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac       149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc       197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc      245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac      293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc      341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc      389
   Val Trp Asn Asn Lys Thr Pro His Ala Ile Ala Ala Ile Ser Met Ala
           110                 115                 120
   aac ggc ccc ggc ccc cac gct gat ggc tcc ttc tct gat gag atg agc      437
   Asn Gly Pro Gly Pro His Ala Asp Gly Ser Phe Ser Asp Glu Met Ser
       125                 130                 135
   acc att ctg gac aat ctt gcc acc agg gac ttc atc aac tgg ctg att      485
   Thr Ile Leu Asp Asn Leu Ala Thr Arg Asp Phe Ile Asn Trp Leu Ile
   140                 145                 150                 155
   caa acc aag atc act gac taagatatcc tgcagacatg ataagataca             533
   Gln Thr Lys Ile Thr Asp
                   160
   ttgatgagtt tggacaaacc acaactagaa tgcagtgaaa aaaatgcttt atttgtgaaa    593
   tttgtgatgc tattgcttta tttgtaacca ttataagctg caataaacaa gttaacaaca    653
   acaattgcat tcattttatg tttcaggttc agggggagat gtgggaggtt ttttaaagca    713
   agtaaaacct ctacaaatgt ggtagatccg tagtaagaaa aacttagggt gaaagttcat    773
   cgcggccgc                                                            782
<210> 54
   <211> 161
   <212> PRT
   <213> Artificial
<220>
   <223> Synthetic Construct
<400> 54
   Met Ile Lys Leu Lys Phe Gly Val Phe Phe Thr Val Leu Leu Ser Ser
   1 5 10 15
   Ala Tyr Ala His Gly Thr Pro Gln Asn Ile Thr Asp Leu Cys Ala Glu
   20 25 30
   Tyr His Asn Thr Gln Ile His Thr Leu Asn Asp Lys Ile Phe Ser Tyr
   35 40 45
   Thr Glu Ser Leu Ala Gly Lys Arg Glu Met Ala Ile Ile Thr Phe Lys
   50 55 60
   Asn Gly Ala Thr Phe Gln Val Glu Val Pro Gly Ser Gln His Ile Asp
   65 70 75 80
   Ser Gln Lys Lys Ala Ile Glu Arg Met Lys Asp Thr Leu Arg Ile Ala
   85 90 95
   Tyr Leu Thr Glu Ala Lys Val Glu Lys Leu Cys Val Trp Asn Asn Lys
   100 105 110
   Thr Pro His Ala Ile Ala Ala Ile Ser Met Ala Asn Gly Pro Gly Pro
   115 120 125
   His Ala Asp Gly Ser Phe Ser Asp Glu Met Ser Thr Ile Leu Asp Asn
   130 135 140
   Leu Ala Thr Arg Asp Phe Ile Asn Trp Leu Ile Gln Thr Lys Ile Thr
   145 150 155 160
   Asp
<210> 55
   <211> 42
   <212> PRT
   <213> Homo sapiens
<400> 55
<210> 56
   <211> 530
   <212> DNA
   <213> Artificial
<220>
   <223> an artificially synthesized sequence
<220>
   <221> CDS
   <222> (18)..(203)
<400> 56
   gcggccgcca aggttca atg agc gtg ccc acc cag gtg ctg ggc ctg ctg        50
                      Met Ser Val Pro Thr Gln Val Leu Gly Leu Leu
                      1               5                   10
   ctg ctg tgg ctg acc gac gcc aga tgc gac gcc gag ttc aga cac gac        98
   Leu Leu Trp Leu Thr Asp Ala Arg Cys Asp Ala Glu Phe Arg His Asp
               15                  20                  25
   agc ggc tac gag gtg cac cac cag aag ctg gtg ttc ttc gcc gag gac       146
   Ser Gly Tyr Glu Val His His Gln Lys Leu Val Phe Phe Ala Glu Asp
           30                  35                  40
   gtg ggc agc aac aag ggc gcc atc atc ggc ctg atg gtg ggc ggc gtg       194
   Val Gly Ser Asn Lys Gly Ala Ile Ile Gly Leu Met Val Gly Gly Val
       45                  50                  55
   gtg atc gcc taagaattca cgcgtggtac ctctagagtc gacccgggcc               243
   Val Ile Ala
   60
   cgcttcgagc agacatgata agatacattg atgagtttgg acaaaccaca actagaatgc     303
   agtgaaaaaa atgctttatt tgtgaaattt gtgatgctat tgctttattt gtaaccatta     363
   taagctgcaa taaacaagtt aacaacaaca attgcattca ttttatgttt caggttcagg     423
   gggagatgtg ggaggttttt taaagcaagt aaaacctcta caaatgtggt aaaatcgata     483
   aggatccacc gtagtaagaa aaacttaggg tgaaagttcg cggccgc                   530
<210> 57
   <211> 62
   <212> PRT
   <213> Artificial
<220>
   <223> Synthetic Construct
<400> 57
   Met Ser Val Pro Thr Gln Val Leu Gly Leu Leu Leu Leu Trp Leu Thr
   1               5                   10                  15
   Asp Ala Arg Cys Asp Ala Glu Phe Arg His Asp Ser Gly Tyr Glu Val
               20                  25                  30
   His His Gln Lys Leu Val Phe Phe Ala Glu Asp Val Gly Ser Asn Lys
           35                  40                  45
   Gly Ala Ile Ile Gly Leu Met Val Gly Gly Val Val Ile Ala
       50                  55                  60
<210> 58
   <211> 45
   <212> DNA
   <213> Artificial
<220>
   <223> an artificially synthesized sequence
<400> 58
   gcgaattcgc caccatgagc gtgcccaccc aggtgctggg cctgc 45
<210> 59
   <211> 45
   <212> DNA
   <213> Artificial
<220>
   <223> an artificially synthesized sequence
<400> 59
   gctacgaggt gcaccaccag aagctggtgt tcttcgccga ggacg 45
<210> 60
   <211> 45
   <212> DNA
   <213> Artificial
<220>
   <223> an artificially synthesized sequence
<400> 60
   cgtcgcatct ggcgtcggtc agccacagca gcagcaggcc cagca 45
<210> 61
   <211> 45
   <212> DNA
   <213> Artificial
<220>
   <223> an artificially synthesized sequence
<400> 61
   gcacctcgta gccgctgtcg tgtctgaact cggcgtcgca tctgg 45
<210> 62
   <211> 45
   <212> DNA
   <213> Artificial
<220>
   <223> an artificially synthesized sequence
<400> 62
   tcaggccgat gatggcgccc ttgttgctgc ccacgtcctc ggcga 45
<210> 63
   <211> 45
   <212> DNA
   <213> Artificial
<220>
   <223> an artificially synthesized sequence
<400> 63
   agaattctta ggcgatcacc acgccgccca ccatcaggcc gatga 45
<210> 64
   <211> 25
   <212> DNA
   <213> Artificial
<220>
   <223> an artificially synthesized sequence
<400> 64
   ggcgaattcg ccaccatgag cgtgc 25
<210> 65
   <211> 25
   <212> DNA
   <213> Artificial
<220>
   <223> an artificially synthesized sequence
<400> 65
   taagaattct taggcgatca ccacg 25
<210> 66
   <211> 43
   <212> DNA
   <213> Artificial
<220>
   <223> an artificially synthesized sequence
<400> 66
   atctgcggcc gccaaggttc aatgagcgtg cccacccagg tgc 43
<210> 67
   <211> 60
   <212> DNA
   <213> Artificial
<220>
   <223> an artificially synthesized sequence
<400> 67
   acctgcggcc gcgatgaact ttcaccctaa gtttttctta ctacggtgga tccttatcga 60
<210> 68
   <211> 124
   <212> PRT
   <213> Vibrio cholerae
<400> 68
   Met Ile Lys Leu Lys Phe Gly Val Phe Phe Thr Val Leu Leu Ser Ser
   1 5 10 15
   Ala Tyr Ala His Gly Thr Pro Gln Asn Ile Thr Asp Leu Cys Ala Glu
   20 25 30
   Tyr His Asn Thr Gln Ile His Thr Leu Asn Asp Lys Ile Phe Ser Tyr
   35 40 45
   Thr Glu Ser Leu Ala Gly Lys Arg Glu Met Ala Ile Ile Thr Phe Lys
   50 55 60
   Asn Gly Ala Thr Phe Gln Val Glu Val Pro Gly Ser Gln His Ile Asp
   65 70 75 80
   Ser Gln Lys Lys Ala Ile Glu Arg Met Lys Asp Thr Leu Arg Ile Ala
   85 90 95
   Tyr Leu Thr Glu Ala Lys Val Glu Lys Leu Cys Val Trp Asn Asn Lys
   100 105 110
   Thr Pro His Ala Ile Ala Ala Ile Ser Met Ala Asn
   115 120
<210> 69
   <211> 800
   <212> DNA
   <213> Artificial
<220>
   <223> an artificially synthesized sequence
<220>
   <221> CDS
   <222> (21)..(530)
<400> 69
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc       101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac       149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc       197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc       245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac       293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc       341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc       389
   Val Trp Asn Asn Lys Thr Pro His Ala Ile Ala Ala Ile Ser Met Ala
           110                 115                 120
   aac ggc ccc ggc ccc gac gcc gag ttc aga cac gac agc ggc tac gag       437
   Asn Gly Pro Gly Pro Asp Ala Glu Phe Arg His Asp Ser Gly Tyr Glu
       125                 130                 135
   gtg cac cac cag aag ctg gtg ttc ttc gcc gag gac gtg ggc agc aac       485
   Val His His Gln Lys Leu Val Phe Phe Ala Glu Asp Val Gly Ser Asn
   140                 145                 150                 155
   aag ggc gcc atc atc ggc ctg atg gtg ggc ggc gtg gtg atc gcc           530
   Lys Gly Ala Ile Ile Gly Leu Met Val Gly Gly Val Val Ile Ala
                   160                 165                 170
   taacagacat gataagatac attgatgagt ttggacaaac cacaactaga atgcagtgaa     590
   aaaaatgctt tatttgtgaa atttgtgatg ctattgcttt atttgtaacc attataagct     650
   gcaataaaca agttaacaac aacaattgca ttcattttat gtttcaggtt cagggggaga    710
   tgtgggaggt tttttaaagc aagtaaaacc tctacaaatg tggtagatcc gtagtaagaa     770
   aaacttaggg tgaaagttca tcgcggccgc                                      800
<210> 70
   <211> 170
   <212> PRT
   <213> Artificial
<220>
   <223> Synthetic Construct
<400> 70
   Met Ile Lys Leu Lys Phe Gly Val Phe Phe Thr Val Leu Leu Ser Ser
   1               5                   10                  15
   Ala Tyr Ala His Gly Thr Pro Gln Asn Ile Thr Asp Leu Cys Ala Glu
               20                  25                  30
   Tyr His Asn Thr Gln Ile His Thr Leu Asn Asp Lys Ile Phe Ser Tyr
           35                  40                  45
   Thr Glu Ser Leu Ala Gly Lys Arg Glu Met Ala Ile Ile Thr Phe Lys
       50                  55                  60
   Asn Gly Ala Thr Phe Gln Val Glu Val Pro Gly Ser Gln His Ile Asp
   65                  70                  75                  80
   Ser Gln Lys Lys Ala Ile Glu Arg Met Lys Asp Thr Leu Arg Ile Ala
                   85                  90                  95
   Tyr Leu Thr Glu Ala Lys Val Glu Lys Leu Cys Val Trp Asn Asn Lys
               100                 105                 110
   Thr Pro His Ala Ile Ala Ala Ile Ser Met Ala Asn Gly Pro Gly Pro
           115                 120                 125
   Asp Ala Glu Phe Arg His Asp Ser Gly Tyr Glu Val His His Gln Lys
       130                 135                 140
   Leu Val Phe Phe Ala Glu Asp Val Gly Ser Asn Lys Gly Ala Ile Ile
   145                 150                 155                 160
   Gly Leu Met Val Gly Gly Val Val Ile Ala
                   165                 170
<210> 71
   <211> 84
   <212> DNA
   <213> Artificial
<220>
   <223> an artificially synthesized sequence
<400> 71
   cagcggccgc tggctagcca ccatgatcaa gctgaagttc ggcgtgttct tcaccgtgct 60
   gctgagcagc gcctacgccc acgg 84
<210> 72
   <211> 84
   <212> DNA
   <213> Artificial
<220>
   <223> an artificially synthesized sequence
<400> 72
   acccagatcc acaccctgaa cgacaagatc ttcagctaca ccgagagcct ggccggcaag 60
   agagagatgg ccatcatcac cttc 84
<210> 73
   <211> 85
   <212> DNA
   <213> Artificial
<220>
   <223> an artificially synthesized sequence
<400> 73
   cacatcgaca gccagaagaa ggccatcgag agaatgaagg acaccctgcg gatcgcctac 60
   ctgaccgagg ccaaggtgga gaagc 85
<210> 74
   <211> 86
   <212> DNA
   <213> Artificial
<220>
   <223> an artificially synthesized sequence
<400> 74
   tcagcatggc caacggcccc ggccccgacg ccgagttcag acacgacagc ggctacgagg 60
   tgcaccacca gaagctggtg ttcttc 86
<210> 75
   <211> 85
   <212> DNA
   <213> Artificial
<220>
   <223> an artificially synthesized sequence
<400> 75
   ttcagggtgt ggatctgggt gttgtggtac tcggcgcaca ggtcggtgat gttctggggg 60
   gtgccgtggg cgtaggcgct gctca 85
<210> 76
   <211> 84
   <212> DNA
   <213> Artificial
<220>
   <223> an artificially synthesized sequence
<400> 76
   cttcttctgg ctgtcgatgt gctgggagcc gggcacctcc acctggaagg tggcgccgtt 60
   cttgaaggtg atgatggcca tctc 84
<210> 77
   <211> 86
   <212> DNA
   <213> Artificial
<220>
   <223> an artificially synthesized sequence
<400> 77
   cggggccgtt ggccatgctg atggcggcga tggcgtgggg ggtcttgttg ttccacacgc 60
   acagcttctc caccttggcc tcggtc 86
<210> 78
   <211> 85
   <212> DNA
   <213> Artificial
<220>
   <223> an artificially synthesized sequence
<400> 78
   gatcaccacg ccgcccacca tcaggccgat gatggcgccc ttgttgctgc ccacgtcctc 60
   ggcgaagaac accagcttct ggtgg 85
<210> 79
   <211> 30
   <212> DNA
   <213> Artificial
<220>
   <223> an artificially synthesized sequence
<400> 79
   cagcggccgc tggctagcca ccatgatcaa 30
<210> 80
   <211> 27
   <212> DNA
   <213> Artificial
<220>
   <223> an artificially synthesized sequence
<400> 80
   tcgcggccgc gatgaacttt cacccta 27
<210> 81
   <211> 423
   <212> DNA
   <213> Mus musculus
<220>
   <221> CDS
   <222> (1)..(420)
<400> 81
   atg ggt ctc aac ccc cag cta gtt gtc atc ctg ctc ttc ttt ctc gaa      48
   Met Gly Leu Asn Pro Gln Leu Val Val Ile Leu Leu Phe Phe Leu Glu
   1               5                   10                  15
   tgt acc agg agc cat atc cac gga tgc gac aaa aat cac ttg aga gag      96
   Cys Thr Arg Ser His Ile His Gly Cys Asp Lys Asn His Leu Arg Glu
               20                  25                  30
   atc atc ggc att ttg aac gag gtc aca gga gaa ggg acg cca tgc acg     144
   Ile Ile Gly Ile Leu Asn Glu Val Thr Gly Glu Gly Thr Pro Cys Thr
           35                  40                  45
   gag atg gat gtg cca aac gtc ctc aca gca acg aag aac acc aca gag     192
   Glu Met Asp Val Pro Asn Val Leu Thr Ala Thr Lys Asn Thr Thr Glu
       50                  55                  60
   agt gag ctc gtc tgt agg gct tcc aag gtg ctt cgc ata ttt tat tta     240
   Ser Glu Leu Val Cys Arg Ala Ser Lys Val Leu Arg Ile Phe Tyr Leu
   65                  70                  75                  80
   aaa cat ggg aaa act cca tgc ttg aag aag aac tct agt gtt ctc atg      288
   Lys His Gly Lys Thr Pro Cys Leu Lys Lys Asn Ser Ser Val Leu Met
                   85                  90                  95
   gag ctg cag aga ctc ttt cgg gct ttt cga tgc ctg gat tca tcg ata      336
   Glu Leu Gln Arg Leu Phe Arg Ala Phe Arg Cys Leu Asp Ser Ser Ile
               100                 105                 110
   agc tgc acc atg aat gag tcc aag tcc aca tca ctg aaa gac ttc ctg      384
   Ser Cys Thr Met Asn Glu Ser Lys Ser Thr Ser Leu Lys Asp Phe Leu
           115                 120                 125
   gaa agc cta aag agc atc atg caa atg gat tac tcg tag                  423
   Glu Ser Leu Lys Ser Ile Met Gln Met Asp Tyr Ser
       130                 135                 140
<210> 82
   <211> 140
   <212> PRT
   <213> Mus musculus
<400> 82
   Met Gly Leu Asn Pro Gln Leu Val Val Ile Leu Leu Phe Phe Leu Glu
   1               5                   10                  15
   Cys Thr Arg Ser His Ile His Gly Cys Asp Lys Asn His Leu Arg Glu
               20                  25                  30
   Ile Ile Gly Ile Leu Asn Glu Val Thr Gly Glu Gly Thr Pro Cys Thr
           35                  40                  45
   Glu Met Asp Val Pro Asn Val Leu Thr Ala Thr Lys Asn Thr Thr Glu
       50                  55                  60
   Ser Glu Leu Val Cys Arg Ala Ser Lys Val Leu Arg Ile Phe Tyr Leu
   65                  70                  75                  80
   Lys His Gly Lys Thr Pro Cys Leu Lys Lys Asn Ser Ser Val Leu Met
                   85                  90                  95
   Glu Leu Gln Arg Leu Phe Arg Ala Phe Arg Cys Leu Asp Ser Ser Ile
               100                 105                 110
   Ser Cys Thr Met Asn Glu Ser Lys Ser Thr Ser Leu Lys Asp Phe Leu
           115                 120                 125
   Glu Ser Leu Lys Ser Ile Met Gln Met Asp Tyr Ser
       130                 135                 140
<210> 83
   <211> 45
   <212> DNA
   <213> Artificial
<220>
   <223> an artificially synthesized sequence
<400> 83
   attgcggccg ccaaggttca atgggtctca acccccagct agttg 45
<210> 84
   <211> 44
   <212> DNA
   <213> Artificial
<220>
   <223> an artificially synthesized sequence
<400> 84
   gtctgaactc ggcgtcgctg ccgccgcccg agtaatccat ttgc 44
<210> 85
   <211> 44
   <212> DNA
   <213> Artificial
<220>
   <223> an artificially synthesized sequence
<400> 85
   gcaaatggat tactcgggcg gcggcagcga cgccgagttc agac 44
<210> 86
   <211> 79
   <212> DNA
   <213> Artificial
<220>
   <223> an artificially synthesized sequence
<400> 86
   attgcggccg cgatgaactt tcaccctaag tttttcttac tacggttagg cgatcaccac 60
   gccgcccacc atcaggccg 79
<210> 87
   <211> 620
   <212> DNA
   <213> Artificial
<220>
   <223> an artificially synthesized sequence
<220>
   <221> CDS
   <222> (18)..(575)
<400> 87
   gcggccgcca aggttca atg ggt ctc aac ccc cag cta gtt gtc atc ctg         50
                      Met Gly Leu Asn Pro Gln Leu Val Val Ile Leu
                      1               5                   10
   ctc ttc ttt ctc gaa tgt acc agg agc cat atc cac gga tgc gac aaa         98
   Leu Phe Phe Leu Glu Cys Thr Arg Ser His Ile His Gly Cys Asp Lys
               15                  20                  25
   aat cac ttg aga gag atc atc ggc att ttg aac gag gtc aca gga gaa        146
   Asn His Leu Arg Glu Ile Ile Gly Ile Leu Asn Glu Val Thr Gly Glu
           30                  35                  40
   ggg acg cca tgc acg gag atg gat gtg cca aac gtc ctc aca gca acg        194
   Gly Thr Pro Cys Thr Glu Met Asp Val Pro Asn Val Leu Thr Ala Thr
       45                  50                  55
   aag aac acc aca gag agt gag ctc gtc tgt agg gct tcc aag gtg ctt        242
   Lys Asn Thr Thr Glu Ser Glu Leu Val Cys Arg Ala Ser Lys Val Leu
   60                  65                  70                  75
   cgc ata ttt tat tta aaa cat ggg aaa act cca tgc ttg aag aag aac        290
   Arg Ile Phe Tyr Leu Lys His Gly Lys Thr Pro Cys Leu Lys Lys Asn
                   80                  85                  90
   tct agt gtt ctc atg gag ctg cag aga ctc ttt cgg gct ttt cga tgc        338
   Ser Ser Val Leu Met Glu Leu Gln Arg Leu Phe Arg Ala Phe Arg Cys
               95                  100                 105
   ctg gat tca tcg ata agc tgc acc atg aat gag tcc aag tcc aca tca        386
   Leu Asp Ser Ser Ile Ser Cys Thr Met Asn Glu Ser Lys Ser Thr Ser
           110                 115                 120
   ctg aaa gac ttc ctg gaa agc cta aag agc atc atg caa atg gat tac        434
   Leu Lys Asp Phe Leu Glu Ser Leu Lys Ser Ile Met Gln Met Asp Tyr
       125                 130                 135
   tcg ggc ggc ggc agc gac gcc gag ttc aga cac gac agc ggc tac gag        482
   Ser Gly Gly Gly Ser Asp Ala Glu Phe Arg His Asp Ser Gly Tyr Glu
   140                 145                 150                 155
   gtg cac cac cag aag ctg gtg ttc ttc gcc gag gac gtg ggc agc aac        530
   Val His His Gln Lys Leu Val Phe Phe Ala Glu Asp Val Gly Ser Asn
                   160                 165                 170
   aag ggc gcc atc atc ggc ctg atg gtg ggc ggc gtg gtg atc gcc            575
   Lys Gly Ala Ile Ile Gly Leu Met Val Gly Gly Val Val Ile Ala
               175                 180                 185
   taaccgtagt aagaaaaact tagggtgaaa gttcatcgcg gccgc                      620
<210> 88
   <211> 186
   <212> PRT
   <213> Artificial
<220>
   <223> Synthetic Construct
<400> 88
   Met Gly Leu Asn Pro Gln Leu Val Val Ile Leu Leu Phe Phe Leu Glu
   1               5                   10                  15
   Cys Thr Arg Ser His Ile His Gly Cys Asp Lys Asn His Leu Arg Glu
               20                  25                  30
   Ile Ile Gly Ile Leu Asn Glu Val Thr Gly Glu Gly Thr Pro Cys Thr
           35                  40                  45
   Glu Met Asp Val Pro Asn Val Leu Thr Ala Thr Lys Asn Thr Thr Glu
       50                  55                  60
   Ser Glu Leu Val Cys Arg Ala Ser Lys Val Leu Arg Ile Phe Tyr Leu
   65                  70                  75                  80
   Lys His Gly Lys Thr Pro Cys Leu Lys Lys Asn Ser Ser Val Leu Met
                   85                  90                  95
   Glu Leu Gln Arg Leu Phe Arg Ala Phe Arg Cys Leu Asp Ser Ser Ile
               100                 105                 110
   Ser Cys Thr Met Asn Glu Ser Lys Ser Thr Ser Leu Lys Asp Phe Leu
           115                 120                 125
   Glu Ser Leu Lys Ser Ile Met Gln Met Asp Tyr Ser Gly Gly Gly Ser
       130                 135                 140
   Asp Ala Glu Phe Arg His Asp Ser Gly Tyr Glu Val His His Gln Lys
   145                 150                 155                 160
   Leu Val Phe Phe Ala Glu Asp Val Gly Ser Asn Lys Gly Ala Ile Ile
                   165                 170                 175
   Gly Leu Met Val Gly Gly Val Val Ile Ala
               180                 185
<210> 89
   <211> 99
   <212> DNA
   <213> Artificial
<220>
   <223> an artificially synthesized sequence
<400> 89
   gccagatgcg ccgaggaggc cttcgacctg tggaacgagt gcgccaaggc ctgcgtgctg 60
   gacctgaagg acggcgtgag aagcagcaga atgagcgtg 99
<210> 90
   <211> 96
   <212> DNA
   <213> Artificial
<220>
   <223> an artificially synthesized sequence
<400> 90
   gcgtcgttgc cgccctccag caccatgctg tagtgcagca cgccctggcc gttggtgtcg 60
   gcgatggcgg ggtccacgct cattctgctg cttctc 96
<210> 91
   <211> 99
   <212> DNA
   <213> Artificial
<220>
   <223> an artificially synthesized sequence
<400> 91
   ctggagggcg gcaacgacgc cctgaagctg gccatcgaca acgccctgag catcaccagc 60
   gacggcctga ccatcagact ggagggcggc gtggagccc 99
<210> 92
   <211> 97
   <212> DNA
   <213> Artificial
<220>
   <223> an artificially synthesized sequence
<400> 92
   ccgatgggca ccagccagtt caggctccag ctgcctctgg cctgtctggt gtagctgtat 60
   ctcacgggct tgttgggctc cacgccgccc tccagtc 97
<210> 93
   <211> 96
   <212> DNA
   <213> Artificial
<220>
   <223> an artificially synthesized sequence
<400> 93
   gaactggctg gtgcccatcg gccacgagaa gcccagcaac atcaaggtgt tcatccacga 60
   gctgaacgcc ggcaaccagc tgagccacat gagccc 96
<210> 94
   <211> 98
   <212> DNA
   <213> Artificial
<220>
   <223> an artificially synthesized sequence
<400> 94
   cgtgggctct cacgaagaag gtggcgtctc tggccagctt ggccagcagc tcgtcgccca 60
   tctcgatggt gtagatgggg ctcatgtggc tcagctgg 98
<210> 95
   <211> 99
   <212> DNA
   <213> Artificial
<220>
   <223> an artificially synthesized sequence
<400> 95
   caccttcttc gtgagagccc acgagagcaa cgagatgcag cccaccctgg ccatcagcca 60
   cgccggcgtg agcgtggtga tggcccaggc ccagcccag 99
<210> 96
   <211> 99
   <212> DNA
   <213> Artificial
<220>
   <223> an artificially synthesized sequence
<400> 96
   gttgtacacg ccgtccaggg ggtccagcag gcacagcacc ttgccgctgg cccactcgct 60
   ccatctcttc tctcttctgg gctgggcctg ggccatcac 99
<210> 97
   <211> 96
   <212> DNA
   <213> Artificial
<220>
   <223> an artificially synthesized sequence
<400> 97
   gaccccctgg acggcgtgta caactacctg gcccagcaga gatgcaacct ggacgacacc 60
   tgggagggca agatctacag agtgctggcc ggcaac 96
<210> 98
   <211> 98
   <212> DNA
   <213> Artificial
<220>
   <223> an artificially synthesized sequence
<400> 98
   cgccgccgcc gccctcgggg aagtgcagtc tgtggctgat cacggtgggc ttgatgtcca 60
   ggtcgtgctt ggcggggttg ccggccagca ctctgtag 98
<210> 99
   <211> 22
   <212> DNA
   <213> Artificial
<220>
   <223> an artificially synthesized sequence
<400> 99
   acaagagaaa aaacatgtat gg 22
<210> 100
   <211> 35
   <212> DNA
   <213> Artificial
<220>
   <223> an artificially synthesized sequence
<400> 100
   cgaaggcctc ctcggcgcat ctggcgtcgg tcagc 35
<210> 101
   <211> 35
   <212> DNA
   <213> Artificial
<220>
   <223> an artificially synthesized sequence
<400> 101
   cgagggcggc ggcggcgacg ccgagttcag acacg 35
<210> 102
   <211> 30
   <212> DNA
   <213> Artificial
<220>
   <223> an artificially synthesized sequence
<400> 102
   gcatcatcag tcacacttgg gcctagtacg 30
<210> 103
   <211> 32
   <212> DNA
   <213> Artificial
<220>
   <223> an artificially synthesized sequence
<400> 103
   gatgcgccga ggaggccttc gacctgtgga ac 32
<210> 104
   <211> 34
   <212> DNA
   <213> Artificial
<220>
   <223> an artificially synthesized sequence
<400> 104
   gaactcggcg tcgccgccgc cgccctcggg gaag 34
<210> 105
   <211> 1008
   <212> DNA
   <213> Artificial
<220>
   <223> an artificially synthesized sequence
<220>
   <221> CDS
   <222> (10)..(963)
<400> 105
   caaggttca atg agc gtg ccc acc cag gtg ctg ggc ctg ctg ctg ctg tgg     51
             Met Ser Val Pro Thr Gln Val Leu Gly Leu Leu Leu Leu Trp
             1               5                   10
   ctg acc gac gcc aga tgc gcc gag gag gcc ttc gac ctg tgg aac gag        99
   Leu Thr Asp Ala Arg Cys Ala Glu Glu Ala Phe Asp Leu Trp Asn Glu
   15                  20                  25                  30
   tgc gcc aag gcc tgc gtg ctg gac ctg aag gac ggc gtg aga agc agc       147
   Cys Ala Lys Ala Cys Val Leu Asp Leu Lys Asp Gly Val Arg Ser Ser
                   35                  40                  45
   aga atg agc gtg gac ccc gcc atc gcc gac acc aac ggc cag ggc gtg       195
   Arg Met Ser Val Asp Pro Ala Ile Ala Asp Thr Asn Gly Gln Gly Val
               50                  55                  60
   ctg cac tac agc atg gtg ctg gag ggc ggc aac gac gcc ctg aag ctg       243
   Leu His Tyr Ser Met Val Leu Glu Gly Gly Asn Asp Ala Leu Lys Leu
           65                  70                  75
   gcc atc gac aac gcc ctg agc atc acc agc gac ggc ctg acc atc aga       291
   Ala Ile Asp Asn Ala Leu Ser Ile Thr Ser Asp Gly Leu Thr Ile Arg
       80                  85                  90
   ctg gag ggc ggc gtg gag ccc aac aag ccc gtg aga tac agc tac acc       339
   Leu Glu Gly Gly Val Glu Pro Asn Lys Pro Val Arg Tyr Ser Tyr Thr
   95                  100                 105                 110
   aga cag gcc aga ggc agc tgg agc ctg aac tgg ctg gtg ccc atc ggc       387
   Arg Gln Ala Arg Gly Ser Trp Ser Leu Asn Trp Leu Val Pro Ile Gly
                   115                 120                 125
   cac gag aag ccc agc aac atc aag gtg ttc atc cac gag ctg aac gcc       435
   His Glu Lys Pro Ser Asn Ile Lys Val Phe Ile His Glu Leu Asn Ala
               130                 135                 140
   ggc aac cag ctg agc cac atg agc ccc atc tac acc atc gag atg ggc       483
   Gly Asn Gln Leu Ser His Met Ser Pro Ile Tyr Thr Ile Glu Met Gly
           145                 150                 155
   gac gag ctg ctg gcc aag ctg gcc aga gac gcc acc ttc ttc gtg aga       531
   Asp Glu Leu Leu Ala Lys Leu Ala Arg Asp Ala Thr Phe Phe Val Arg
       160                 165                 170
   gcc cac gag agc aac gag atg cag ccc acc ctg gcc atc agc cac gcc        579
   Ala His Glu Ser Asn Glu Met Gln Pro Thr Leu Ala Ile Ser His Ala
   175                 180                 185                 190
   ggc gtg agc gtg gtg atg gcc cag gcc cag ccc aga aga gag aag aga        627
   Gly Val Ser Val Val Met Ala Gln Ala Gln Pro Arg Arg Glu Lys Arg
                   195                 200                 205
   tgg agc gag tgg gcc agc ggc aag gtg ctg tgc ctg ctg gac ccc ctg        675
   Trp Ser Glu Trp Ala Ser Gly Lys Val Leu Cys Leu Leu Asp Pro Leu
               210                 215                 220
   gac ggc gtg tac aac tac ctg gcc cag cag aga tgc aac ctg gac gac        723
   Asp Gly Val Tyr Asn Tyr Leu Ala Gln Gln Arg Cys Asn Leu Asp Asp
           225                 230                 235
   acc tgg gag ggc aag atc tac aga gtg ctg gcc ggc aac ccc gcc aag        771
   Thr Trp Glu Gly Lys Ile Tyr Arg Val Leu Ala Gly Asn Pro Ala Lys
       240                 245                 250
   cac gac ctg gac atc aag ccc acc gtg atc agc cac aga ctg cac ttc        819
   His Asp Leu Asp Ile Lys Pro Thr Val Ile Ser His Arg Leu His Phe
   255                 260                 265                 270
   ccc gag ggc ggc ggc ggc gac gcc gag ttc aga cac gac agc ggc tac        867
   Pro Glu Gly Gly Gly Gly Asp Ala Glu Phe Arg His Asp Ser Gly Tyr
                   275                 280                 285
   gag gtg cac cac cag aag ctg gtg ttc ttc gcc gag gac gtg ggc agc        915
   Glu Val His His Gln Lys Leu Val Phe Phe Ala Glu Asp Val Gly Ser
               290                 295                 300
   aac aag ggc gcc atc atc ggc ctg atg gtg ggc ggc gtg gtg atc gcc        963
   Asn Lys Gly Ala Ile Ile Gly Leu Met Val Gly Gly Val Val Ile Ala
           305                 310                 315
   taaccgtagt aagaaaaact tagggtgaaa gttcatcgcg gccgc                     1008
<210> 106
   <211> 318
   <212> PRT
   <213> Artificial
<220>
   <223> Synthetic Construct
<400> 106
   Met Ser Val Pro Thr Gln Val Leu Gly Leu Leu Leu Leu Trp Leu Thr
   1               5                   10                  15
   Asp Ala Arg Cys Ala Glu Glu Ala Phe Asp Leu Trp Asn Glu Cys Ala
               20                  25                  30
   Lys Ala Cys Val Leu Asp Leu Lys Asp Gly Val Arg Ser Ser Arg Met
           35                  40                  45
   Ser Val Asp Pro Ala Ile Ala Asp Thr Asn Gly Gln Gly Val Leu His
   50 55 60
   Tyr Ser Met Val Leu Glu Gly Gly Asn Asp Ala Leu Lys Leu Ala Ile
   65 70 75 80
   Asp Asn Ala Leu Ser Ile Thr Ser Asp Gly Leu Thr Ile Arg Leu Glu
   85 90 95
   Gly Gly Val Glu Pro Asn Lys Pro Val Arg Tyr Ser Tyr Thr Arg Gln
   100 105 110
   Ala Arg Gly Ser Trp Ser Leu Asn Trp Leu Val Pro Ile Gly His Glu
   115 120 125
   Lys Pro Ser Asn Ile Lys Val Phe Ile His Glu Leu Asn Ala Gly Asn
   130 135 140
   Gln Leu Ser His Met Ser Pro Ile Tyr Thr Ile Glu Met Gly Asp Glu
   145 150 155 160
   Leu Leu Ala Lys Leu Ala Arg Asp Ala Thr Phe Phe Val Arg Ala His
   165 170 175
   Glu Ser Asn Glu Met Gln Pro Thr Leu Ala Ile Ser His Ala Gly Val
   180 185 190
   Ser Val Val Met Ala Gln Ala Gln Pro Arg Arg Glu Lys Arg Trp Ser
   195 200 205
   Glu Trp Ala Ser Gly Lys Val Leu Cys Leu Leu Asp Pro Leu Asp Gly
   210 215 220
   Val Tyr Asn Tyr Leu Ala Gln Gln Arg Cys Asn Leu Asp Asp Thr Trp
   225 230 235 240
   Glu Gly Lys Ile Tyr Arg Val Leu Ala Gly Asn Pro Ala Lys His Asp
   245 250 255
   Leu Asp Ile Lys Pro Thr Val Ile Ser His Arg Leu His Phe Pro Glu
   260 265 270
   Gly Gly Gly Gly Asp Ala Glu Phe Arg His Asp Ser Gly Tyr Glu Val
   275 280 285
   His His Gln Lys Leu Val Phe Phe Ala Glu Asp Val Gly Ser Asn Lys
   290 295 300
   Gly Ala Ile Ile Gly Leu Met Val Gly Gly Val Val Ile Ala
   305 310 315
<210> 107
   <211> 34
   <212> DNA
   <213> Artificial
<220>
   <223> an artificially synthesized sequence
<400> 107
   aacagatatc actggtggtg cacctcgtag ccgc 34
<210> 108
   <211> 33
   <212> DNA
   <213> Artificial
<220>
   <223> an artificially synthesized sequence
<400> 108
   atctgatatc agacatgata agatacattg atg 33
<210> 109
   <211> 722
   <212> DNA
   <213> Artificial
<220>
   <223> an artificially synthesized sequence
<220>
   <221> CDS
   <222> (21)..(449)
<400> 109
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc       101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac       149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc       197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc       245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac       293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc       341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc       389
   Val Trp Asn Asn Lys Thr Pro His Ala Ile Ala Ala Ile Ser Met Ala
           110                 115                 120
   aac ggc ccc ggc ccc gac gcc gag ttc aga cac gac agc ggc tac gag       437
   Asn Gly Pro Gly Pro Asp Ala Glu Phe Arg His Asp Ser Gly Tyr Glu
       125                 130                 135
   gtg cac cac cag tgatatcaga catgataaga tacattgatg agtttggaca           489
   Val His His Gln
   140
   aaccacaact agaatgcagt gaaaaaaatg ctttatttgt gaaatttgtg atgctattgc     549
   tttatttgta accattataa gctgcaataa acaagttaac aacaacaatt gcattcattt     609
   tatgtttcag gttcaggggg agatgtggga ggttttttaa agcaagtaaa acctctacaa     669
   atgtggtaga tccgtagtaa gaaaaactta gggtgaaagt tcatcgcggc cgc             722
<210> 110
   <211> 143
   <212> PRT
   <213> Artificial
<220>
   <223> Synthetic Construct
<400> 110
   Met Ile Lys Leu Lys Phe Gly Val Phe Phe Thr Val Leu Leu Ser Ser
   1               5                   10                  15
   Ala Tyr Ala His Gly Thr Pro Gln Asn Ile Thr Asp Leu Cys Ala Glu
               20                  25                  30
   Tyr His Asn Thr Gln Ile His Thr Leu Asn Asp Lys Ile Phe Ser Tyr
           35                  40                  45
   Thr Glu Ser Leu Ala Gly Lys Arg Glu Met Ala Ile Ile Thr Phe Lys
       50                  55                  60
   Asn Gly Ala Thr Phe Gln Val Glu Val Pro Gly Ser Gln His Ile Asp
   65                  70                  75                  80
   Ser Gln Lys Lys Ala Ile Glu Arg Met Lys Asp Thr Leu Arg Ile Ala
                   85                  90                  95
   Tyr Leu Thr Glu Ala Lys Val Glu Lys Leu Cys Val Trp Asn Asn Lys
               100                 105                 110
   Thr Pro His Ala Ile Ala Ala Ile Ser Met Ala Asn Gly Pro Gly Pro
           115                 120                 125
   Asp Ala Glu Phe Arg His Asp Ser Gly Tyr Glu Val His His Gln
       130                 135                 140
<210> 111
   <211> 29
   <212> DNA
   <213> Artificial
<220>
   <223> an artificially synthesized sequence
<400> 111
   cggccccggg gccgttggcc atgctgatg 29
<210> 112
   <211> 34
   <212> DNA
   <213> Artificial
<220>
   <223> an artificially synthesized sequence
<400> 112
   gatgcccggg cagacatgat aagatacatt gatg 34
<210> 113
   <211> 542
   <212> DNA
   <213> Artificial
<220>
   <223> an artificially synthesized sequence
<220>
   <221> CDS
   <222> (30)..(491)
<400> 113
   gcggccgcca aggttcagaa ttcgccacc atg agc gtg ccc acc cag gtg ctg      53
                                   Met Ser Val Pro Thr Gln Val Leu
                                   1               5
   ggc ctg ctg ctg ctg tgg ctg acc gac gcc aga tgc gac gcc gag ttc     101
   Gly Leu Leu Leu Leu Trp Leu Thr Asp Ala Arg Cys Asp Ala Glu Phe
       10                  15                  20
   aga cac gac agc ggc tac gag gtg cac cac cag aag aag gac gcc gag     149
   Arg His Asp Ser Gly Tyr Glu Val His His Gln Lys Lys Asp Ala Glu
   25                  30                  35                  40
   ttc aga cac gac agc ggc tac gag gtg cac cac cag aag aag gac gcc     197
   Phe Arg His Asp Ser Gly Tyr Glu Val His His Gln Lys Lys Asp Ala
                   45                  50                  55
   gag ttc aga cac gac agc ggc tac gag gtg cac cac cag aag aag gac     245
   Glu Phe Arg His Asp Ser Gly Tyr Glu Val His His Gln Lys Lys Asp
               60                  65                  70
   gcc gag ttc aga cac gac agc ggc tac gag gtg cac cac cag aag aag     293
   Ala Glu Phe Arg His Asp Ser Gly Tyr Glu Val His His Gln Lys Lys
           75                  80                  85
   gac gcc gag ttc aga cac gac agc ggc tac gag gtg cac cac cag aag     341
   Asp Ala Glu Phe Arg His Asp Ser Gly Tyr Glu Val His His Gln Lys
       90                  95                  100
   aag gac gcc gag ttc aga cac gac agc ggc tac gag gtg cac cac cag     389
   Lys Asp Ala Glu Phe Arg His Asp Ser Gly Tyr Glu Val His His Gln
   105                 110                 115                 120
   aag aag gac gcc gag ttc aga cac gac agc ggc tac gag gtg cac cac     437
   Lys Lys Asp Ala Glu Phe Arg His Asp Ser Gly Tyr Glu Val His His
                   125                 130                 135
   cag aag aag gac gcc gag ttc aga cac gac agc ggc tac gag gtg cac     485
   Gln Lys Lys Asp Ala Glu Phe Arg His Asp Ser Gly Tyr Glu Val His
               140                 145                 150
   cac cag taagaattcc cgtagtaaga aaaacttagg gtgaaagttc atcgcggccg c    542
   His Gln
<210> 114
   <211> 154
   <212> PRT
   <213> Artificial
<220>
   <223> Synthetic Construct
<400> 114
   Met Ser Val Pro Thr Gln Val Leu Gly Leu Leu Leu Leu Trp Leu Thr
   1               5                   10                  15
   Asp Ala Arg Cys Asp Ala Glu Phe Arg His Asp Ser Gly Tyr Glu Val
               20                  25                  30
   His His Gln Lys Lys Asp Ala Glu Phe Arg His Asp Ser Gly Tyr Glu
           35                  40                  45
   Val His His Gln Lys Lys Asp Ala Glu Phe Arg His Asp Ser Gly Tyr
       50                  55                  60
   Glu Val His His Gln Lys Lys Asp Ala Glu Phe Arg His Asp Ser Gly
   65                  70                  75                  80
   Tyr Glu Val His His Gln Lys Lys Asp Ala Glu Phe Arg His Asp Ser
                   85                  90                  95
   Gly Tyr Glu Val His His Gln Lys Lys Asp Ala Glu Phe Arg His Asp
               100                 105                 110
   Ser Gly Tyr Glu Val His His Gln Lys Lys Asp Ala Glu Phe Arg His
           115                 120                 125
   Asp Ser Gly Tyr Glu Val His His Gln Lys Lys Asp Ala Glu Phe Arg
       130                 135                 140
   His Asp Ser Gly Tyr Glu Val His His Gln
   145                 150
<210> 115
   <211> 29
   <212> DNA
   <213> Artificial
<220>
   <223> an artificially synthesized sequence
<400> 115
   tggctgaccg atcccgggcc cgacgccga 29
<210> 116
   <211> 26
   <212> DNA
   <213> Artificial
<220>
   <223> an artificially synthesized sequence
<400> 116
   ttactacggg atatcttact ggtggt 26
<210> 117
   <211> 776
   <212> DNA
   <213> Artificial
<220>
   <223> an artificially synthesized sequence
<220>
   <221> CDS
   <222> (21)..(500)
<400> 117
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc       101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac      149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc      197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc      245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac      293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc      341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc      389
   Val Trp Asn Asn Lys Thr Pro His Ala Ile Ala Ala Ile Ser Met Ala
           110                 115                 120
   aac ggc ccc ggg ccc gac gcc gag ttc aga cac gac agc ggc tac gag      437
   Asn Gly Pro Gly Pro Asp Ala Glu Phe Arg His Asp Ser Gly Tyr Glu
       125                 130                 135
   gtg cac cac cag aag aag gac gcc gag ttc aga cac gac agc ggc tac      485
   Val His His Gln Lys Lys Asp Ala Glu Phe Arg His Asp Ser Gly Tyr
   140                 145                 150                 155
   gag gtg cac cac cag taagatgggc agacatgata agatacattg atgagtttgg      540
   Glu Val His His Gln
                   160
   acaaaccaca actagaatgc agtgaaaaaa atgctttatt tgtgaaattt gtgatgctat    600
   tgctttattt gtaaccatta taagctgcaa taaacaagtt aacaacaaca attgcattca    660
   ttttatgttt caggttcagg gggagatgtg ggaggttttt taaagcaagt aaaacctcta    720
   caaatgtggt agatccgtag taagaaaaac ttagggtgaa agttcatcgc ggccgc        776
<210> 118
   <211> 160
   <212> PRT
   <213> Artificial
<220>
   <223> Synthetic Construct
<400> 118
   Met Ile Lys Leu Lys Phe Gly Val Phe Phe Thr Val Leu Leu Ser Ser
   1               5                   10                  15
   Ala Tyr Ala His Gly Thr Pro Gln Asn Ile Thr Asp Leu Cys Ala Glu
               20                  25                  30
   Tyr His Asn Thr Gln Ile His Thr Leu Asn Asp Lys Ile Phe Ser Tyr
   35 40 45
   Thr Glu Ser Leu Ala Gly Lys Arg Glu Met Ala Ile Ile Thr Phe Lys
   50 55 60
   Asn Gly Ala Thr Phe Gln Val Glu Val Pro Gly Ser Gln His Ile Asp
   65 70 75 80
   Ser Gln Lys Lys Ala Ile Glu Arg Met Lys Asp Thr Leu Arg Ile Ala
   85 90 95
   Tyr Leu Thr Glu Ala Lys Val Glu Lys Leu Cys Val Trp Asn Asn Lys
   100 105 110
   Thr Pro His Ala Ile Ala Ala Ile Ser Met Ala Asn Gly Pro Gly Pro
   115 120 125
   Asp Ala Glu Phe Arg His Asp Ser Gly Tyr Glu Val His His Gln Lys
   130 135 140
   Lys Asp Ala Glu Phe Arg His Asp Ser Gly Tyr Glu Val His His Gln
   145 150 155 160
<210> 119
   <211> 878
   <212> DNA
   <213> Artificial
<220>
   <223> an artificially synthesized sequence
<220>
   <221> CDS
   <222> (21)..(602)
<400> 119
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc       101
   Val Leu Leu Ser Ser Ala Tyr Ala His Gly Thr Pro Gln Asn Ile Thr
               15                  20                  25
   gac ctg tgc gcc gag tac cac aac acc cag atc cac acc ctg aac gac       149
   Asp Leu Cys Ala Glu Tyr His Asn Thr Gln Ile His Thr Leu Asn Asp
           30                  35                  40
   aag atc ttc agc tac acc gag agc ctg gcc ggc aag aga gag atg gcc       197
   Lys Ile Phe Ser Tyr Thr Glu Ser Leu Ala Gly Lys Arg Glu Met Ala
       45                  50                  55
   atc atc acc ttc aag aac ggc gcc acc ttc cag gtg gag gtg ccc ggc      245
   Ile Ile Thr Phe Lys Asn Gly Ala Thr Phe Gln Val Glu Val Pro Gly
   60                  65                  70                  75
   tcc cag cac atc gac agc cag aag aag gcc atc gag aga atg aag gac      293
   Ser Gln His Ile Asp Ser Gln Lys Lys Ala Ile Glu Arg Met Lys Asp
                   80                  85                  90
   acc ctg cgg atc gcc tac ctg acc gag gcc aag gtg gag aag ctg tgc      341
   Thr Leu Arg Ile Ala Tyr Leu Thr Glu Ala Lys Val Glu Lys Leu Cys
               95                  100                 105
   gtg tgg aac aac aag acc ccc cac gcc atc gcc gcc atc agc atg gcc      389
   Val Trp Asn Asn Lys Thr Pro His Ala Ile Ala Ala Ile Ser Met Ala
           110                 115                 120
   aac ggc ccc ggg ccc gac gcc gag ttc aga cac gac agc ggc tac gag      437
   Asn Gly Pro Gly Pro Asp Ala Glu Phe Arg His Asp Ser Gly Tyr Glu
       125                 130                 135
   gtg cac cac cag aag aag gac gcc gag ttc aga cac gac agc ggc tac      485
   Val His His Gln Lys Lys Asp Ala Glu Phe Arg His Asp Ser Gly Tyr
   140                 145                 150                 155
   gag gtg cac cac cag aag aag gac gcc gag ttc aga cac gac agc ggc      533
   Glu Val His His Gln Lys Lys Asp Ala Glu Phe Arg His Asp Ser Gly
                   160                 165                 170
   tac gag gtg cac cac cag aag aag gac gcc gag ttc aga cac gac agc      581
   Tyr Glu Val His His Gln Lys Lys Asp Ala Glu Phe Arg His Asp Ser
               175                 180                 185
   ggc tac gag gtg cac cac cag taagatgggc agacatgata agatacattg         632
   Gly Tyr Glu Val His His Gln
           190
   atgagtttgg acaaaccaca actagaatgc agtgaaaaaa atgctttatt tgtgaaattt    692
   gtgatgctat tgctttattt gtaaccatta taagctgcaa taaacaagtt aacaacaaca    752
   attgcattca ttttatgttt caggttcagg gggagatgtg ggaggttttt taaagcaagt    812
   aaaacctcta caaatgtggt agatccgtag taagaaaaac ttagggtgaa agttcatcgc    872
   ggccgc                                                               878
<210> 120
   <211> 194
   <212> PRT
   <213> Artificial
<220>
   <223> Synthetic Construct
<400> 120
   Met Ile Lys Leu Lys Phe Gly Val Phe Phe Thr Val Leu Leu Ser Ser
   1 5 10 15
   Ala Tyr Ala His Gly Thr Pro Gln Asn Ile Thr Asp Leu Cys Ala Glu
   20 25 30
   Tyr His Asn Thr Gln Ile His Thr Leu Asn Asp Lys Ile Phe Ser Tyr
   35 40 45
   Thr Glu Ser Leu Ala Gly Lys Arg Glu Met Ala Ile Ile Thr Phe Lys
   50 55 60
   Asn Gly Ala Thr Phe Gln Val Glu Val Pro Gly Ser Gln His Ile Asp
   65 70 75 80
   Ser Gln Lys Lys Ala Ile Glu Arg Met Lys Asp Thr Leu Arg Ile Ala
   85 90 95
   Tyr Leu Thr Glu Ala Lys Val Glu Lys Leu Cys Val Trp Asn Asn Lys
   100 105 110
   Thr Pro His Ala Ile Ala Ala Ile Ser Met Ala Asn Gly Pro Gly Pro
   115 120 125
   Asp Ala Glu Phe Arg His Asp Ser Gly Tyr Glu Val His His Gln Lys
   130 135 140
   Lys Asp Ala Glu Phe Arg His Asp Ser Gly Tyr Glu Val His His Gln
   145 150 155 160
   Lys Lys Asp Ala Glu Phe Arg His Asp Ser Gly Tyr Glu Val His His
   165 170 175
   Gln Lys Lys Asp Ala Glu Phe Arg His Asp Ser Gly Tyr Glu Val His
   180 185 190
   His Gln
<210> 121
   <211> 1082
   <212> DNA
   <213> Artificial
<220>
   <223> an artificially synthesized sequence
<220>
   <221> CDS
   <222> (21)..(806)
<400> 121
   gcggccgctg gctagccacc atg atc aag ctg aag ttc ggc gtg ttc ttc acc      53
                         Met Ile Lys Leu Lys Phe Gly Val Phe Phe Thr
                         1               5                   10
   gtg ctg ctg agc agc gcc tac gcc cac ggc acc ccc cag aac atc acc 101
   Val Leu Leu Ser Ser Ala Tyr Ala His Gly Thr Pro Gln Asn Ile Thr
   15 20 25
   gac ctg tgc gcc gag tac cac aac acc cag atc cac acc ctg aac gac 149
   Asp Leu Cys Ala Glu Tyr His Asn Thr Gln Ile His Thr Leu Asn Asp
   30 35 40
   aag atc ttc agc tac acc gag agc ctg gcc ggc aag aga gag atg gcc 197
   Lys Ile Phe Ser Tyr Thr Glu Ser Leu Ala Gly Lys Arg Glu Met Ala
   45 50 55
   atc atc acc ttc aag aac ggc gcc acc ttc cag gtg gag gtg ccc ggc 245
   Ile Ile Thr Phe Lys Asn Gly Ala Thr Phe Gln Val Glu Val Pro Gly
   60 65 70 75
   tcc cag cac atc gac agc cag aag aag gcc atc gag aga atg aag gac 293
   Ser Gln His Ile Asp Ser Gln Lys Lys Ala Ile Glu Arg Met Lys Asp
   80 85 90
   acc ctg cgg atc gcc tac ctg acc gag gcc aag gtg gag aag ctg tgc 341
   Thr Leu Arg Ile Ala Tyr Leu Thr Glu Ala Lys Val Glu Lys Leu Cys
   95 100 105
   gtg tgg aac aac aag acc ccc cac gcc atc gcc gcc atc agc atg gcc 389
   Val Trp Asn Asn Lys Thr Pro His Ala Ile Ala Ala Ile Ser Met Ala
   110 115 120
   aac ggc ccc ggg ccc gac gcc gag ttc aga cac gac agc ggc tac gag 437
   Asn Gly Pro Gly Pro Asp Ala Glu Phe Arg His Asp Ser Gly Tyr Glu
   125 130 135
   gtg cac cac cag aag aag gac gcc gag ttc aga cac gac agc ggc tac 485
   Val His His Gln Lys Lys Asp Ala Glu Phe Arg His Asp Ser Gly Tyr
   140 145 150 155
   gag gtg cac cac cag aag aag gac gcc gag ttc aga cac gac agc ggc 533
   Glu Val His His Gln Lys Lys Asp Ala Glu Phe Arg His Asp Ser Gly
   160 165 170
   tac gag gtg cac cac cag aag aag gac gcc gag ttc aga cac gac agc 581
   Tyr Glu Val His His Gln Lys Lys Asp Ala Glu Phe Arg His Asp Ser
   175 180 185
   ggc tac gag gtg cac cac cag aag aag gac gcc gag ttc aga cac gac 629
   Gly Tyr Glu Val His His Gln Lys Lys Asp Ala Glu Phe Arg His Asp
   190 195 200
   agc ggc tac gag gtg cac cac cag aag aag gac gcc gag ttc aga cac 677
   Ser Gly Tyr Glu Val His His Gln Lys Lys Asp Ala Glu Phe Arg His
   205 210 215
   gac agc ggc tac gag gtg cac cac cag aag aag gac gcc gag ttc aga 725
   Asp Ser Gly Tyr Glu Val His His Gln Lys Lys Asp Ala Glu Phe Arg
   220 225 230 235
   cac gac agc ggc tac gag gtg cac cac cag aag aag gac gcc gag ttc 773
   His Asp Ser Gly Tyr Glu Val His His Gln Lys Lys Asp Ala Glu Phe
   240 245 250
   aga cac gac agc ggc tac gag gtg cac cac cag taagatgggc agacatgata 826
   Arg His Asp Ser Gly Tyr Glu Val His His Gln
   255 260
   agatacattg atgagtttgg acaaaccaca actagaatgc agtgaaaaaa atgctttatt 886
   tgtgaaattt gtgatgctat tgctttattt gtaaccatta taagctgcaa taaacaagtt 946
   aacaacaaca attgcattca ttttatgttt caggttcagg gggagatgtg ggaggttttt 1006
   taaagcaagt aaaacctcta caaatgtggt agatccgtag taagaaaaac ttagggtgaa 1066
   agttcatcgc ggccgc 1082
<210> 122
   <211> 262
   <212> PRT
   <213> Artificial
<220>
   <223> Synthetic Construct
<400> 122
   Met Ile Lys Leu Lys Phe Gly Val Phe Phe Thr Val Leu Leu Ser Ser
   1 5 10 15
   Ala Tyr Ala His Gly Thr Pro Gln Asn Ile Thr Asp Leu Cys Ala Glu
   20 25 30
   Tyr His Asn Thr Gln Ile His Thr Leu Asn Asp Lys Ile Phe Ser Tyr
   35 40 45
   Thr Glu Ser Leu Ala Gly Lys Arg Glu Met Ala Ile Ile Thr Phe Lys
   50 55 60
   Asn Gly Ala Thr Phe Gln Val Glu Val Pro Gly Ser Gln His Ile Asp
   65 70 75 80
   Ser Gln Lys Lys Ala Ile Glu Arg Met Lys Asp Thr Leu Arg Ile Ala
   85 90 95
   Tyr Leu Thr Glu Ala Lys Val Glu Lys Leu Cys Val Trp Asn Asn Lys
   100 105 110
   Thr Pro His Ala Ile Ala Ala Ile Ser Met Ala Asn Gly Pro Gly Pro
   115 120 125
   Asp Ala Glu Phe Arg His Asp Ser Gly Tyr Glu Val His His Gln Lys
   130 135 140
   Lys Asp Ala Glu Phe Arg His Asp Ser Gly Tyr Glu Val His His Gln
   145 150 155 160
   Lys Lys Asp Ala Glu Phe Arg His Asp Ser Gly Tyr Glu Val His His
                   165                 170                 175
   Gln Lys Lys Asp Ala Glu Phe Arg His Asp Ser Gly Tyr Glu Val His
               180                 185                 190
   His Gln Lys Lys Asp Ala Glu Phe Arg His Asp Ser Gly Tyr Glu Val
           195                 200                 205
   His His Gln Lys Lys Asp Ala Glu Phe Arg His Asp Ser Gly Tyr Glu
       210                 215                 220
   Val His His Gln Lys Lys Asp Ala Glu Phe Arg His Asp Ser Gly Tyr
   225                 230                 235                 240
   Glu Val His His Gln Lys Lys Asp Ala Glu Phe Arg His Asp Ser Gly
                   245                 250                 255
   Tyr Glu Val His His Gln
               260

## Description

### Technical Field

The present invention relates to methods for enhancing the expression of a recombinant protein from an RNA viral vector, methods for preparing recombinant proteins useful for pharmaceuticals and the like by using the above-described method, RNA viral vectors that express the recombinant proteins, and such.

### Background Art

Many recombinant proteins and peptides are currently used as pharmaceutical compositions. It is thought that the type and number of biopharmaceuticals will increase in the future, and that they will form a larger part of pharmaceuticals. Many bioactive proteins and peptides have been suggested as candidates for pharmaceutical compositions. In such cases, the availability and applicability of proteins and peptides can be greatly improved when their preparation is simplified by increasing their expression levels.

The same is also applicable to gene transfer RNA viral vectors used in gene therapy or such. When a gene transfer RNA viral vector is used to apply a gene to gene therapy, genetic vaccine, monoclonal antibody preparation or the like, the expression level of the inserted gene is directly linked to the vector function. Thus, the vector performance can be improved by increasing the expression level, which leads to great improvement of the availability and applicability of proteins.

The AB5 toxin produced by bacteria is a proteinaceous exotoxin produced by toxin-producing bacteria and secreted to the outside of bacterial cells. The AB5 toxin consists of one active subunit (A subunit) having toxicity and five binding subunits (B subunits) having cell-binding activity. Cholera toxin is an AB5 toxin. It has been reported that the nontoxic cholera toxin B subunit (CTB) is applied to infection and chronic diseases as a molecular adjuvant, for example, for influenza virus vaccine (Isaka M. et al., Microbiol. Immunol. 52(2): 55-63,2008), pertussis vaccine (Isaka M. et al., Vaccine, 21(11-12): 1165-73, 2003), respiratory syncytial (RS) virus vaccine (Singh S. R. et al., Viral Immunol. 20(2): 261-75, 2007; Oien N. L. et al., Vaccine, Jun. 12(8): 731-5, 1994), cancer vaccine (Wakabayashi A. et al., J Immunol. 180(6): 4000-10, 2008), and Alzheimer's disease vaccine (Maier M. et al., Vaccine, 23(44): 5149-59, 2005). However, there is no report that describes expressing a recombinant protein by using an RNA viral vector or enhancing the expression of a recombinant protein by using an RNA viral vector.

### Prior Art Documents

### [Non-patent Documents]

[Non-patent Document 1] Isaka M. et al., Microbiol Immunol. 2008; 52(2): 55-63
[Non-patent Document 2] Isaka M. et al., Vaccine. 2003; 21(11-12): 1165-73
[Non-patent Document 3] Singh S.R. et al., Viral Immunol. 2007; 20(2): 261-75
[Non-patent Document 4] Oien N.L. et al., Vaccine. 1994 Jun; 12(8): 731-5
[Non-patent Document 5] Wakabayashi A. et al., J Immunol. 2008; 180(6): 4000-10
[Non-patent Document 6] Maier M. et al., Vaccine. 2005; 23(44): 5149-59

### Disclosure of the Invention

### [Problems to be Solved by the Invention]

The present invention was achieved in view of the above circumstances. An objective of the present invention is to provide methods for enhancing the expression of recombinant proteins and peptides that are useful as pharmaceutical compositions from RNA viral vectors to simplify the preparation of recombinant proteins and peptides, and provide more effective gene transfer vectors that yield high expression of therapeutic genes by introducing the above-described technology into RNA virus-derived gene transfer vectors when applied in gene therapy, gene vaccination, monoclonal antibody preparation, and such.

### [Means for Solving the Problems]

The present inventors revealed that when a protein or peptide is expressed from RNA virus, there are not a few cases where the protein or peptide expression level is low and its preparation is difficult due to the property of the protein or peptide to be expressed. In particular, the present inventors found that the expression efficiency is extremely low when short peptides, proteins with low solubility, or such are expressed from RNA viral vectors.

Meanwhile, the AB toxin B subunit (AB5B) is a protein of more than ten KD. Its three-dimensional structural analysis has demonstrated that it forms a pentamer (McCann J.A. et al., Biochemistry. 36(30): 9169-78, 1997; Pickens J.C. et al., Chem Biol. 11(9): 1205-1215, 2004). Cholera toxin B (CTB), which is an AB5B, has been used as an adjuvant. However, it is not known whether the level of protein expression is altered when using CTB. During the course of expressing a recombinant protein as a CTB fusion protein, the present inventors found that the expression level was significantly increased when the recombinant protein was expressed as a CTB fusion protein using a Sendai virus vector as compared to when expressing the recombinant protein alone.

To analyze this phenomenon more closely, the present inventors selected various types of recombinant proteins as an example, and assessed the change (increase or decrease) in their expression levels by fusing them with AB5B. Furthermore, to evaluate the difference between AB5B and other carrier proteins (fusion proteins), the receptor-binding domain (Ia) of Pseudomonas exotoxin A (PEDI) and interleukin-4 (IL-4) were selected and their fusion with AB5B was compared.

The obtained result was highly convincing. All peptides tested, including corticotropin releasing factor (CRF), endothelin-1 (ET-1), neuropeptide Y (NPY), glucagon-like peptide-2 (GLP-2), and amyloid β-42 (Aβ42: amyloid β), whose sizes are 120 amino acids or less, showed an markedly increased level of expression (several dozen fold or more) when fused with AB5B. This demonstrates that the level of expression from an RNA viral vector is significantly increased by fusing with AB5B. Furthermore, comparison of AB5B with other carrier protein such as PEDI and IL-4 showed that AB5B had a significantly greater expression-enhancing effect. It is highly important that AB5B functions more effectively than globular proteins such as IL-4.

When expressed from an RNA viral vector, proteins of a relatively short chain length are expressed only with very poor expression efficiency. Thus, it is difficult to express such proteins at sufficient levels. However, the expression levels of even proteins of a short chain length can be markedly increased by expressing them as an AB5B fusion protein based on the present invention.

Increasing the expression level of a recombinant protein is beneficial for preparing the recombinant protein itself. When such a recombinant protein/peptide is expressed, it may be used without any modification in some cases. However, AB5B may be preferably removed in other cases. In such cases, AB5B can be cleaved off by introducing in advance a protease cleavage sequence such as an enterokinase recognition sequence (DDDDK↓), a Factor Xa recognition sequence (IEGR↓), a thrombin recognition sequence (LVPR↓GS), or an HRV 3C protease recognition sequence (LEVLFQ↓GP) between AB5B and a recombinant protein/peptide of interest. Alternatively, cholera toxin (CTB) can be affinity-purified by using a Ni column, because it exhibits weak affinity for Ni column (M.T. Dertzbaugh and L.M. Cox, Protein Engineering, 11: 577-581, 1998; US Pat. No. 6008329).

Furthermore, fusion with AB5B is an effective way to enhance the *in vivo*, *ex vivo,* or *in vitro* expression of a recombinant protein in gene therapy, gene vaccination, monoclonal antibody preparation, or the like using a gene transfer RNA viral vector. The enhanced expression is directly linked to vector function, and thus can increase vector performance.

Specifically, the present invention relates to methods for enhancing the expression of a recombinant protein from an RNA viral vector by fusing the recombinant protein with an AB5B protein, methods for preparing a recombinant protein useful as a pharmaceutical by using the above-described methods, RNA viral vectors encoding an AB5B fusion protein, and such.

The present invention is characterized by the embodiments as defined in the claims. Accordingly, it relates to the following items:
1. A minus-strand RNA viral vector encoding a recombinant protein, wherein the protein is encoded as a fusion protein with an AB5 toxin B subunit.
2. A method for producing a minus-strand RNA viral vector with enhanced capacity for expressing a recombinant protein, which comprises the step of producing an RNA viral vector encoding a fusion protein in which the recombinant protein is fused with an AB5 toxin B subunit.
3. A method for increasing the expression level of a recombinant protein from a minus-strand RNA viral vector, which comprises the step of expressing the recombinant protein as a fusion protein with an AB5 toxin B subunit from an RNA viral vector.
4. The vector of item 1 or the method of item 2 or 3, wherein the AB5 toxin B subunit is cholera toxin B (CTB).
5. The vector of item 1 or 4 or the method of any one of item 2 to 4, wherein the recombinant protein is a peptide of 120 amino acids or less.
6. The vector of item 5 or the method of item 5, wherein the recombinant protein is a peptide of 50 amino acids or less.
7. The vector of any one of items 1 and 4 to 6 or the method of any one of items 2 to 6, wherein the recombinant protein is a neuropeptide or endocrine peptide.
8. The vector of any one of items 1 and 4 to 7 or the method of any one of items 2 to 7, wherein the recombinant protein is a protein of low solubility.
9. A pharmaceutical composition comprising the vector of any one of items 1 and 4 to 8 and a pharmaceutically acceptable carrier.
10. A cell introduced with the vector of any one of items 1 and 4 to 8.
11. A method for producing a recombinant protein, which comprises the step of expressing the vector of any one of items 1 and 4 to 8.
12. A virus-like particle comprising the vector of any one of items 1 and 4 to 8.

More specifically, the present invention relates to:
[1] an RNA viral vector encoding a recombinant protein, wherein the protein is encoded as a fusion protein with an AB5 toxin B subunit;
[2] the vector of [1], wherein the AB5 toxin B subunit is cholera toxin B (CTB);
[3] the vector of [1] or [2], wherein the recombinant protein is a peptide of 120 amino acids or less;
[4] the vector of [3], wherein the recombinant protein is a peptide of 50 amino acids or less;
[5] the vector of any one of [1] to [4], wherein the recombinant protein is a neuropeptide or endocrine peptide;
[6] the vector of any one of [1] to [5], wherein the recombinant protein is a protein of low solubility;
[7] the vector of any one of [1] to [6], wherein the RNA viral vector is a minus-strand RNA viral vector;
[8] a pharmaceutical composition comprising the vector of any one of [1] to [7] and a pharmaceutically acceptable carrier;
[9] a cell introduced with the vector of any one of [1] to [7];
[10] a method for producing a recombinant protein, which comprises the step of expressing the vector of any one of [1] to [7];
[11] a method for producing an RNA viral vector with enhanced capacity for expressing a recombinant protein, which comprises the step of producing an RNA viral vector encoding a fusion protein in which the recombinant protein is fused with an AB5 toxin B subunit;
[12] the method of [11], wherein the AB5 toxin B subunit is cholera toxin B (CTB);
[13] the method of [11] or [12], wherein the recombinant protein is a peptide of 120 amino acids or less;
[14] the method of [13], wherein the recombinant protein is a peptide of 50 amino acids or less;
[15] the method of any one of [11] to [14], wherein the recombinant protein is a neuropeptide or endocrine peptide;
[16] the method of any one of [1] to [15], wherein the recombinant protein is a protein of low solubility;
[17] the method of any one of [1] to [16], wherein the RNA viral vector is a minus-strand RNA viral vector;
[18] a method for increasing the expression level of a recombinant protein from an RNA viral vector, which comprises the step of expressing the recombinant protein as a fusion protein with an AB5 toxin B subunit from an RNA viral vector;
[19] the method of [18], wherein the AB5 toxin B subunit is cholera toxin B (CTB);
[20] the method of [18] or [19], wherein the recombinant protein is a peptide of 120 amino acids or less;
[21] the method of [20], wherein the recombinant protein is a peptide of 50 amino acids or less;
[22] the method of any one of [18] to [21], wherein the recombinant protein is a neuropeptide or endocrine peptide;
[23] the method of any one of [18] to [22], wherein the recombinant protein is a protein with low solubility;
[24] the method of any one of [18] to [23], wherein the RNA viral vector is a minus-strand RNA viral vector;
[25] a virus-like particle comprising the vector of any one of [1] to [7]; and such.

It is intended that in each of the claims described above, inventions comprising any combination of two or more inventions described in each claim that recites the same claim are also included in the antecedent claim recited. Furthermore, it is intended that any inventive elements and technical elements described herein, and any combinations thereof are also included in the present invention. In addition, it is intended that any inventions excluding any elements described herein or any combinations thereof are also included in the present invention. Herein, for example, when a specific embodiment is stated as "preferable", the specification discloses not only the embodiment itself, but also inventions that exclude the embodiment from the disclosed antecedent inventions comprising the embodiment.

### [Effects of the Invention]

The present invention enables one to increase the expression level of a recombinant protein from an RNA viral vector. Among recombinant proteins, in particular, peptides that are only expressed at a low level from an RNA viral vector, such as short peptide proteins and proteins with low solubility, can be effectively expressed at increased expression levels from the RNA viral vector by using the present invention. Furthermore, the RNA viral vectors of the present invention can be used as gene transfer vectors to achieve effective gene therapy, gene vaccination, monoclonal antibody preparation, and such.

### Brief Description of the Drawings

Fig. 1 is a schematic diagram showing the structures of SeV cDNAs (pSeV18+CTB-mCRF/ΔF, pSeV18+CTB-mETI/ΔF, pSeV18+CTB-mPYY/ΔF, pSeV18+CTB-mGLP2/ΔF, pSeV18+mCRF/ΔF, pSeV18+mET1/ΔF, pSeV18+mPYY/ΔF, and pSeV18+mGLP2/ΔF). CTB-mCRF, CTB-mET1, CTB-mPYY, CTB-mGLP2, mCRF, mET1, mPYY, mGLP2 genes were each excised from pCI at their *Not*I sites and then inserted into the *Not*I site of pSeV18+NotI/ΔF.
Fig. 2 is a schematic diagram showing the structure of the Aβ42 *Not*I fragment.
Fig. 3 is a schematic diagram showing construction of the Aβ42 *Not*I fragment.
Fig. 4 is a schematic diagram showing the structure of the CTB-Aβ42 *Not*I fragment.
Fig. 5 is a diagram showing the expression quantity of CTB-mCRF and mCRF in cell lysates and culture supernatants of BHK21 cells.
Fig. 6 is a diagram showing the expression quantity of CTB-mET1 and mET1 in cell lysates and culture supernatants of BHK21 cells.
Fig. 7 is a diagram showing the expression quantity of CTB-mPYY and mPYY in cell lysates and culture supernatants of BHK21 cells.
Fig. 8 is a diagram showing the expression quantity of CTB-mGLP-2 and mGLP-2 in cell lysates and culture supernatants of BHK21 cells.
Fig. 9 is a diagram showing the expression quantity of Aβ42, IL-4-Aβ42, PEDI-Aβ42, and CTB-Aβ42 in cell lysates and culture supernatants of BHK21 cells.
Fig. 10 is a schematic diagram showing construction of the CTB-Aβ15 *NotI* fragment.
Fig. 11 is a schematic diagram showing construction of the CTB-Aβ15x2, CTB-Aβ 15x4, and CTB-Aβ 15x8 *NotI* fragments.
Fig. 12 is a Western blot photograph showing the amount of Aβ antigen in cell lysates and culture supernatants of BHK cells infected with an SeV vector carrying the CTB-Aβ42, CTB-Aβ 15, CTB-Aβ15x2, CTB-Aβ 15x4, or CTB-Aβ 15x8 gene.
Fig. 13 is a diagram showing the GM1-binding activity in cell lysates and culture supernatants of BHK cells infected with an SeV vector carrying the CTB-Aβ42, CTB-Aβ 15, CTB-Aβ15x2, CTB-Aβ 15x4, or CTB-Aβ 15x8 gene.
Fig. 14 is a diagram showing the titer of anti-Aβ antibody in C57BL/6N mice intramuscularly administered with an SeV vector carrying the CTB-Aβ42, CTB-Aβ 15x8, or GFP gene.
Fig. 15 is a diagram showing the titer of anti-Aβ antibody in C57BL/6N mice intramuscularly, intradermally, or intranasally administered with an SeV vector carrying the CTB-Aβ15x8 gene.
Fig. 16 is a diagram showing the titer of anti-Aβ antibody in C57BL/6N mice intranasally administered with an SeV vector carrying the CTB-Aβ 15, CTB-Aβ15x2, CTB-Aβ15x4, or CTB-Aβ15x8 gene.
Fig. 17 is a graph showing the titer of anti-Aβ antibody in C57BL/6N mice intramuscularly administered with an SeV vector carrying the CTB-Aβ42 gene and then subjected to booster immunization with the CTB-Aβ42 protein purified from E. *coli.*
Fig. 18 is a graph showing the titer of anti-Aβ antibody in C57BL/6N mice intramuscularly administered with an SeV vector carrying the CTB-Aβ15x8 gene and then subjected to booster immunization with the same SeV vector.
Fig. 19 shows in graphs the titer of anti-Aβ antibody in C57BL/6N mice intramuscularly administered with an SeV vector carrying the CTB-Aβ42 or CTB-Aβ15x8 gene and then subjected to booster immunization with the same SeV vector. Panel B shows only results obtained by using the SeV vector carrying the CTB-Aβ42 gene shown in panel A.
Fig. 20 shows in graphs the titer of anti-Aβ antibody in C57BL/6N mice intranasally administered with an SeV vector carrying the CTB-Aβ15x8 gene and then subjected to booster immunization with the same SeV vector.
Fig. 21 shows in a graph the titer of anti-Aβ antibody in Tg2576 mice intramuscularly administered with an SeV vector carrying the CTB-Aβ15x8, CTB-Aβ42, or GFP gene and then subjected to booster immunization with the CTB-Aβ42 protein.
Fig. 22 shows in diagrams the intracerebral quantity of Aβ in Tg2576 mice intramuscularly administered with an SeV vector carrying the CTB-Aβ15x8, CTB-Aβ42, or GFP gene and then subjected to booster immunization with the CTB-Aβ42 protein.
Fig. 23 shows in photographs the distribution of areas of positive 6E10 immunostaining in histopathological brain samples. In the control SeV18+GFP/ΔF group (A), many dispersed Aβ deposits (brown) positive for 6E10 staining are seen in the olfactory bulb, hippocampus, and cerebral neocortex. In contrast, the number of Aβ deposits is clearly smaller in the SeV 18+CTB-Aβ15x8/ΔF administration group (B).
Fig. 24 is a graph of image analysis of samples immunostained with 6E10.
In the SeV18+CTB-Aβ15x8/ΔF administration group (right), there is an apparent reducing trend of percent area in various brain regions as compared to the control SeV 18+GFP/ΔF group (left). In particular, there is a great difference in the hippocampus.

### Made for Carrying Out the Invention

The present invention relates to RNA viral vectors encoding a recombinant protein, wherein the recombinant protein is encoded as a fusion protein with an AB5 toxin B subunit (AB5B) protein, methods for producing the vectors, compositions comprising such vectors, methods for increasing the expression level of a recombinant protein using the vectors, and such. The present inventors revealed that the expression of a protein of interest could be significantly enhanced by using a nucleic acid encoding AB5B in combination with an RNA viral vector, Specifically, the expression level of a protein of interest from an RNA viral vector can be markedly increased by expressing the protein as an AB5B-fusion protein.

Herein, the RNA virus refers to a virus that has RNA genome and does not have any DNA phase in its life cycle. The RNA virus of the present invention has no reverse transcriptase (specifically, the RNA virus does not include retroviruses). Specifically, the viral propagation is achieved by replication of viral genome using RNA-dependent RNA polymerase and is not mediated by DNA. The RNA virus includes single-stranded RNA viruses (including both plus- and minus-strand RNA viruses) and double-stranded RNA viruses. The RNA viruses include viruses with envelopes (enveloped viruses) and viruses without envelope (non-enveloped viruses). Vectors derived from an enveloped virus are preferred. Specifically, the RNA viruses of the present invention include viruses belonging to the following viral families:
*Arenaviridae,* including Lassa virus;
*Orthomyxoviridae,* including influenza viruses (including Influenza virus A, B, and C, and Thogoto-like viruses);
*Coronaviridae,* including SARS virus;
*Togaviridae,* including rubella virus;
*Paramyxoviridae,* including mumps virus, measles virus, Sendai virus, and RS virus; *Picornaviridae,* including poliovirus, Coxsackievirus, and echovirus;
*Filoviridae,* including Marburg virus and Ebola virus;
*Flaviviridae,* including yellow fever virus, dengue fever virus, hepatitis C virus, and hepatitis G virus;
*Bunyaviridae* (including the genera Bunyavirus, Hantavirus, Nairovirus, Phlebovirus, etc.); *Rhabdoviridae,* including rabies virus; and
*Reoviridae.*

Herein, the viral vector refers to a vector (vehicle) that has a genomic nucleic acid derived from a virus and is used to express a gene inserted into the genomic nucleic acid. The viral vector of the present invention also includes complexes such as infectious virions, viral core, complex of viral genome and proteins, and non-infectious particles (non-infectious virions or virus-like particles), which have an ability to express inserted genes when they are introduced into cells. For example, in the case of an RNA virus, the ribonucleoprotein (the viral core) consisting of the viral genome and viral proteins that bind to the genome is capable of expressing an inserted gene in cells when it is introduced into the cells (WO 00/70055). Such ribonucleoproteins (RNPs) are also included in the viral vectors of the present invention. The vectors can be appropriately introduced into cells by using transfection reagents or the like.

Specific methods for introducing into cells RNPs that contain viral genomic RNAs or non-infectious viral particles (virus-like particles (VLPs)) include those known to those skilled in the art, such as methods that utilize calcium phosphate (Chen, C. & Okayama, H. BioTechniques 6: 632-638, 1988; Chen, C. and Okayama, H., Mol. Cell. Biol. 7: 2745, 1987), DEAE-dextran (Rosenthal, N. Methods Enzymol. 152: 704-709, 1987), various liposome-based transfection reagents (Sambrook, J. et al. (1989) Molecular Cloning: A Laboratory Manual (Cold Spring Harbor Laboratory, Cold Spring Harbor, NY)), or electroporation (Ausubel, F. et al. (1994) In Current Protocols in Molecular Biology (John Wiley and Sons, NY), Vol. 1, Ch. 5 and 9). Chloroquine may be added to the transfection to suppress the degradation in endosomes (Calos, M. P., Proc. Natl. Acad. Sci. USA 80: 3015, 1983). Transfection reagents include, for example, DOTMA (Roche), Superfect Transfection Reagent (QIAGEN, Cat No. 301305), DOTAP, DOPE, DOSPER (Roche #1811169), TransIT-LT1 (Mirus, Product No. MIR 2300), CalPhos^{™} Mammalian Transfection Kit (Clontech #K2051-1), and CLONfectin^{™} (Clontech #8020-1). Enveloped viruses are known to incorporate host cell-derived proteins during virion formation, and such proteins can potentially cause antigenicity and cytotoxicity when introduced into cells (J. Biol. Chem. 272: 16578-16584, 1997). It is thus advantageous to use RNPs without the envelope (WO 00/70055).

Preferred RNA viral vectors of the present invention include, for example, minus-strand RNA viral vectors. A minus-strand RNA viral vector refers to a viral vector derived from a virus containing a minus-strand (an antisense strand that encodes viral proteins) RNA as the genome. The minus-strand RNA is also referred to as negative strand RNA. In particular, the minus-strand RNA viruses of the present invention include single-stranded minus-strand RNA viruses (also referred to as non-segmented minus-strand RNA viruses). The "single-stranded negative-strand RNA virus" refers to viruses having a single-stranded negative strand (i.e., a minus strand) RNA as the genome. Such viruses include viruses belonging to *Paramyxoviridae* (including the genera Paramyxovirus, Morbillivirus, Rubulavirus, and Pneumovirus), *Rhabdoviridae* (including the genera Vesiculovirus, Lyssavirus, and Ephemerovirus), *Filoviridae,* and taxonomically belong to *Mononegavirales* (Virus, Vol. 57(1): 29-36, 2007; Annu. Rev. Genet. 32: 123-162, 1998; Fields Virology Fourth edition, Philadelphia, Lippincott-Raven, 1305-1340, 2001; Microbiol. Immunol. 43: 613-624, 1999; Field Virology, Third edition pp. 1205-1241, 1996).

In particular, the minus-strand RNA viral vectors of the present invention include paramyxovirus vectors. The paramyxovirus vectors are viral vectors derived from a virus belonging to *Paramyxoviridae.* Such viruses include, for example, Sendai virus belonging to *Paramyxoviridae.* The viruses also include, for example, Newcastle disease virus, mumps virus, measles virus, respiratory syncytial (RS) virus, rinderpest virus, distemper virus, simian parainfluenza virus (SV5), human parainfluenza viruses 1, 2, and 3; influenza virus belonging to *Orthomyxoviridae;* and vesicular stomatitis virus and rabies virus belonging to *Rhabdoviridae.*

The viruses that can be used in the present invention also include, for example, Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b), mumps virus (Mumps), and Newcastle disease virus (NDV). More preferably, the viruses of the present invention include viruses selected from the group consisting of: Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), and Nipah virus (Nipah).

The vectors used in the present invention include viruses belonging to *Paramyxovirinae* (including the genera Respirovirus, Rubulavirus, and Morbillivirus) or derivatives thereof, for example, those belonging to the genus Respirovirus (also referred to as the genus Paramyxovirus) or derivatives thereof. The derivatives include viruses that are genetically-modified or chemically-modified in a manner not to impair their gene-transferring ability. Examples of viruses of the genus Respirovirus applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10).

The RNA viral vectors may be derived from natural strains, wild-type strains, mutant strains, laboratory-passaged strains, artificially constructed strains, or the like. The viral vectors of the present invention may be transmissible or non-transmissible. Herein, transmissibility means that when a viral vector infects a host cell, the virus is replicated in the cell to produce infectious virions. The viral vectors may be those having the same structure as that of a virus isolated from a natural source or modified artificially by genetic recombination. For example, such viral vectors may have mutations or deletions in some genes of a wild-type virus. Alternatively, it is possible to use incomplete viruses such as DI particles (J. Virol. 68: 8413-8417, 1994). For example, it is also preferred to use viruses having a mutation or deletion in at least one of the genes encoding the viral envelope proteins or coat proteins. Such a viral vector can replicate its genome, for example, in infected cells, but it cannot form infectious virions. Such replication-deficient viral vectors are highly safe, because there is no risk of spreading the infection. For example, it is possible to use minus-strand RNA viral vectors that lack at least one of the genes encoding envelope proteins such as F, H, HN, G, M, and M1, or spike proteins, or an arbitrary combination of two or more, three or more, or four or more of them (WO 00/70055; WO 00/70070; Li, H.-O. et al., J. Virol. 74(14): 6564-6569, 2000). When the genomic RNA encodes proteins required for genomic replication (for example, N, P, and L proteins), the genome can be amplified in infected cells. To produce defective viruses, for example, some genes are deleted from the viral genome, and deficient gene products or proteins capable of complementing them are exogenously supplied to virus-producing cells (WO 00/70055; WO 00/70070; Li, H.-O. et al., J. Virol. 74(14): 6564-6569, 2000). There is also a known method for harvesting such viral vectors as non-infectious virions (VLP) without completely complementing the deficient viral proteins (WO 00/70070). Alternatively, by harvesting RNP (for example, RNP consisting ofN, L, and P proteins, and genomic RNA), the vectors can be produced without complementing the envelope proteins.

Meanwhile, when enveloped virus-derived viral vectors are prepared, viral vectors that contain in their envelope proteins different from the original viral envelope proteins can be produced. Viruses containing a desired foreign envelope protein can be produced, for example, by expressing the protein in virus-producing cells at the time of virus production. Such proteins are not particularly limited, and it is possible to use any desired protein such as adhesion factor, ligand, or receptor that confers infectivity to mammalian cells. Specifically, for example, the proteins include vesicular stomatitis virus (VSV) G protein (VSV-G). The VSV-G protein may be derived from any VSV strain, and includes, but is not limited to, for example, VSV-G protein derived from the Indiana serotype strain (J. Virology 39: 519-528, 1981).

The RNA viral vectors of the present invention encode a recombinant protein in a form of a fusion protein with AB5 toxin B subunit. Herein, the AB5 toxin refers to a toxin shared by many pathogenic bacteria, which consists of a single copy of A subunit and five copies of B subunit (Merritt E and Hol W. "AB5 toxins", Curr Opin Struct Biol 5(2): 165-71, 1995; Lencer W and Saslowsky D "Raft trafficking of AB5 subunit bacterial toxins", Biochim Biophys Acta 1746(3): 314-21, 2005). Such AB5 toxin includes, for example, enterotoxin of *Campylobacter jejuni,* cholera toxin (*Vibrio cholerae*), heat-labile enterotoxin (for example, LT and LT-II) (*Escherichia coli*), pertussis toxin (*Bordetella pertussis*), shiga toxin (*Shigella dysenteriae*), and shiga-like toxin and verotoxin produced by other enterohemorrhagic bacteria. In general, the A subunit is responsible for the toxicity of these toxins, while the B subunit forms a pentamer involved in bacterial adhesion to cells.

The particularly preferred AB5 toxin of the present invention includes cholera toxin and *E. coli* heat-labile enterotoxin, and they are structurally and functionally similar in the extreme to each other (Hovey BT et al., J Mol Biol., 285(3): 1169-78, 1999; Ricci S. et al., Infect Immun. 68(2): 760-766, 2000; Tinker J. K. et al., Infect Immun. 73(6): 3627-3635, 2005). Specifically, the B subunit of cholera toxin or *E. coli* heat-labile enterotoxin includes proteins comprising a protein of accession numbers ZP_01954889. 1, ZP_01976878.1, NP_231099.1, P13811.1, ABV01319.1, or P32890, protein of SEQ ID NO: 68, or the mature form thereof (lacking the N terminal 21 amino acids; for example, amino acids of positions 22 to 124). Nucleotide sequences encoding such a protein include those comprising the nucleotide sequence of NZ_AAWE01000267.1, NC_002505.1, M17874.1, EU113246.1, or M17873.1, or the sequence encoding a mature protein thereof (for example, lacking the 5'-most 63 nucleotides). The preferred AB5 toxin B subunit of the present invention includes those comprising an amino acid sequence described above or an amino acid sequence encoded by a nucleotide sequence described above, and those exhibiting high similarity to an above-described amino acid sequence.

Such AB5 toxin B subunit may comprise not only a naturally occurring sequence but also mutations. It is possible to use a B subunit comprising an amino acid sequence, for example, with an addition, deletion, substitution, and/or insertion of one or a small number of amino acids (for example, several amino acids, 3 amino acids or less, 5 amino acids or less, 10 amino acids or less, 15 amino acids or less, or 20 amino acids or less), as long as it does not significantly reduce the expression of the fusion protein, as compared to when using the naturally-occurring B subunit. It is also possible to use polypeptides with an N- and/or C-terminal amino acid deletion or addition of one to several residues (for example, 2, 3, 4, 5, 6, 10, 15, or 20 residues) and polypeptides with an amino acid substitution of one to several residues (for example, 2, 3, 4, 5, 6, 10, 15, or 20 residues). Usable variants include, for example, fragments, analogues, derivatives of a naturally occurring protein, and fusion proteins of a naturally occurring protein with other polypeptides (for example, those additionally containing a heterologous signal peptide or antibody fragment). Specifically, polypeptides that comprise a sequence with a substitution, deletion, and/or addition of one or more amino acids in the amino acid sequence of a wild-type B subunit and have an activity comparable to the wild-type B subunit to increase the expression of the fused recombinant protein, can be used as AB5 toxin B subunit of the present invention. Such modified AB5 toxin B subunits preferably retain the activity of forming a pentamer. When a fragment of a wild-type protein is used, it typically comprises a continuous region of 70% or more, preferably 80% or more, and more preferably 90% or more of the wild-type polypeptide (in its mature form when it is a secretory protein).

Amino acid sequence variants can be prepared, for example, by introducing mutations into DNAs that encode naturally occurring polypeptides (Walker and Gaastra, eds. (1983) Techniques in Molecular Biology (MacMillan Publishing Company, New York); Kunkel Proc. Natl. Acad. Sci. USA 82: 488-492, 1985; Kunkel et al., Methods Enzymol. 154: 367-382, 1987; Sambrook et al. (1989) Molecular Cloning: A Laboratory Manual (Cold Spring Harbor Laboratory Press, Plainview, N.Y.); U.S. Patent No. 4,873,192). Guidance for substituting amino acids without affecting the polypeptide's biological activity includes, for example, Dayhoff et al. (1978) in Atlas of Protein Sequence and Structure (Natl. Biomed. Res. Found., Washington D.C.)). AB5B includes, for example, the R192G mutant of *E. coli* enterotoxin LT (Lemere et al., Neurobiol. Aging 23: 991-1000, 2002; Seabrook et al., Neurobiol. Aging 25: 1141-1151, 2004; Seabrook et al., Vaccine 22: 4075-7483, 2004).

The number of amino acids to be modified is not particularly limited; however, it is, for example, 30% or less of the total amino acids in the mature form of a naturally occurring polypeptide, preferably 25% or less, more preferably 20% or less, even more preferably 15% or less, and still more preferably 10% or less, 5% or less, 3% or less, or 1% or less, and for example, 15 amino acids or less, preferably 10 amino acids or less, even more preferably 8 amino acids or less, still more preferably 5 amino acids or less, and yet more preferably 3 amino acids or less. In amino acid substitution, the original activity of a protein is expected to be retained by substituting amino acids having side chains with similar properties. Herein, this type of substitution is referred to as conservative substitution. Conservative substitutions include substitutions between amino acids within the same group, such as basic amino acids (for example, lysine, arginine, and histidine), acidic amino acids (for example, aspartic acid and glutamic acid), non-charged polar amino acids (for example, glycine, asparagine, glutamine, serine, threonine, tyrosine, and cysteine), nonpolar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine). Conservative substitutions also include, for example, substitutions between amino acids that give a positive score (for example, +1 or higher, +2 or higher, +3 or higher, or +4 or higher score) in the BLOSUM62 substitution matrix (S. Henikoff and J.G. Henikoff, Proc. Acad. Natl. Sci. USA 89: 10915-10919, 1992).

Modified proteins exhibit high homology to the amino acid sequence of a wild-type protein. High homology amino acid sequences include those with an identity of, for example, 70% or higher, 75% or higher, 80% or higher, 85% or higher, 90% or higher, 93% or higher, 95% or higher, or 96% or higher. The amino acid sequence identity can be determined, for example, using the BLASTP program (Altschul, S. F. et al., J. Mol. Biol. 215: 403-410, 1990). For example, the search can be carried out on the BLAST web page of National Center for Biotechnology Information (NCBI) using default parameters (Altschul S.F. et al., Nature Genet. 3: 266-272, 1993; Madden, T.L. et al., Meth. Enzymol. 266: 131-141, 1996; Altschul S.F. et al., Nucleic Acids Res. 25: 3389-3402, 1997; Zhang J. & Madden T.L., Genome Res. 7: 649-656, 1997). Sequence identity can be determined, for example, by comparing two sequences using the blast2sequences program to prepare an alignment of the two sequences (Tatiana A et al., FEMS Microbiol Lett. 174: 247-250, 1999). Gaps are treated in the same way as mismatches. For example, an identity score is calculated over the entire amino acid sequence of a naturally-occurring protein (its mature form after secretion). Specifically, sequence identity is determined by calculating the ratio of the number of identical amino acids to the total number of amino acids in a wild-type protein (the mature form in the case of a secretory protein).

Meanwhile, the AB5 toxin B subunit includes proteins encoded by nucleic acids that hybridize under stringent conditions to the entire or a portion of the coding region of a gene encoding a wild-type protein, and have an activity comparable to that of the wild-type protein (an activity of increasing the expression in RNA virus). The proteins preferably form a pentamer. For hybridization, a probe is prepared from either a nucleic acid comprising the coding sequence of the gene of a wild type protein or a complementary sequence thereof, or a nucleic acid targeted in the hybridization. The identification can be achieved by detecting whether the probe hybridizes to other nucleic acids. Stringent hybridization conditions include, for example, conditions where hybridization is carried out using a solution containing 5x SSC, 7%(W/V) SDS, 100 µg/ml denatured salmon sperm DNA, and 5x Denhardt's solution (1x Denhardt's solution contains 0.2% polyvinylpyrrolidone, 0.2% bovine serum albumin, and 0.2% Ficoll) at 50°C, preferably at 60°C, and more preferably at 65°C, and where post-hybridization washing is carried out with shaking for two hours at the same temperature as for the hybridization using 2x SSC, preferably 1x SSC, more preferably 0.5x SSC, and still more preferably 0.1x SSC.

The signal peptide (typically, the first 21 amino acids) of an original AB5 toxin B subunit may be fused intactly with a recombinant protein. Alternatively, the signal peptide may be removed or, for example, a signal peptide of a protein derived from eukaryotic cells may be attached to the N terminus or replaced with the original signal peptide (see Examples). Specifically, it is possible to use the signal sequence of a desired secretory protein such as immunoglobulin kappa light chain, interleukin (IL)-2, or tissue plasminogen activator (tPA); however, such signal sequences are not limited to these examples.

Furthermore, the fusion proteins may also contain tags, linkers, and spacers. For example, the target recombinant protein and AB5 toxin B subunit may be linked together directly or via a linker (or spacer). The sequence of linker/spacer is not particularly limited; however, the sequence may consist of, for example, 1 to 15 amino acids, preferably 1 to 8 amino acids, for example, 2 to 6 amino acids, for example, about 4 amino acids. Specifcally, the sequence includes, but is not limited to, KK (lysine-lysine), GP (glycine-proline), GPGP (glycine-proline-glycine-proline), GGS (glycine-glycine-serine), GGGS, GGGGS, repeats thereof, and various combinations thereof. In general, the target recombinant protein and AB5B are fused together so that AB5B is located on the N terminal side of the recombinant protein, i.e., the target recombinant protein is located on the C terminal side of AB5B.

Recombinant proteins to be fused with an AB5 toxin B subunit are not particularly limited; however, in particular, it is possible to markedly increase the expression of proteins that have been difficult to express at sufficient levels from RNA viral vectors. Such proteins include, for example, proteins of a short peptide chain. The short polypeptides include, for example, peptides consisting of 300 amino acids or less, preferably 250, 220, 210, 200, 180, 160, 140, 120, 100, 90, 80, 75, 70, 65, 62, 60, 55, 50, 45, 40, 35, 30, 25, or 20 amino acids, or less than any one of these numbers of amino acids (the number of amino acids excludes the portion of the AB5 toxin B subunit). The recombinant proteins to be expressed consist of, for example, seven amino acids or more, preferably 8, 10, or 15 amino acids or more (for example, 20 or more, 25 or more, 30 or more, 40 or more, or 41 or more).

More specifically, the present invention provides RNA viral vectors encoding a recombinant protein which is a peptide consisting of, for example, 120 amino acids or less, or 50 amino acids or less, and which is encoded as a fusion protein with an AB5 toxin B subunit. Herein, the "recombinant protein" refers to a protein which is fused with an AB5 toxin B subunit. That is, the AB5 toxin B subunit is not included in the length of the recombinant protein. For example, the length of the recombinant protein is the length of the portion of the fusion protein that excludes the AB5 toxin B subunit.

Proteins/peptides with low water solubility are also preferred as recombinant proteins. In particular, the expression of proteins consisting of a short peptide chain with low solubility from RNA viral vectors can be efficiently increased according to the present invention. A "protein with low water solubility" means that when vigorously vortexing at 20 ± 5°C for 30 seconds every five minutes, the volume of water or physiological saline required to dissolve 1 mg of protein within 30 minutes is 1 mL or more, preferably the volume of water or physiological saline required to dissolve 1 mg of protein within 30 minutes is 10 mL or more, and more preferably 100 mL or more.

In the present invention, proteins to be fused with AB5B particularly include neuropeptides and endocrine peptides.

RNA viral vectors of the present invention that express a recombinant protein as an AB5B fusion protein are characteristic in that they increase the expression level of the recombinant protein expressed from the RNA viral vector to, for example, 1.5 times or more, preferably twice or more, 2.5 times or more, 3 times or more, 3.5 times or more, 4 times or more, 4.5 times or more, 5 times or more, 6 times or more, 7 times or more, 8 times or more, 9 times or more, or 10 times or more than the same recombinant protein expressed alone without AB5B fusion from the same RNA viral vector. Host cells can be appropriately selected, and include, for example, BHK21 cells (ATCC CCL-10).

The present invention enables high expression of proteins that have been difficult to achieve high expression from RNA viral vectors. The target proteins include desired proteins that have been difficult to achieve high expression from RNA viral vectors. Such proteins include, for example, those characterized by a relative ratio of 0.8 or less, preferably 0.75 or less, 0.7 or less, 0.65 or less, 0.6 or less, 0.55 or less, 0.5 or less, 0.45 or less, 0.4 or less, 0.35 or less, 0.3 or less, 0.25 or less, or 0.2 or less, when comparing the ratio of the expression level of a protein when expressed from a nucleic acid encoding the protein in an RNA viral vector to the expression level of the protein when expressed using an expression plasmid vector carrying the same nucleic acid encoding the protein as an insert (i.e., the level of expression from an RNA viral vector/the level of expression from a plasmid vector) with a ratio determined, for example, using a nucleic acid encoding mouse IL-4 (SEQ ID NOs: 81 and 82). Expression plasmid vectors include, for example, those having the CMV promoter, such as pcDNA^{™} 3.1 (Invitrogen). The vectors are transfected under appropriate conditions (for example, 1 µg of plasmid is transfected in a 24-well plate using Lipofectamine^{™} 2000 (Invitrogen)). Meanwhile, the RNA viral vector is introduced into the same host cells, for example, at an MOI of 3. The cells are harvested 72 hours after gene transfection. The concentration of the protein in the culture supernatant is determined when the recombinant protein of interest is a secretory protein. Alternatively, the amount of the expressed protein relative to the total amount of protein in cells is determined. The concentration or amount of the protein is determined for mouse IL-4 and a target protein, and comparison can be made between the two values.

The vectors of the present invention may carry other foreign genes at any sites other than the insertion site of the gene encoding a fusion protein with an AB5 toxin B subunit. Such foreign genes are not limited, and include, for example, marker genes for monitoring vector infection, genes for immunoregulatory cytokines, hormones, receptors, antibodies, and fragments thereof, or other genes.

Recombinant RNA viral vectors may be reconstituted using known methods. Specifically, the vectors can be produced via the steps of: (a) introducing into cells or transcribing in cells the genomic RNA of RNA virus encoding a recombinant protein as a fusion protein with AB5 toxin B subunit, or an RNA encoding the complementary strand thereof in the presence of viral proteins that constitute the RNP containing the viral genome RNA; and (b) harvesting the generated virus or RNP containing the genomic RNA. The "viral proteins that constitute the RNP" described above typically refer to proteins that, together with the viral genome RNA, form RNP and constitute a nucleocapsid. These are a group of proteins necessary for genome replication and gene expression, and in the minus-strand RNA virus they are typically N (nucleocapsid (also referred to as nucleoprotein (NP))), P (phospho), and L (large) proteins. Although these notations vary depending on viral species, corresponding groups of proteins are known to those skilled in the art (Anjeanette Robert et al., Virology 247: 1-6, 1998).

Desired mammalian cells, avian cells, or the like can be used to produce the viral vectors. Specifically, such cells include, for example, culture cells such as monkey kidney-derived LLC-MK₂ cells (ATCC CCL-7), CV-1 cells (for example, ATCC CCL-70), and hamster kidney-derived BHK cells (for example, ATCC CCL-10), and human-derived cells. Alternatively, to obtain a viral vector on a large scale, the vector can be amplified by infecting embryonated hen eggs with the viral vector obtained from the above-described hosts. Methods for manufacturing viral vectors using hen eggs have been already developed (Nakanishi, et al., ed. (1993), "State-of-the-Art Technology Protocol in Neuroscience Research III, Molecular Neuron Physiology", Koseisha, Osaka, pp. 153-172). Specifically, for example, a fertilized egg is placed in an incubator, and cultured for 9 to 12 days at 37°C to 38°C to grow an embryo. After inoculation of the viral vector into the allantoic cavity, the egg is incubated for several days (for example, three days) to propagate the viral vector. Then, allantoic fluid containing the vector is collected from the egg. Virus vectors can be separated and purified from allantoic fluid by conventional methods (Tashiro, M., "Virus Experiment Protocol", Nagai, Ishihama, ed., Medical View Co., Ltd., pp. 68-73, 1995).

The viral proteins required for the particle formation may be expressed from the transcribed viral genome RNA or supplied in trans from a source other than the genomic RNA. For example, to reconstitute the minus-strand RNA viral vector, N, P, and L proteins can be supplied by introducing into cells plasmids or the like that express them. The transcribed genomic RNAs are allowed to replicate in the presence of these viral proteins, resulting in formation of infectious virus particles. In order to express viral proteins and RNA genome in cells, a vector linked with DNA encoding the proteins or genome downstream of an appropriate promoter is introduced into host cells. Such promoters include, for example, CMV promoter (Foecking, M. K. and Hofstetter H., Gene 45: 101-105, 1986), retroviral LTR (Shinnik, T. M., Lerner, R. A. & Sutcliffe, Nature 293: 543-548, 1981), EF1-α promoter, CAG promoter (Niwa, H. et al., Gene 108: 193-199, 1991; Japanese Patent Application Kokai Publication No. (JP-A) H03-168087 (unexamined, published Japanese patent application)).

In the production of defective viruses in which the genes of envelope proteins or such have been deleted, the infectivity of produced viruses can be complemented by expressing the deleted proteins and/or other viral proteins or such that can complement the function in virus-producing cells (WO 00/70055; WO 00/70070; WO 03/025570; Li, H.-O. et al., J. Virol. 74(14): 6564-6569, 2000). For example, the viruses may also be pseudotyped with envelope proteins of minus-strand RNA viruses of a different origin from the virus from which the viral vector genome is derived. Such an envelope protein used may be, for example, the G protein of vesicular stomatitis virus (VSV) (VSV-G) (J. Virology 39: 519-528, 1981) (Hirata, T. et al., J. Virol. Methods, 104: 125-133, 2002; Inoue, M. et al., J. Virol. 77: 6419-6429, 2003; Inoue M. et al., J Gene Med. 6: 1069-1081, 2004). Genes to be deleted from the genome include, for example, genes of spike proteins such as F, HN, H, and G, genes of envelope-lining proteins such as M, and any combinations thereof. Deletion of a spike protein gene is effective in rendering minus-strand RNA viral vectors nontransmissible, whereas deletion of the gene of an envelope-lining protein such as M protein is effective in disabling the particle formation from infected cells. For example, F gene-defective minus-strand RNA viral vectors (Li, H.-O. et al., J. Virol. 74: 6564-6569,2000), M gene-defective minus-strand RNA viral vectors (Inoue, M. et al., J. Virol. 77: 6419-6429, 2003), and the like are preferably used. Moreover, greater safety would be assured with vectors defective in any combination of at least two of F, HN (or H) and M genes. For example, vectors lacking both M and F genes are nontransmissible and defective in particle formation while retaining high level infectivity and gene expression ability.

For instance, in an example of the production of F gene-defective recombinant viruses, a plasmid expressing a minus-strand RNA viral genome defective in F gene or a complementary strand thereof is transfected into host cells along with an expression vector expressing F protein and expression vectors for N, P, and L proteins. Alternatively, viruses can be more efficiently produced by using host cells in which the F gene has been integrated into their chromosomes (WO 00/70070). In this case, a sequence-specific recombinase such as Cre/loxP and FLP/FRT and a target sequence thereof are preferably used so that the F gene can be inducibly expressed (see WO 00/70055 ; WO 00/70070; Hasan, M. K. et al., J. General Virology 78: 2813-2820, 1997). Specifically, for example, the envelope protein genes are integrated into a vector having a recombinase target sequence, such as the Cre/loxP inducible expression plasmid pCALNdlw (Arai, T. et al., J. Virology 72: 1115-1121, 1998). The expression is induced by, for example, infection with the adenovirus AxCANCre at an MOI of 3 to 5 (Saito et al., Nucl. Acids Res. 23: 3816-3821, 1995; and Arai, T. et al., J. Virol. 72: 1115-1121, 1998).

In the vectors of the present invention, any viral gene comprised may be altered from the wild-type gene, for example, to reduce the immunogenicity of viral proteins, or to enhance the efficiency of RNA transcription or replication. Specifically, for example, the transcriptional or replicational function of minus-strand RNA viral vector can be enhanced by altering at least one of the replication factor genes, N, P, and L. The HN protein, which is an envelope protein, has both hemagglutinin activity and neuraminidase activity. For example, the viral stability in blood can be enhanced by attenuating the hemagglutinin activity, and infectivity can be controlled by modifying the neuraminidase activity. The membrane fusion ability can be controlled by altering the F protein. Furthermore, for example, the antigen-presenting epitopes of the F protein or HN protein which may act as antigenic molecules on the cell surface can be analyzed, and this information can be used to prepare viral vectors that have a reduced antigenicity of these proteins. Furthermore, a temperature-sensitive mutation may be introduced into a viral gene to suppress release of secondarily released particles (or virus-like particles (VLPs)) (WO 2003/025570).

Specific examples of viral protein mutations include mutation of Glu at position 86 (E86) of the SeV P protein, substitution of another amino acid for Leu at position 511 (L511) of the SeV P protein, or substitution of homologous sites in the P protein of a different minus-strand RNA virus. Specific examples include substitution of Lys at position 86, and substitution of Phe at position 511. Regarding the L protein, examples include substitution of other amino acids for Asn at position 1197 (N1197) and/or for Lys at position 1795 (K1795) in the SeV L protein, or substitution of homologous sites in the L protein of another minus-strand RNA virus, and specific examples include substitution of Ser at position 1197, and substitution of Glu at 1795. Mutations of the P gene and L gene can significantly increase the effects of persistent infectivity, suppression of the release of secondary virions, and suppression of cytotoxicity. For example, the following mutations can be introduced: G69E, T116A, and A183S for the M gene; and A262T, G264, and K461G for the HN gene. However, mutations that can be introduced are not limited thereto (WO 2003/025570).

The minus-strand RNA viruses, for example, can be produced by the following known methods: WO 97/16539; WO 97/16538; WO 00/70055; WO 00/70070; WO 01/18223; WO 03/025570; WO 2005/071092; WO 2006/137517; WO 2007/083644; WO 2008/007581; Hasan, M. K. et al., J. Gen. Virol. 78: 2813-2820, 1997; Kato, A. et al., EMBO J. 16: 578-587, 1997; Yu, D. et al., Genes Cells 2: 457-466, 1997; Durbin, A. P. et al., Virology 235: 323-332, 1997; Whelan, S. P. et al., Proc. Natl. Acad. Sci. USA 92: 8388-8392, 1995; Schnell. M. J. et al., EMBO J. 13: 4195-4203, 1994; Radecke, F. et al., EMBO J. 14: 5773-5784, 1995; Lawson, N. D. et al., Proc. Natl. Acad. Sci. USA 92: 4477-4481; Garcin, D. et al., EMBO J. 14: 6087-6094, 1995; Kato, A. et al., Genes Cells 1: 569-579, 1996; Baron, M. D. and Barrett, T., J. Virol. 71: 1265-1271, 1997; Bridgen, A. and Elliott, R. M., Proc. Natl. Acad. Sci. USA 93: 15400-15404, 1996; Tokusumi, T. et al., Virus Res. 86: 33-38, 2002; Li, H.-O. et al., J. Virol. 74: 6564-6569, 2000. Following these methods, minus-strand RNA viruses including parainfluenza virus, vesicular stomatitis virus, rabies virus, measles virus, rinderpest virus, Sendai virus, and the like can be reconstituted from DNA.

The methods for producing plus-strand RNA viruses include the following examples:
(1) Coronavirus
   Enjuanes L, Sola 1, Alonso S, Escors D, Zuniga S. Coronavirus reverse genetics and development of vectors for gene expression. Curr Top Microbiol Immunol. 287: 161-97, 2005. Review.
(2) Togavirus
   Yamanaka R, Zullo SA, Ramsey J, Onodera M, Tanaka R, Blaese M, Xanthopoulos KG. Induction of therapeutic antitumor antiangiogenesis by intratumoral injection of genetically engineered endostatin-producing Semliki Forest virus. Cancer Gene Ther. 2001 Oct; 8(10): 796-802.
   Datwyler DA, Eppenberger HM, Koller D, Bailey JE, Magyar JP. Efficient gene delivery into adult cardiomyocytes by recombinant Sindbis virus. J Mol Med. 1999 Dec; 77(12): 859-64.
(3) Picornavirus
   Lee SG, Kim DY, Hyun BH, Bae YS. Novel design architecture for genetic stability of recombinant poliovirus: the manipulation of G/C contents and their distribution patterns increases the genetic stability of inserts in a poliovirus-based RPS-Vax vector system. J Virol. 2002 Feb; 76(4): 1649-62.
   Mueller S, Wimmer E. Expression of foreign proteins by poliovirus polyprotein fusion: analysis of genetic stability reveals rapid deletions and formation of cardioviruslike open reading frames. J Virol. 1998 Jan; 72(1): 20-31.
(4) Flavivirus
   Yun SI, Kim SY, Rice CM, Lee YM. Development and application of a reverse genetics system for Japanese encephalitis virus. J Virol. 2003 Jun; 77(11): 6450-65.
   Arroyo J, Guirakhoo F, Fenner S, Zhang ZX, Monath TP, Chambers TJ. Molecular basis for attenuation of neurovirulence of a yellow fever Virus/Japanese encephalitis virus chimera vaccine (ChimeriVax-JE). J Virol. 2001 Jan; 75(2): 934-42.
(5) Reovirus
   Roner MR, Joklik WK. Reovirus reverse genetics: Incorporation of the CAT gene into the reovirus genome. Proc Natl Acad Sci U S A. 2001 Jul 3; 98(14): 8036-41. Epub 2001 Jun 26.

For methods for propagating other RNA viruses and preparing recombinant viruses, see Experimental Virology, Detailed Discussion, 2nd Edition (Ed. Students' Association of The National Institute of Health; Maruzen, 1982).

The present invention also relates to methods for producing recombinant proteins, which comprise expressing from an RNA viral vector a recombinant protein encoded as a fusion protein with an AB5 toxin B subunit and collecting the expressed protein. Specifically, the vector is introduced into cells, and the fusion protein is expressed from the vector in the cells. By collecting the expressed fusion protein, the recombinant protein can be obtained as the fusion protein. If needed, it is also possible to collect the "recombinant protein alone by cleaving it from the fusion protein. As described above, AB5B portion can be cleaved off by introducing a sequence-specific protease cleavage sequence such as an enterokinase recognition sequence (DDDDK↓), a Factor Xa recognition sequence (IEGR↓), a thrombin recognition sequence (LVPR↓GS), or an HRV 3C protease recognition sequence (LEVLFQ↓GP) between the recombinant protein of interest and AB5B. The cells are not limited as long as the vectors of the present invention can be introduced into them. Such cells include cultured mammalian cells (cells derived from mice, rats, monkeys, and humans) such as LLC-MK₂ cells (ATCC CCL-7), CV-1 cells (for example, ATCC CCL-70), and BHK cells (or example, ATCC CCL-10). Alternatively, it is possible to express the protein using hen eggs to prepare a large amount of viral vector (Nakanishi, et al., ed. (1993) "State-of-the-Art Technology Protocol in Neuroscience Research III, Molecular Neuron Physiology", Koseisha, Osaka, pp. 153-172).

The present invention also relates to compositions that comprise a vector of the present invention or cells introduced with the vector. The compositions of the present invention are useful for any purpose where high-level expression of a recombinant protein is required because they enable efficient expression of the protein. Specifically, the compositions of the present invention are used to enhance the expression of a protein or to achieve enhanced protein expression. In the production of the compositions of the present invention, the vector or cells may be combined with desired pharmaceutically acceptable carriers or vehicles, if needed. The "pharmaceutically acceptable carriers or vehicles" include desired solutions in which the vector or cells can be suspended, for example, phosphate-buffered saline (PBS), sodium chloride solutions, Ringer's solution, and culture media. When the vector is amplified using hen eggs or such, it may contain allantoic fluid. Furthermore, the compositions comprising the vector may contain carriers or vehicles such as deionized water and an aqueous solution of 5% dextrose. In addition, the compositions may also contain vegetable oils, suspending agents, surfactants, stabilizers, biocidal agents, or such. It is also possible to add preservatives or other additives. The compositions of the present invention can be used as desired reagents or pharmaceutical compositions.

Furthermore, the compositions of the present invention can be combined with, as a carrier, an organic substance such as a biopolymer, or an inorganic substance such as hydroxyapatite; specifically, a collagen matrix, a polylactate polymer or copolymer, a polyethylene glycol polymer or copolymer, and a chemical derivative thereof, etc.

The present invention also provides uses of a nucleic acid encoding an AB5 toxin B subunit for enhancing protein expression. The present invention also provides reagents for enhancing protein expression, which comprise an AB5 toxin B subunit or nucleic acid encoding the subunit. The present invention also provides expression-enhancing agents, which comprise an AB5 toxin B subunit or a nucleic acid encoding the subunit. A nucleic acid encoding an AB5 toxin B subunit is ligated in frame to a nucleic acid encoding a protein of interest to give a nucleic acid encoding a fusion protein consisting of the two. The resulting nucleic acid can be expressed, for example, using an RNA viral vector to significantly increase the expression level of the protein as compared to expressing the protein alone.

Furthermore, the present invention provides methods for increasing the expression level of a recombinant protein, which comprise the step of expressing the recombinant protein as a fusion protein with an AB5 toxin B subunit. Such an AB5 toxin B subunit may be, for example, cholera toxin B (CTB). Meanwhile, as described above, the recombinant protein may be, for example, a peptide of 120 amino acids or less, or a peptide of 50 amino acids or less. The recombinant protein may be a neuropeptide or endocrine peptide. Herein, the "recombinant protein" refers to a protein that is fused with an AB5 toxin B subunit. Alternatively, the recombinant protein may be, for example, a protein with low solubility. The recombinant protein is expressed, for example, from an RNA viral vector, preferably, for example, from a minus-strand RNA viral vector.

The present invention also provides uses of an AB5 toxin B subunit or a nucleic acid encoding it for increasing protein expression. The present invention also relates to AB5 toxin B subunit and nucleic acids encoding it for use in increasing protein expression. The present invention also relates to compositions for use in enhancing protein expression, which comprise the subunit or a nucleic acid encoding it. Whether the expression level is increased can be tested, for example, by expressing the recombinant protein without CTB fusion from the same vector and comparing the expression level of the recombinant proteins.

Furthermore, the present invention relates to uses of the genomic RNA of an RNA virus encoding a fusion protein between a recombinant protein and an AB5 toxin B subunit, or the complementary strand thereof (antigenome RNA), or a DNA encoding at least either of the two, in producing an RNA viral vector with increased recombinant protein expression, cells introduced with the vector, or a composition comprising either of the two. The present invention also relates to uses of a DNA encoding the genomic RNA of an RNA virus encoding a fusion protein between a recombinant protein and an AB5 toxin B subunit, or the complementary strand thereof (antigenome RNA) for increasing the expression level of the recombinant protein.

Furthermore, the present invention relates to compositions for achieving enhanced expression of a recombinant protein, which comprise an RNA viral vector encoding the recombinant protein as a fusion protein with an AB5 toxin B subunit. The present invention also relates to uses of an RNA viral vector encoding the recombinant protein as a fusion protein with an AB5 toxin B subunit for enhancing the expression of the recombinant protein. The present invention also provides agents (expression enhancers) for use in enhancing the expression of a recombinant protein, which comprise an RNA viral vector encoding the recombinant protein as a fusion protein with an AB5 toxin B subunit. The present invention also provides uses of an RNA viral vector encoding a recombinant protein as a fusion protein with an AB5 toxin B subunit, in producing pharmaceutical agents, reagents, and/or pharmaceuticals with enhanced capacity of expressing the recombinant protein.

Compositions comprising the vectors of the present invention or compositions comprising the cells introduced with the vectors can be used *in vitro,* for *in vivo* administration, and for *ex vivo* administration *via* cells. When RNA viral vectors are administered *via* cells, they are introduced into appropriate culture cells, cells harvested from animals as inoculation targets, or the like. For infecting cells with the RNA viruses outside the body (for example, in a test tube or dish), the infection is carried out *in vitro* (or *ex vivo*), for example, in a desired physiological aqueous solution such as culture medium, physiological saline, blood, plasma, serum, or body fluid, where the multiplicity of infection (MOI; the number of infectious viruses per cell) preferably ranges from 1 to 1,000, more preferably 2 to 500, even more preferably 3 to 300, and still more preferably 5 to 100.

The dose of a composition of the present invention varies depending on the disease, patient's weight, age, sex, and symptoms, purpose of administration, dosage form of the composition to be introduced, administration method, a gene to be introduced, etc.; however, those skilled in the art can determine the administration route. In addition, the administered dose may be selected depending on the type of animal to be administration, site or frequency of administration, etc. Subjects to be administered with a composition comprising the vector of the present invention preferably include mammals (including humans and nonhuman mammals). Specifically, such mammals include humans, nonhuman primates such as monkeys, rodents such as mice and rats, rabbits, goats, sheep, pigs, bovines, dogs, cats, and all other mammals.

The vectors of the present invention can be preferably used as virus-like particles (VLP), and can also be used in the same way as conventional viral particles.

### Examples

Hereinbelow, the present invention will be specifically described in the context of the Examples; however, it is not to be construed as being limited thereto.

### [Example 1] Construction of an SeV vector carrying the mCRF gene

An mCRF fragment (SEQ ID NO: 3) in which an Igκ secretion signal is attached to a mouse CRF peptide gene (accession number NM_205769) was synthesized by PCR using as a template a human Aβ42 gene to which an Igκ secretion signal is attached (SEQ ID NO: 1). The primers used are shown in SEQ ID NOs: 4 to 8. Then, using the CTB-Aβ42 NotI fragment (Example 10; SEQ ID NO: 14) as a template, PCR was carried out to synthesize an EcoRV-EIS fragment (SEQ ID NO: 9). The primers used are shown in SEQ ID NOs: 10 and 11. Next, the mCRF fragment was digested with *Xho*I and *EcoR*V, while the EcoRV-EIS fragment was digested with *Eco*RV and *Not*I. The resulting fragments were subcloned into a pCI vector (Promega) to construct an mCRF gene NotI fragment to which a Sendai virus transcription signal was attached (pCl-mCRF) (SEQ ID NO: 12).

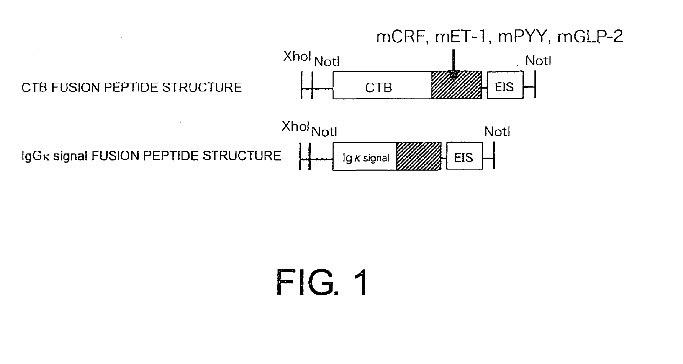

A cDNA of the F gene-deficient SeV vector (WO 00/70070) (pSeV18+NotI/ΔF) was digested with *NotI,* and the mCRF gene NotI fragment (SEQ ID NO: 12) was inserted at the NotI site to construct F gene-deficient SeV cDNA carrying the mCRF gene (pSeV18+mCRF /ΔF) (Fig. 1).

### [Example 2] Construction of SeV vector carrying fusion gene of mCRF and CTB

Using the CTB-Aβ42 NotI fragment (Example 10; SEQ ID NO: 14) as a template, PCR was carried out to synthesize a CTB-mCRF fragment (SEQ ID NO: 16). The primers used are shown in SEQ ID NOs: 3, 4, 5, 17, and 18. Next, the CTB-mCRF fragment was digested with *Xho*I and *EcoRV,* while the pCI-mCRF prepared above was digested with *Xho*I and *EcoRV.* A CTB-mCRF gene NotI fragment to which Sendai virus transcription signal was attached (SEQ ID NO: 19) was constructed.

A cDNA of the F gene-deficient SeV vector (WO 00/70070) (pSeV18+NotI/ΔF) was digested with *Not*I, and the CTB-mCRF gene NotI fragment (SEQ ID NO: 19) was inserted at the NotI site to construct F gene-deficient SeV cDNA carrying the CTB-mCRF gene (pSeV18+CTB-mCRF/ΔF) (Fig. 1).

### [Example 3] Construction of SeV vector carrying mET-1 gene

An mET1 fragment (SEQ ID NO: 21) in which an Igκ secretion signal is attached to a mouse ET1 peptide gene (accession number NP_034234) was synthesized by PCR using as a template a human Aβ42 gene to which an Igκ secretion signal is attached (SEQ ID NO: 1). The primers used are shown in SEQ ID NOs: 7, 22, 23, and 24. Then, using the CTB-Aβ42 NotI fragment (SEQ ID NO: 14) as a template, PCR was carried out to synthesize an EcoRV-PstI-EIS fragment (SEQ ID NO: 25). The primers used are shown in SEQ ID NOs: I 1 and 26. Next, the mET1 fragment was digested with *Xho*I and *Eco*RV, while the EcoRV-PstI-EIS fragment was digested with *Eco*RV and *Not*I. The resulting fragments were subcloned into pCl vector to construct an mET1 gene NotI fragment to which Sendai virus transcription signal was attached (pCI-mET1) (SEQ ID NO: 27).

A cDNA of the F gene-deficient SeV vector (WO 00/70070) (pSeV18+NotI/ΔF) was digested with *Not*I, and the mET1 gene NotI fragment (SEQ ID NO: 27) was inserted at the NotI site to construct F gene-deficient SeV cDNA carrying the mET1 gene (pSeV18+mET1/ΔF) (Fig. 1).

### [Example 4] Construction of SeV vector carrying fusion gene of mET-1 and CTB

Using the CTB-Aβ42 NotI fragment (SEQ ID NO: 14) as a template, PCR was carried out to synthesize a CTB-mET1 fragment (SEQ ID NO: 29). The primers used are shown in SEQ ID NOs: 17, 22, 23, and 30. Next, the CTB-mET1 fragment was digested with *Xho*I and *Eco*RV, while the pCI-mET1 prepared above was digested with *Xho*I and *Eco*RV. A CTB-mET1 gene NotI fragment to which Sendai virus transcription signal was attached (SEQ ID NO: 31) was constructed.

A cDNA of the F gene-deficient SeV vector (WO 00/70070) (pSeV18+NotI/ΔF) was digested with *NotI,* and the CTB-mET1 gene *Not*I fragment (SEQ ID NO: 31) was inserted at the *Not*I site to construct F gene-deficient SeV cDNA carrying the CTB-mET1 gene (pSeV18+CTB-mET1/ΔF) (Fig. 1).

### [Example 5] Construction of SeV vector carrying mPYY gene

An mPYY fragment (SEQ ID NO: 33) in which the Igκ secretion signal is attached to a mouse PYY peptide gene (accession number NM_145435.1) was synthesized by PCR using as a template a human Aβ42 gene to which an Igκ secretion signal is attached (SEQ ID NO: 1). The primers used are shown in SEQ ID NOs: 7, 34, 35, 36, and 37. Next, the mPYY fragment was digested with *Xho*I and *Eco*RV, while the EcoRV-EIS fragment (SEQ ID NO: 9) was digested with *Eco*RV and *Not*I. The resulting fragments were subcloned into pCI vector to construct an mPYY gene NotI fragment to which Sendai virus transcription signal was attached (SEQ ID NO: 38).

A cDNA of the F gene-deficient SeV vector (WO 00/70070) (pSeV18+NotI/ΔF) was digested with *Not*I, and the mPYY gene NotI fragment (SEQ ID NO: 38) prepared above was inserted at the NotI site to construct F gene-deficient SeV cDNA carrying the mPYY gene (pSeV18+mPYY/AF) (Fig. 1).

### [Example 6] Construction of SeV vector carrying fusion gene of mPYY and CTB

Using the CTB-Aβ42 NotI fragment (SEQ ID NO: 14) as a template, PCR was carried out to synthesize a CTB-mPYY fragment (SEQ ID NO: 40). The primers used are shown in SEQ ID NOs: 17, 34, 35, 36, and 41. Next, the CTB-mPYY fragment was digested with *Xho*I and *Eco*RV, while the EcoRV-EIS fragment (SEQ ID NO: 9) was digested with *Eco*RV and *Not*I. The resulting fragments were subcloned into pCI vector to construct a CTB-mPYY gene NotI fragment to which Sendai virus transcription signal was attached (SEQ ID NO: 42).

A cDNA of the F gene-deficient SeV vector (WO 00/70070) (pSeV18+NotI/ΔF) was digested with *Not*I, and the CTB-mPYY gene NotI fragment (SEQ ID NO: 42) was inserted at the NotI site to construct F gene-deficient SeV cDNA carrying the CTB-mPYY gene (pSeV18+CTB-mPYY/ΔF) (Fig. 1).

### [Example 7] Construction of SeV vector carrying mGLP-2 gene

An mGLP-2 fragment (SEQ ID NO: 44) in which the Igκ secretion signal is attached to a mouse GLP-2 peptide gene (accession number NM_175681.2) was synthesized by PCR using as a template a human Aβ42 gene to which an Igκ secretion signal is attached (SEQ ID NO: 1). The primers used are shown in SEQ ID NOs: 7, 45, 46, 47, and 48. Next, the mGLP-2 fragment was digested with *Xho*I and *Eco*RV, while the EcoRV-PstI-EIS fragment (SEQ ID NO: 25) was digested with *Eco*RV and *Not*I. The resulting fragments were subcloned into pCI vector to construct an mGLP-2 gene NotI fragment to which Sendai virus transcription signal was attached (SEQ ID NO: 49).

A cDNA of the F gene-deficient SeV vector (WO 00/70070) (pSeV18+NotI/ΔF) was digested with *Not*I, and the mGLP-2 gene *Not*I fragment (SEQ ID NO: 49) was inserted at the NotI site to construct F gene-deficient SeV cDNA carrying the mGLP-2 gene (pSeV18+mGLP-2/ΔF) (Fig. 1).

### [Example 8] Construction of SeV vector carrying fusion gene of GLP-2 and CTB

Using the CTB-Aβ42 NotI fragment (SEQ ID NO: 14) as a template, PCR was carried out to synthesize a CTB-mGLP-2 fragment (SEQ ID NO: 51). The primers used are shown in SEQ ID NOs: 17, 45, 46, 47, and 52. Next, the CTB-mGLP-2 fragment was digested with *Xho*I and *Eco*RV, while the EcoRV-PstI-EIS fragment (SEQ ID NO: 25) was digested with *Eco*RV and *Not*I. The resulting fragments were subcloned into pCI vector to construct a CTB-mGLP-2 gene NotI fragment to which Sendai virus transcription signal was attached (SEQ ID NO: 53).

A cDNA of the F gene-deficient SeV vector (WO 00/70070) (pSeV 18+NotI/ΔF) was digested with *Not*I, and the CTB-mGLP-2 gene NotI fragment (SEQ ID NO: 53) was inserted at the NotI site to construct F gene-deficient SeV cDNA carrying the CTB-mGLP-2 gene (pSeV18+CTB-mGLP-2/ΔF) (Fig. 1).

### [Example 9] Construction of SeV vector carrying the Aβ42 gene

### (1) Preparation of Aβ42 gene NotI fragment

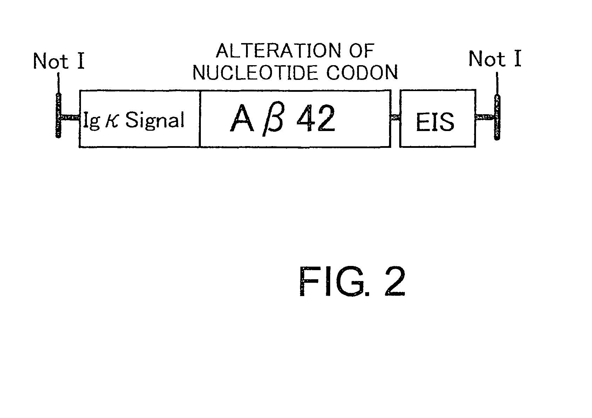

The Aβ42 gene was assembled by PCR using a number of primers that covered the entire length of the human amyloid β peptide sequence (1-42) (SEQ ID NO: 55). The nucleotide sequence of Aβ42 was optimized by taking the human codon usage into consideration. The resulting sequence had a structure where the Igκ secretory signal was attached to the N terminus and the transcription signal of Sendai virus was added to the C terminus (Fig. 2; SEQ ID NO: 56).

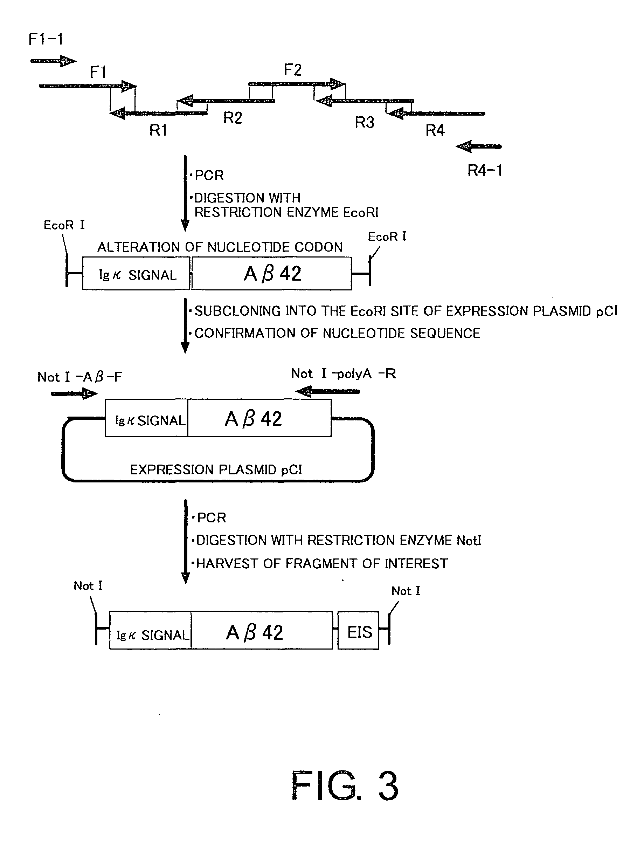

The construction method is shown in Fig. 3. First, the six types of long primers covering the entire region of Igκ signal and Aβ42, F1 (SEQ ID NO: 58), F2 (SEQ ID NO: 59), R1 (SEQ ID NO: 60), R2 (SEQ ID NO: 61), R3 (SEQ ID NO: 62), and R4 (SEQ ID NO: 63), were combined together. PCR was carried out using the primer mixture without template. Then, the resulting PCR product was used as a template for the second round PCR and two primers F1-1 (SEQ ID NO: 64) and R4-1 (SEQ ID NO: 65), each having a restriction enzyme *Eco*RI recognition sequence, were used. The obtained PCR product was cleaved by restriction enzyme *Eco*RI and subcloned into the pCI expression plasmid (Promega) at the *Eco*RI site. A clone having the correct nucleotide sequence was selected by sequencing.

PCR was carried out using the selected plasmid as a template, as well as primer NotI-Aβ-F (SEQ ID NO: 66) with the addition of a *NotI* recognition sequence and primer NotI-polyA-R (SEQ ID NO: 67) with the addition of a Sendai virus transcription signal and *NotI* recognition sequence. The resulting PCR product was digested with the restriction enzyme *Not*I to give the Aβ42 *Not*I fragment of interest (Fig. 2; SEQ ID NO: 56).

### (2) Construction of Sendai virus cDNA carrying the Aβ42 gene

A cDNA of the F gene-deficient SeV vector (WO 00/70070) (pSeV18+NotI/ΔF) was digested with *Not*I, and the Aβ42 *Not*I fragment was inserted at the *NotI* site to construct F gene-deficient SeV cDNA carrying the Aβ42 gene (pSeV18+Aβ42/ΔF).

### [Example 10] Construction of SeV vector carrying a fusion gene of Aβ42 and CTB (CTB-Aβ42)

### (1) Preparation of CTB-Aβ42 gene NotI fragment

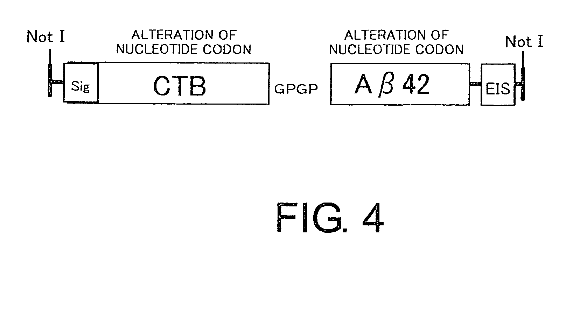

The CTB-Aβ42 *Not*I fragment had a structure in which a cholera toxin B subunit sequence (SEQ ID NO: 68) containing a secretory signal is linked *via* a GPGP amino acid linker to the N terminus of the human amyloid β sequence (1-42) and a Sendai virus transcription signal is attached to its C terminus (Fig. 4; SEQ ID NO: 69). To improve the expression efficiency, the nucleotide sequences of CTB and Aβ were altered according to the human codon usage without altering their amino acid sequences.

This gene was constructed by synthesizing its entire length by PCR using long primers that fully covered the gene. Specifically, eight types of long primers that covered the entire length of the CTB-Aβ region were synthesized: CTB-AβF-1 (SEQ ID NO: 71), F-2 (SEQ ID NO: 72), F-3 (SEQ ID NO: 73), F-4 (SEQ ID NO: 74), R-1 (SEQ ID NO: 75), R-2 (SEQ ID NO: 76), R-3 (SEQ ID NO: 77), and R-4 (SEQ ID NO: 78). By using a mixture of these eight types of primers, PCR was carried out to prepare a corresponding fragment from the N terminus to Aβ42. To prepare the C-terminal fragment containing the Sendai virus transcription signal, PCR was carried out using the pCI plasmid (Promega) as a template and two types of primers, CTB-Aβ F1-2 (SEQ ID NO: 79) and CTB-Aβ R5-2 (SEQ ID NO: 80). The PCR yielded a PCR fragment that covered the entire length of CTB-Aβ. The PCR fragment was subcloned into the pGEM-T Easy plasmid (Promega) by TA cloning. After the nucleotide sequence was confirmed, the plasmid was amplified and digested with restriction enzyme *Not*I to prepare a CTB-Aβ42 *Not*I fragment of interest (SEQ ID NO: 69).

### (2) Construction of Sendai virus cDNA carrying the CTB-Aβ42 gene

A cDNA of F gene-deficient SeV vector (WO 00/70070) (pSeV18+NotI/ΔF) was digested with *Not*I, and the CTB-Aβ42 *Not*I fragment was inserted into the *NotI* site to construct an F gene-deficient SeV cDNA carrying the CTB-Aβ42 gene (pSeV 18+CTB-Aβ42/ΔF).

### [Example 11] Construction of SeV vector carrying a fusion gene of Aβ42 and IL-4

### (1) Preparation of a NotI fragment of the Aβ42 and IL-4 fusion gene

The Aβ42 gene and mouse IL-4 were fused together by the assemble PCR method based on partial overlaps.

The Aβ42 gene was prepared from a plasmid containing an Aβ42 *Eco*RI fragment (Example 9; Fig. 3). Meanwhile, the mouse IL-4 gene (SEQ ID NO: 81) was prepared as a cDNA by the following procedure. mRNA was extracted from mouse (BALB/cA) spleen, and reverse transcribed using an IL-4-specific primer. The resulting cDNA was amplified by PCR and subcloned into a cloning plasmid. The resulting plasmid having mouse IL-4 cDNA as an insert was used in the construction.

Specifically, PCR was carried out using the mouse IL-4 plasmid as a template and two types of primers, NotI-IL4-F (SEQ ID NO: 83) and IL4-R (SEQ ID NO: 84). Meanwhile, using Aβ42 plasmid as a template, PCR was carried out with primers Aβ42-F (SEQ ID NO: 85) and NotI-Aβ42-R (SEQ ID NO: 86), and IL-4 and Aβ42 PCR fragments were obtained. The primers IL4-R and Aβ42-F were designed to partially overlap with each other. Thus, the two genes were fused into a single fusion gene by PCR using a mixture of IL-4 and Aβ42 PCR fragments as template and the primers NotI-IL4-F and NotI-Aβ42-R. The resulting PCR fragment was subcloned into a cloning plasmid. After the nucleotide sequence was confirmed, the plasmid was cleaved with restriction enzyme *Not*I to prepare a *Not*I fragment of interest containing the fusion gene of Aβ42 and IL-4 (SEQ ID NO: 87).

### (2) Construction of a Sendai virus cDNA carrying the Aβ42 gene

A cDNA of F gene-deficient SeV vector (WO 00/70070) (pSeV18+NotI/ΔF) was digested with *Not*I. The mIL4-Aβ42 *Not*I fragment prepared as mentioned above was inserted into the *Not*I site to construct an F gene-deficient SeV cDNA carrying the Aβ42 gene (pSeV18+mIL4-Aβ42/ΔF).

### [Example 12] Reconstitution and amplification of Sendai virus vector

Virus reconstitution and amplification were carried out according to the report of Li *et al.* (Li, H.-O. et al., J. Virology 74: 6564-6569, 2000; WO 00/70070) and a modified method thereof (WO 2005/071092). Since the vectors used were F gene deficient, helper cells for the F protein, in which the F protein is expressed by the Cre/loxP expression induction system, were used. This system uses the pCALNdLw plasmid (Arai, T. et al., J. Virol. 72: 1115-1121, 1988), which has been designed in a way that expression of gene products is induced by the Cre DNA recombinase. To express the inserted gene, a transformant with the plasmid is infected with a recombinant adenovirus expressing the Cre DNA recombinase (AxCANCre) by the method of Saito *et al.* (Saito, I. et al., Nucl. Acid. Res. 23: 3816-3821, 1995; Arai, T. et al., J. Virol. 72: 1115-1121, 1998).

F gene-deficient SeV vectors each carrying the CTB-mCRF, CTB-mET1, CTB-mPYY, CTB-mGLP2, mCRF, mET1, mPYY, mGLP2, Aβ42, CTB-Aβ42, or mIL4-Aβ42 gene (SeV18+CTB-mCRF/ΔF, SeV18+CTB-mET1/ΔF, SeV18+CTB-mPYY/ΔF, SeV18+CTB-mGLP2/ΔF, SeV18+mCRF/ΔF, SeV18+mET1/ΔF, SeV18+mPYY/ΔF, SeV18+mGLP2/ΔF, SeV18+Aβ42/ΔF, SeV18+CTB-Aβ42/ΔF, or SeV18+mIL4-Aβ42/ΔF, respectively) were prepared by the methods described above.

### [Example13] Construction of SeV vector carrying a fusion gene of Aβ42 and PEDI

### (1) Construction of SeV vector cDNA carrying the Aβ42-PEDI fusion gene

The PEDI gene was amplified by PCR using ten types of primers: PEDI-1F (SEQ ID NO: 89), PEDI-2R (SEQ ID NO: 90), PEDI-3F (SEQ ID NO: 91), PEDI-4R (SEQ ID NO: 92), PEDI-5F (SEQ ID NO: 93), PEDI-6R (SEQ ID NO: 94), PEDI-7F (SEQ ID NO: 95), PEDI-8R (SEQ ID NO: 96), PEDI-9F (SEQ ID NO: 97), and PEDI-10R (SEQ ID NO: 98). The PEDI gene and Aβ42 were fused together and inserted into the SeV vector by the following procedure. Fragment 1 was prepared by PCR using the SeV vector carrying Aβ42 as a template with primers SeVF6 (SEQ ID NO: 99) and S-PEDI-C (SEQ ID NO: 100). Fragment 3 was prepared by PCR using the same template and primers PEDI-Ab-N (SEQ ID NO: 101) and SEVR280 (SEQ ID NO: 102). Fragment 2 was prepared by PCR using the PEDI gene as a template and primers PEDI-N (SEQ ID NO: 103) and PEDI-C (SEQ ID NO: 104). Using these fragments as a template, overlap PCR was carried out to prepare the PEDI-Aβ42 NotI fragment (SEQ ID NO: 105). The resulting fragment was inserted into the *NotI* site of pSeV18+/ΔF to prepare a cDNA of F-deficient SeV vector carrying the PEDI-Aβ42 fusion gene.

### (2) Reconstitution of an F-deficient SeV vector carrying PEDI-Aβ42

An SeV vector carrying the PEDI-Aβ42 gene (SeV18+PEDI-Aβ42/ΔF) was reconstituted in the same way as described in Example 4 according to the method of Li *et al.* (Li, H.-O. et al., J. Virology 74: 6564-6569, 2000; WO 00/70070) and its modified method (WO 2005/071092).

[Example 14] Effects of CTB fusion on mCRF expression: comparison of the expression abilities of SeV vector for CTB-mCRF fusion protein expression and SeV vector for mCRF expression (1) BHK21 cells were infected with SeV18+mCRF/ΔF and SeV18+CTB-mCRF/ΔF to assess the expression level of mCRF and CTB-mCRF peptides. BHK21 cells were plated in collagen-coated 6-well plates at 1 x 10⁶ cells, and infected with each SeV vector diluted to an MOI of 10 with serum-free medium (VPSFM). GMEM containing 10% FBS was added to the plates after one hour. The medium was replaced with serum-free medium (VPSFM) 24 hours later, and the cells and culture supernatants were harvested after 48 hours to prepare cell lysates. Uninfected cells were used as a control.

### (2) Quantitation by ELISA

CRF was quantified using a CRF ELISA kit (Yanaihara Institute Inc.). The expression level was determined by absorbance (O.D. 450) measurement using a plate reader.

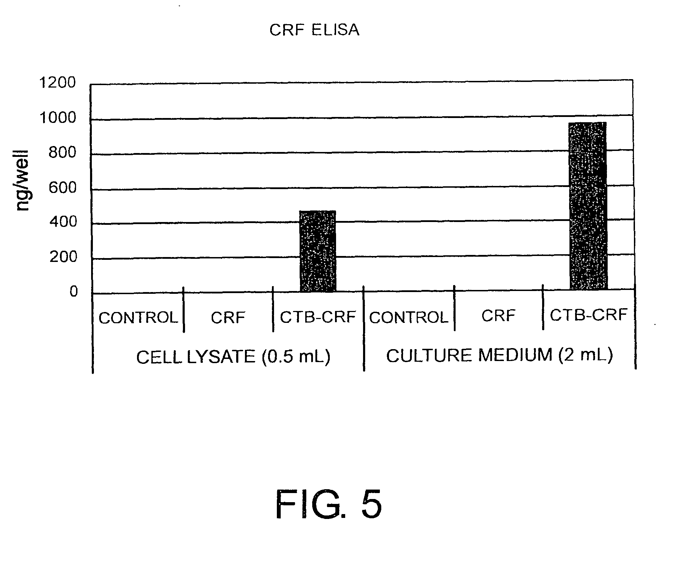

The result is shown in Fig. 5. The results showed that the expression level of CTB-mCRF was markedly increased, while mCRF was expressed at a negligible level.

[Example 15] Effects of CTB fusion on mET-1 expression: comparison of the expression abilities of SeV vector for CTB-mET1 fusion protein expression and SeV vector for mET1 expression (1) BHK21 cells were infected with SeV18+mET-1/ΔF and SeV18+CTB-mET-1/ΔF to assess the expression level of mET-1 and CTB-mET1 peptides. BHK21 cells were plated in collagen-coated 6-well plates at 1 x 10⁶ cells, and infected with each SeV vector diluted to an MOI of 10 with serum-free medium (VPSFM). GMEM containing 10% FBS was added to the plates after one hour. The medium was replaced with serum-free medium (VPSFM) 24 hours later, and the cells and culture supernatants were harvested after 48 hours to prepare cell lysates. Uninfected cells were used as a control.

### (2) Quantitation by ELISA

ET1 was quantified by detecting with an anti-mouse ET1 antibody and a peroxidase-labeled anti-rabbit IgG antibody after immobilizing samples directly onto 96-well plate and blocking the plate with 1% BSA/TBS-T buffer. The expression level was determined by absorbance (O.D. 450) measurement using a plate reader.

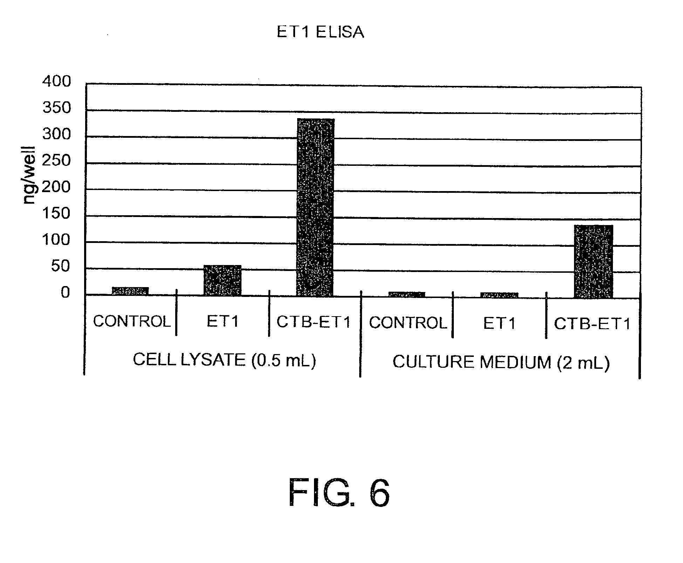

The result is shown in Fig. 6. The expression level of CTB-mET1 was markedly increased, while mET1 was expressed at a negligible level.

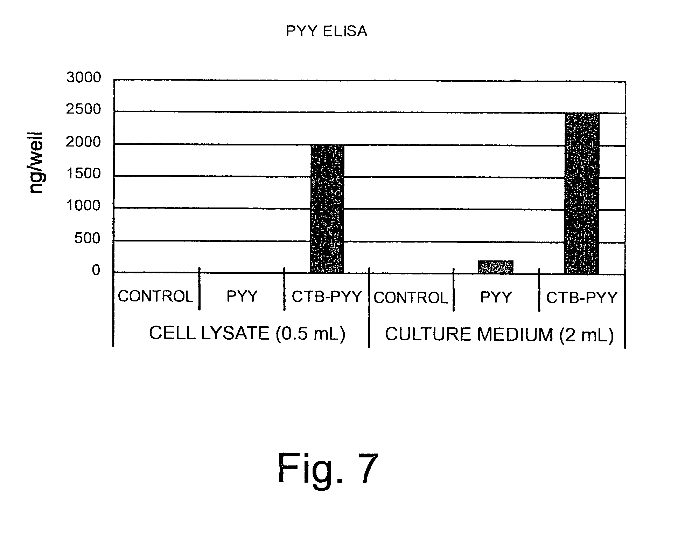

[Example 16] Effects of CTB fusion on mPYY expression: comparison of the expression abilities of SeV vector for CTB-mPYY fusion protein expression and SeV vector for mPYY expression
(1) BHK21 cells were infected with SeV18+mPYY/ΔF and SeV18+CTB-mPYY/ΔF to assess the expression level of mPYY and CTB-mPYY peptides. BHK21 cells were plated in collagen-coated 6-well plates at 1 x 10⁶ cells, and infected with each SeV vector diluted to an MOI of 10 with serum-free medium (VPSFM). GMEM containing 10% FBS was added to the plates after one hour. The medium was replaced with serum-free medium (VPSFM) 24 hours later, and the cells and culture supernatants were harvested after 48 hours to prepare cell lysates. Uninfected cells were used as a control.
(2) Quantitation by ELISA
   mPYY was quantified using a rat PYY ELISA kit (Yanaihara Institute Inc.). The expression level was determined by absorbance (O.D. 450) measurement using a plate reader.
   The result is shown in Fig. 7. The expression level of CTB-mPYY was markedly increased, while mPYY was expressed at a negligible level.

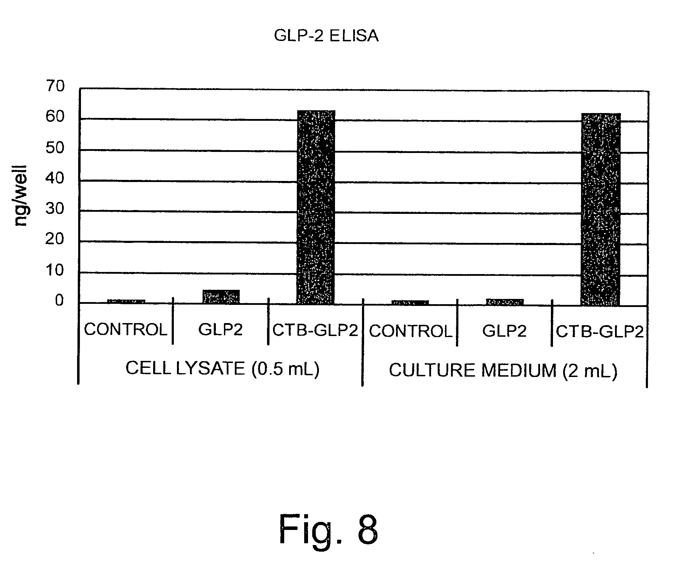

[Example 17] Effects of CTB fusion on mGLP-2 expression: comparison of the expression abilities of SeV vector for CTB-mGLP-2 fusion protein expression and SeV vector for mGLP-2 expression
(1) BHK21 cells were infected with SeV18+mGLP-2/ΔF and SeV18+CTB-mGLP-2/ΔF to assess the expression level of mGLP-2 and CTB-mGLP-2 peptides. BHK21 cells were plated in collagen-coated 6-well plates at 1 x 10⁶ cells, and infected with each SeV vector diluted to an MOI of 10 with serum-free medium (VPSFM). GMEM containing 10% FBS was added to the plates after one hour. The medium was replaced with serum-free medium (VPSFM) 24 hours later, and the cells and culture supernatants were harvested after 48 hours to prepare cell lysates. Uninfected cells were used as a control.
(2) Quantitation by ELISA mGLP-2 was quantified using a GLP-2 ELISA kit (Yanaihara Institute Inc.). The expression level was determined by absorbance (O.D. 450) measurement using a plate reader.
   The result is shown in Fig. 8. The expression level of CTB-mGLP-2 was markedly increased, while mGLP-2 was expressed at a negligible level.

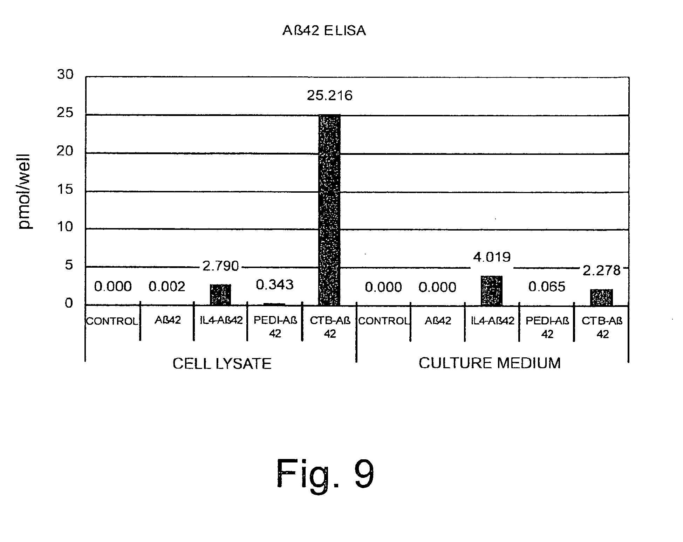

[Example 18] Comparison of the effects of CTB fusion, PEDI fusion, and IL-4 fusion on Aβ42 expression: comparison of the expression abilities of SeV vector for CTB-Aβ42 fusion protein expression, SeV vector for PEDI-Aβ42 fusion protein expression, and SeV vector for IL-4-Aβ42 fusion protein expression
(1) BHK21 cells were infected with SeV18+Aβ42/ΔF, SeV18+IL-4-Aβ42/ΔF, SeV18+PEDI-Aβ42/ΔF, and SeV18+CTB-Aβ42/ΔF to assess the level of Aβ42 antigen. BHK21 cells were plated in collagen-coated 6-well plates at 1 x 10⁶ cells/well, and infected with each SeV vector diluted to an MOI of 10 with serum-free medium (VPSFM). GMEM containing 10% FBS was added to the plates after one hour. The medium was replaced with serum-free medium (VPSFM) 24 hours later, and the cells and culture supernatants were harvested after 48 hours to prepare cell lysates.
(2) Quantitation by ELISA
   Aβ42 was quantified using a human Aβ42 ELISA kit (Wako Pure Chemical Industries). The expression level was determined by absorbance (O.D. 450) measurement using a plate reader.
   The result is shown in Fig. 9. The expression levels of IL-4-Aβ42, PEDI-Aβ42, and CTB-Aβ42 were increased, while Aβ42 was expressed at a negligible level. Each intracellular expression level was 1,395 times, 171.5 times, or 12,608 times that of kkAβ42.

### [Example 19] Effect of CTB fusion on Aβ42 expression: comparison of the expression abilities between SeV vector to express Aβ42 alone and SeV vector to express CTB-Aβ42 fusion protein

Confluent BHK-21 cells (plated at 3 x 10⁵ cells/well in 12-well plates) plated on the previous day were infected at an MOI of 10 with an SeV vector carrying Aβ42 alone or an SeV vector carrying CTB-Aβ42, and then cultured in the VP-SFM medium (1 ml/well) at 37°C under 5% CO₂. The culture supernatants were collected and cell lysates were prepared on day 4. The culture supernatants were concentrated ten-fold by acetone precipitation, and samples were prepared from them using 1x SDS Loading Buffer. The cell lysates were prepared in 150 µl/well using 1x SDS Loading Buffer. The prepared culture supernatants and cell lysates were heated at 98°C for 10 minutes. Then, SDS-PAGE (using 15% Wako gel) and Western blotting (using antibody 6E10) were carried out, and proteins were quantified using the Aβ42 peptide as control at 1, 0.5, 0.25, and 0.125 ng/lane. With the SeV vector carrying Aβ42 alone, the Aβ expression level was only 4.4 ng/well or 7.2 x 10⁻³ ng/well in the cell lysate or supernatant, respectively. On the other hand, with the SeV vector carrying Aβ42-CTB fusion gene, the Aβ expression level was 2,500 ng/well and 200 ng/well in the cell lysate and supernatant, respectively. That is, the Aβ expression level was greatly improved to be 568 and 27,778 times higher in the lysate and supernatant, respectively.

### [Example 21] Construction of SeV vectors carrying the Aβ15-CTB fusion gene (CTB-Aβ15) or a fusion gene of tandem Aβ15 repeats and CTB (CTB-Aβ15x2, CTB-Aβ15x4, or CTB-Aβ15x8)

### (1) Preparation of CTB-Aβ15 gene NotI fragment

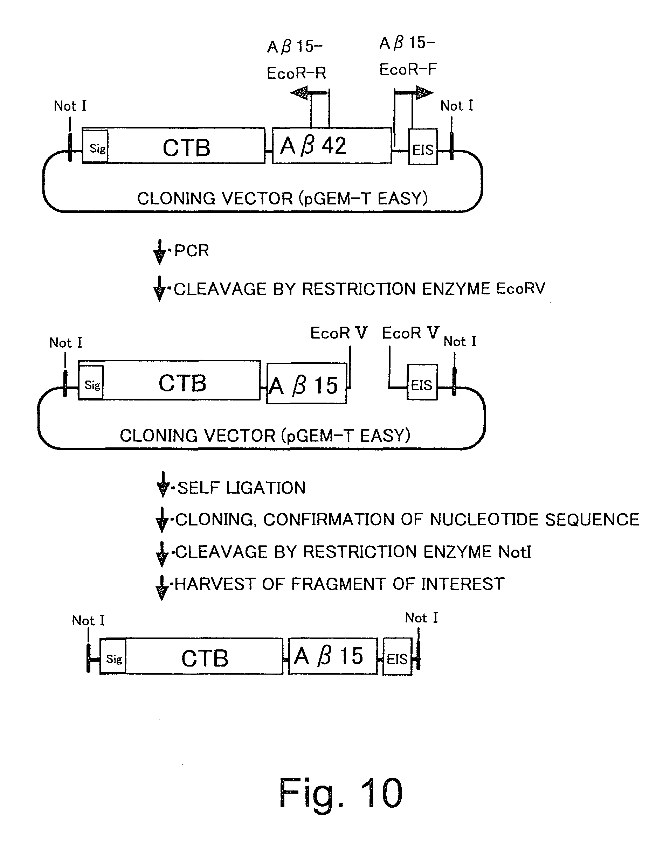

The CTB-Aβ15 gene *Not*I fragment was prepared based on the CTB-Aβ42 gene (Fig. 10).

Inverse PCR was carried out using a plasmid containing the CTB-Aβ42 *Not*I fragment as a template and two types of primers, Aβ15-EcoR-R (SEQ ID NO: 107) and Aβ15-EcoR-F (SEQ ID NO: 108), both of which have the addition of an *Eco*RV restriction site. The resulting PCR product was cleaved with restriction enzyme *Eco*RV. Then, the product was self ligated to prepare a plasmid containing the CTB-Aβ15 fragment. The plasmid was cleaved with restriction enzyme *Not*I to give the CTB-Aβ15 *Nnt*I fragment of interest (SEQ ID NO: 109).

### (2) Preparation of NotI fragments from a CTB-tandem Aβ15 gene (CTB-Aβ15x2, CTB-Aβ15x4, or CTB-Aβ15x8)

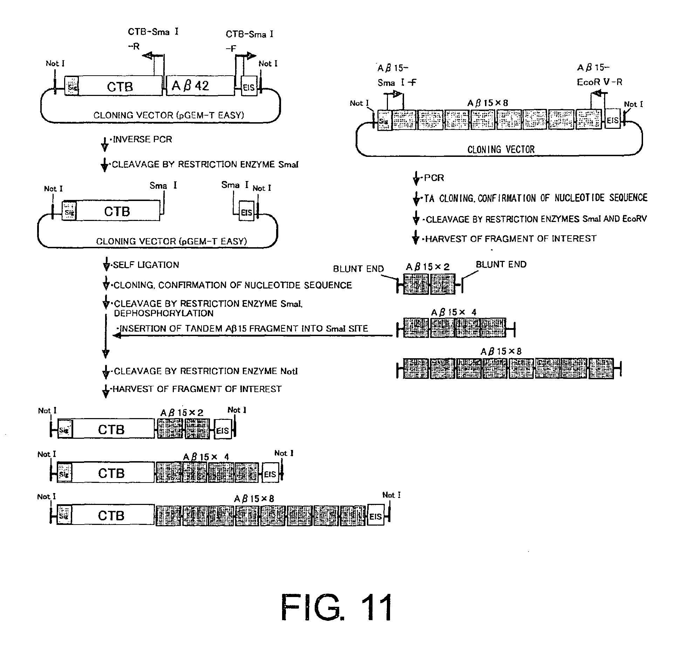

*Not*I fragments were prepared from a CTB-tandem Aβ15 gene using two types of genes (Fig. 11).

The method removed the Aβ42 region from the CTB-Aβ42-containing plasmid, and instead introduced a restriction enzyme site into the plasmid. A tandem Aβ15 fragment added with the restriction enzyme site was then inserted into that site of the plasmid by PCR.

Specifically, inverse PCR was carried out using as a template the plasmid containing the *Not*I fragment from CTB-Aβ42 (Example 10; SEQ ID NO: 69), and two types of primers CTB-SmaI-R (SEQ ID NO: 111) and CTB-SmaI-F (SEQ ID NO: 112), both of which have the addition of a *Sma*I restriction enzyme site. The resulting PCR product was cleaved with *Sma*I, and then self ligated to give an Aβ42-deficient plasmid. Then, the tandem Aβ15 fragment was inserted into the *Sma*I site of the plasmid.

The tandem Aβ15 fragment was prepared based on a plasmid containing a tandem eight Aβ15 *Not*I fragment (SEQ ID NO: 113) by the following method. PCR was carried out using the plasmid as a template as well as two types of primers Aβ15-SmaI-F (SEQ ID NO: 115) and Aβ15-EcoRV-R (SEQ ID NO: 116), both of which have the addition of a restriction enzyme site. The PCR yielded PCR products that were different in the number of Aβ15 repeats. The products were cloned by TA cloning. After the nucleotide sequences were confirmed, blunt-ended tandem Aβ15 fragments were excised from the clones with two types of restriction enzymes *Sma*I and *Eco*RI*.* The fragments were each inserted into the *Sma*I site of the Aβ42-deficient plasmid. The resulting plasmids were amplified and cleaved with restriction enzyme *Not*I to give fragments of interest: CTB-Aβ15x2 *Not*I fragment (SEQ ID NO: 117), CTB-Aβ15x4 *Not*I fragment (SEQ ID NO: 119), and CTB-Aβ15x8 *Not*I fragment (SEQ ID NO: 121).

### (3) Construction of Sendai virus cDNAs carrying CTB-Aβ15, CTB-Aβ15x2, CTB-Aβ15x4, or CTB-Aβ15x8 gene

The cDNA of an F gene-deficient SeV vector (WO 00/70070) (pSeV18+NotI/ΔF) was digested with *Not*I, and CTB-Aβ15, CTB-Aβ15x2, CTB-Aβ15x4, and CTB-Aβ15x8 fragments were each inserted into the *Not*I site to construct F gene-deficient SeV cDNAs carrying CTB-Aβ15, CTB-Aβ15x2, CTB-Aβ15x4, or CTB-Aβ15x8 (pSeV18+CTB-Aβ15/ΔF, pSeV18+CTB-Aβ15x2/ΔF, pSeV18+CTB-Aβ15x4/ΔF, and pSeV18+CTB-Aβ15x8/ΔF).

### [Example 22] Comparison of the ability to express Aβ peptide

### (1) Western blotting

The constructed vectors were assessed by Western blotting for the infectivity and expression.

Homogenates and culture supernatants of cells infected with SeV vectors were mixed with an equal volume of SDS-PAGE sample buffer, and heated for thermal denaturation at 98°C for five minutes. The mixtures were subjected to SDS-PAGE using 15% acrylamide gel, and then transferred onto PVDF membrane by the semi-dry blotting method. After blocking with 5% milk/TBS-T, the membrane was incubated with an anti-Aβ antibody (6E10) and then with an HRP-labeled anti-mouse IgG as the secondary antibody. Detection was carried out using chemiluminescent substrate SuperSignal West Femto with a CCD camera.

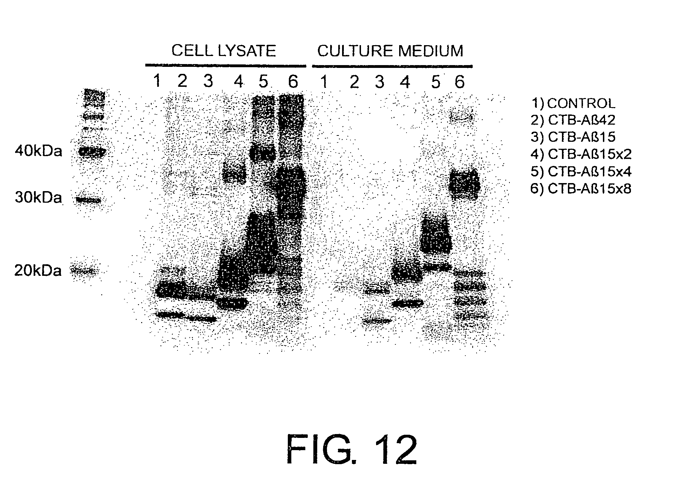

The result showed that CTB-Aβ42, CTB-Aβ15, CTB-Aβ15x2, CTB-Aβ115x4, and CTB-Aβ15x8 were expressed in the BHK cells and secreted to the media. It was also demonstrated that the expression level of CTB-Aβ15x8 was higher than that of CTB-Aβ42, and the quantity of CTB-Aβ15x8 secreted to the medium was considerably larger than that of CTB-Aβ42 (Fig. 12).

### (2) GM1-ELISA

The binding of CTB to GM1 was assessed using ganglioside GM1-immobilized plates.

Ganglioside GM1 (5 µg/ml) was immobilized onto each well of a 96-well plate (Nunc, MaxiSorp plate). After blocking with 20% BlockingOne (Nacalai Tesque), the culture supernatants of cells infected with the SeV vector were added (20- to 2,000,000-fold dilution) to the wells. Following incubation with the HRP-labeled antibody 6E10, detection was carried out using the TMB chromogenic reagent. The amount of binding was assessed by absorbance (O.D. 450) measurement using a plate reader.

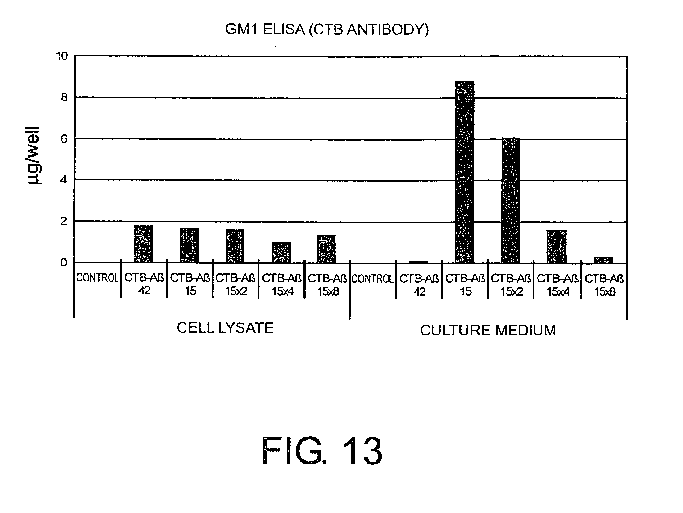

The result showed that CTB-Aβ42, CTB-Aβ15, CTB-Aβ15x2, CTB-Aβ15x4, and CTB-Aβ15x8 secreted to the media bound to GM1. The amount of CTB-Aβ15x8 binding was ten times that of CTB-Aβ42, while the amount of CTB-Aβ15 binding was 100 times that of CTB-Aβ15x8. This suggests that as the number of Aβ15 repeats increases, the GM1-binding ability is reduced (Fig. 13).

### [Example 23] Assessment of the variously constructed SeV vectors for their ability to induce anti-Aβ antibody in normal mice

### (1) Normal mice (comparison of intramuscularly administered CTB-Aβ42 and CTB-Aβ15x8)

SeV vectors carrying CTB-Aβ42, CTB-Aβ15x8, or GFP gene were each administered at a titer of 5 x 10⁷ CIU/head to C57BL/6N mice by intramuscular injection (right hind leg) to assess the antibody titer. 14 days after the treatment, blood was collected from the mice to determine the plasma level of anti-Aβ antibody. The Aβ1-42 peptide (5 µg/ml) was immobilized onto each well of a 96-well plate (Nunc, MaxiSorp plate). After blocking with 20% BlockingOne (Nacalai Tesque), mouse plasma (300- to 300,000-fold dilute) was added to the wells. Following incubation with a peroxidase-labeled mouse IgG antibody, detection was carried out using the TMB chromogenic reagent. The titer of the anti-Aβ antibodies was assessed by absorbance (O.D. 450) measurement using a plate reader. An anti-Aβ antibody (6E10) was used as the standard antibody.

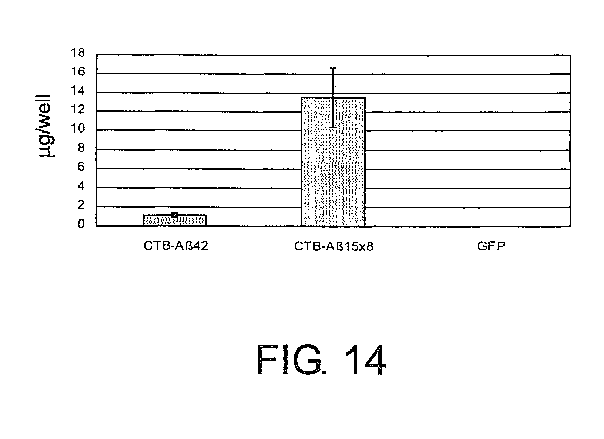

The result showed that the titer of the anti-Aβ antibodies was elevated in the CTB-Aβ42 gene administration group (n=6) and CTB-Aβ15x8 gene administration group (n=6), while it was not increased in the GFP gene administration group (n=6) as a control (Fig. 14). The antibody titer of the CTB-Aβ15x8 gene administration group was 12.23 times that of the CTB-Aβ42 gene administration group.

### (2) Normal mice (intramuscular, intracutaneous, and intranasal administration)

An SeV vector carrying the CTB-Aβ15x8 gene was administered at a titer of 5 x 10⁶ or 5 x 10⁷ CIU/head intranasally, intracutaneously, and intramuscularly (right hind leg) to C57BL/6N mice, while an SeV vector carrying the GFP gene was administered at a titer of 5 x 10⁷ CIU/head intramuscularly (right hind leg) as the control group to assess the antibody titer. 14 days after the treatment, blood was collected from the mice to determine the plasma level of anti-Aβ antibody.

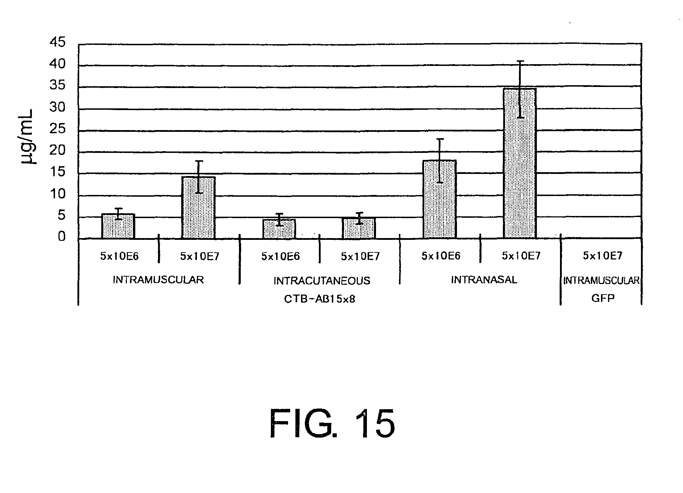

The result showed that the titer of anti-Aβ antibody was elevated in all administration groups except the control group. The antibody titer was lower in the intracutaneous administration group than in the other administration groups. The antibody titer was higher in the intranasal administration group when compared to the intramuscular administration group of the same titer (Fig. 15).

### (3) Normal mice (intranasal administration)

SeV vectors carrying CTB-Aβ15, CTB-Aβ15x2, CTB-Aβ15x4, or CTB-Aβ15x8, and an SeV vector carrying the GFP gene as the control were intranasally administered at a titer of 5 x 10⁷ CIU/head to C57BL/6N mice to assess the antibody titer.

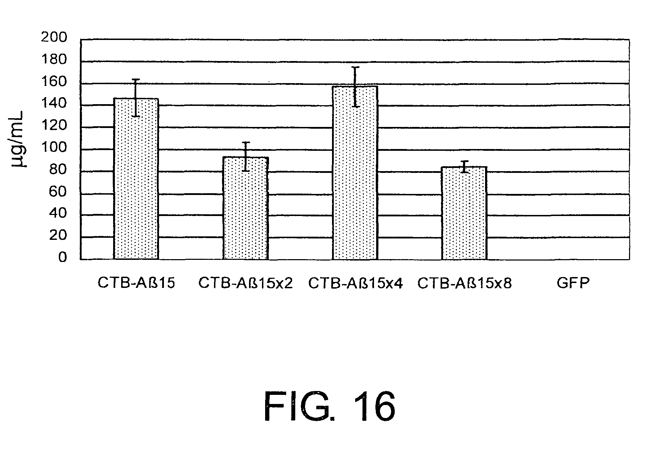

The result showed that the antibody titer was significantly elevated in all administration groups as compared to the control group. In particular, the titer of anti-Aβ antibody was higher in the CTB-Aβ15 and CTB-Aβ15x4 administration groups as compared to the CTB-Aβ 15x8 administration group (Fig. 16).

### [Example 24] Assessment of the variously constructed SeV vectors for booster effects on the induction of anti-Aβ antibody in normal mice

### (1) Normal mice (intramuscular administration): booster immunization with purified CTB-Aβ42 protein

An SeV vector carrying CTB-Aβ42 gene was administered at a titer of 5 x 10⁷ CIU/head intramuscularly (right hind leg) to C57BL/6N mice. After 14 and 28 days, the CTB-Aβ42 protein produced in *E. coli* was intramuscularly (right hind leg) administered at 20 µg/PBS/head, 100 µg/PBS/head, or 100 µg/IFA (Freund's incomplete adjuvant)/head to assess the antibody titer. Every 14 days after the treatment, blood was collected from the mice to determine the plasma level of anti-Aβ antibody.

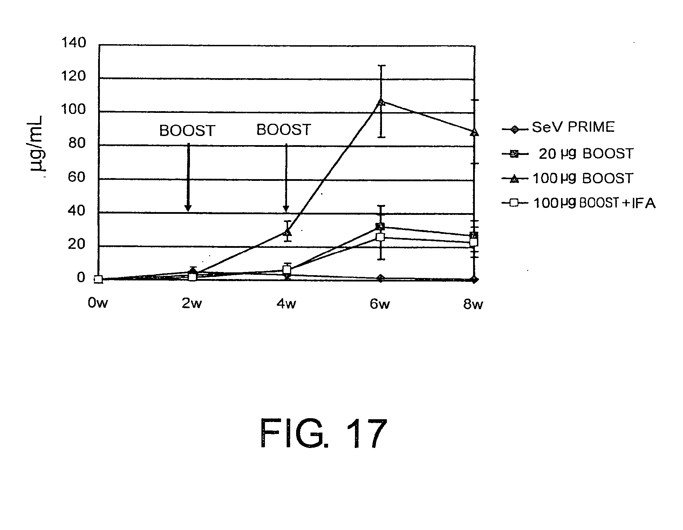

The result showed that the anti-Aβ antibody level was significantly increased in the group immunized with the CTB-Aβ42 gene and booster immunized with the CTB-Aβ42 protein (Fig. 17). After two rounds of booster immunization, the titer of anti-Aβ antibody was 32 µg/ml in the 20-µg booster group, 107 µg/m) in the 100-µg booster group, and 25.9 µg/ml in the [100 µg + IFA] booster group.

### (2) Normal mice (intramuscular administration): booster immunization with SeV vector [1]

SeV vectors carrying the CTB-Aβ42 or CTB-Aβ15x8 gene were administered at a titer of 5 x 10⁷ CIU/head intramuscularly (right hind leg) to C57BL/6N mice, and after 56 days the same SeV vectors were administered intramuscularly (right hind leg) at the same titer to assess the antibody titer.

14 and 28 days after the treatment, blood was collected from the mice to determine the plasma level of anti-Aβ antibody.

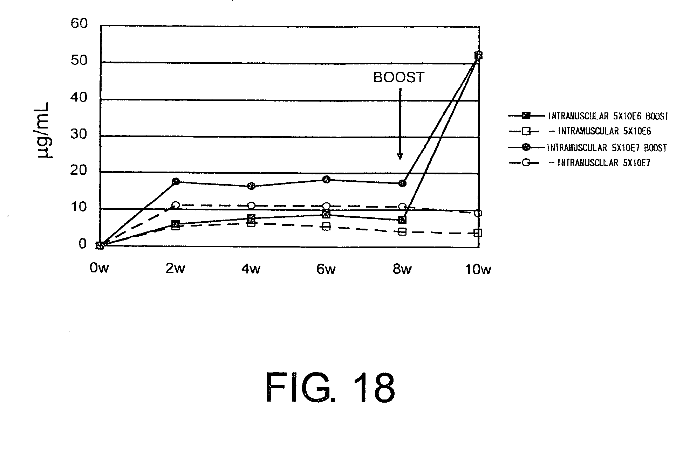

The result showed that the titer of anti-Aβ antibody was significantly elevated in the CTB-Aβ15x8 gene booster group (Fig. 18). Meanwhile, in the CTB-Aβ42 gene booster group, the increase in the anti-Aβ antibody titer was smaller than that of the CTB-Aβ15x8 gene booster group.

### (3) Normal mice (intramuscular administration): booster immunization with SeV vectors [2]

SeV vectors carrying the CTB-Aβ15x8 or CTB-Aβ42 gene were administered at a titer of 5 x 10⁶ or 5 x 10⁷ CIU/head intramuscularly (right hind leg) to C57BL/6N mice, and after 56 days the same SeV vectors were administered intramuscularly (right hind leg) at the same titer to assess the antibody titer.

14 and 28 days after the treatment, blood was collected from the mice to determine the plasma level of anti-Aβ antibody.

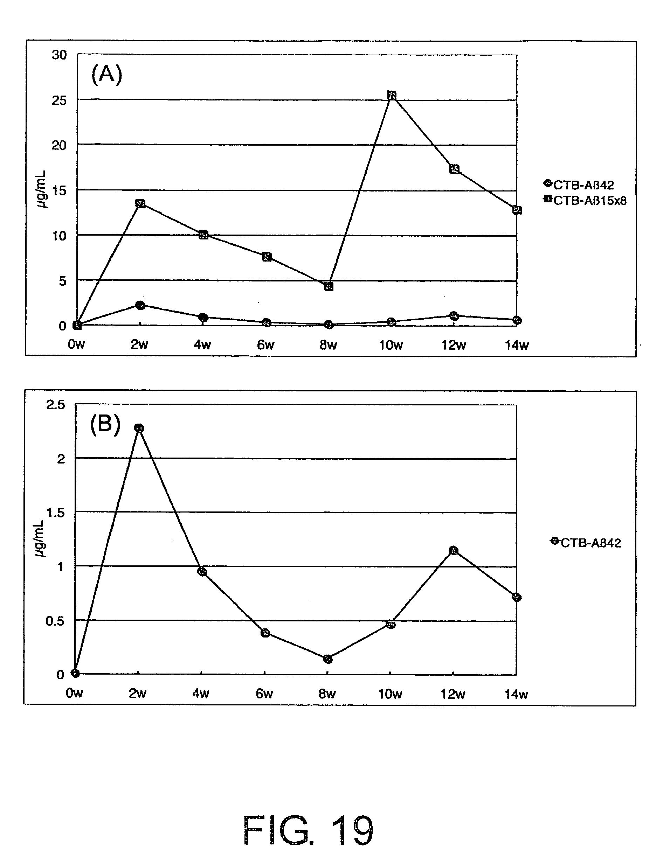

The result showed that clearly both vectors produced the booster effect. In particular, the increase in the anti-Aβ antibody titer was more significant in the CTB-Aβ15x8 gene booster group (Fig. 19).

### (4) Normal mice (intranasal administration): booster immunization with SeV vector

An SeV vector carrying the CTB-Aβ15x8 gene was administered at a titer of 5 x 10⁶ or 5x 10⁷ CIU/head intranasally to C57BL/6N mice, and after 56 days the same SeV vector was administered intranasally at the same titer to assess the antibody titer.

14 and 28 days after the treatment, blood was collected from the mice to determine the plasma level of anti-Aβ antibody.

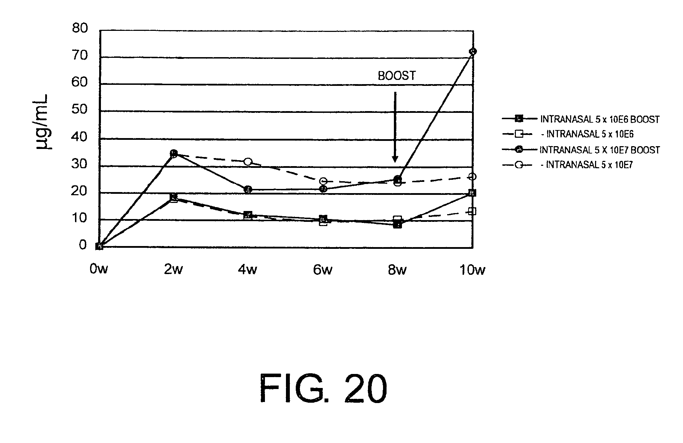

The result showed that the titer of anti-Aβ antibody was significantly elevated in the CTB-Aβ15x8 gene booster group at the rate of 3/3 (Fig. 20).

### [Example 25] Assessment of the constructed various SeV vectors for the effectiveness in APP model mice: intramuscular administration

### (1) Titer of anti-Aβ antibody

SeV 18+CTB-Aβ18x5/ΔF (also referred to as CTB-Aβ15x8) or SeV18+CTB-Aβ42/ΔF (also referred to as CTB-Aβ42), or an SeV vector carrying the GFP gene (SeV 18+GFP/ΔF; hereinafter also referred to as "GFP") as control was administered at 5 x 10⁷ CIU/head intramuscularly (right hind leg) to APP transgenic mice (Tg2576) (13 months old), which served as Alzheimer's disease model mice. After 14 and 28 days, the CTB-Aβ42 protein produced in *E. coli* was administered intramuscularly (right hind leg) to half of the mice in the CTB-Aβ42 gene administration group. 14, 28, 42, and 56 days after SeV vector administration, assay was carried out to determine the plasma titer of anti-Aβ antibody.

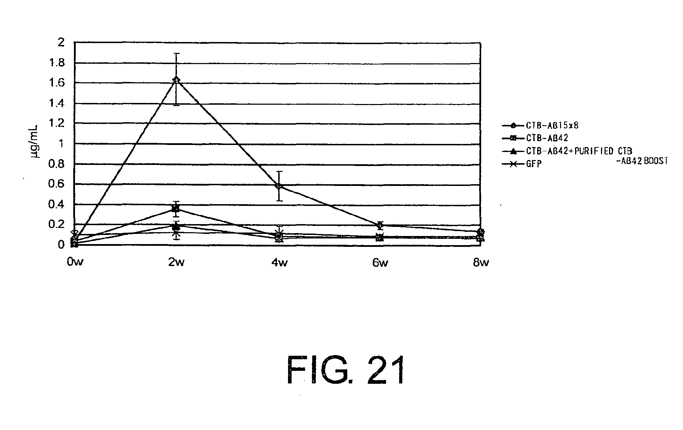

The result showed that the titer of anti-Aβ antibody was significantly elevated in the CTB-Aβ15x8 gene administration group. Meanwhile, in the CTB-Aβ42 gene administration group, the titer of anti-Aβ antibody was only negligibly elevated in half of the mice. The increase in the titer of anti-Aβ antibody was small relative to the CTB-Aβ15x8 gene administration group. The booster immunization did not increase the titer of anti-Aβ antibody in the CTB-Aβ42 protein booster group (Fig. 21).

### (2) Intracerebral Aβ level: ELISA

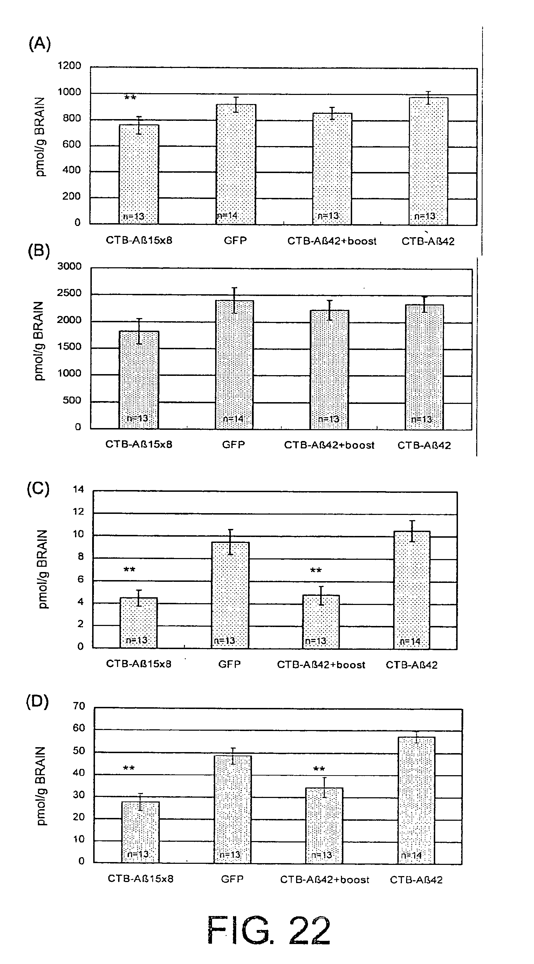

Brain tissues were harvested from the above-described APP mice 56 days after the start of SeV vector administration, and the level of Aβ in the tissue of the left brain hemisphere was determined by ELISA. The brain tissues were homogenized in TBS by sonication. After one hour of centrifugation at 35,000 g, the supernatants were collected as soluble Aβ fractions. Meanwhile, the precipitates were homogenized in 10% formic acid by sonication, and then neutralized with 1 M Tris. The resulting samples were saved as insoluble Aβ fractions. The intracerebral Aβ level was determined using the Aβ42 ELISA kit and Aβ40 ELISA kit (both from Wako Pure Chemical Industries). The result showed that in the CTB-Aβ15x8 gene administration group the Aβ level in the insoluble fraction was reduced to about 80% as compared to the GFP gene administration group. The Aβ level was not reduced in the CTB-Aβ42 gene administration group. Meanwhile, the Aβ level was only slightly reduced in the CTB-Aβ42 protein booster group. In the CTB-Aβ15x8 gene administration group, the Aβ level in the soluble fraction was reduced to about 50% as compared to the GFP gene administration group. The Aβ level was not reduced in the CTB-Aβ42 gene administration group. In the CTB-Aβ42 protein booster group, the Aβ level was reduced to 60% to 70% (Fig. 22).

### (3) Effect of SeV18+CTB-Aβ15x8/ΔF in eliminating senile plaques

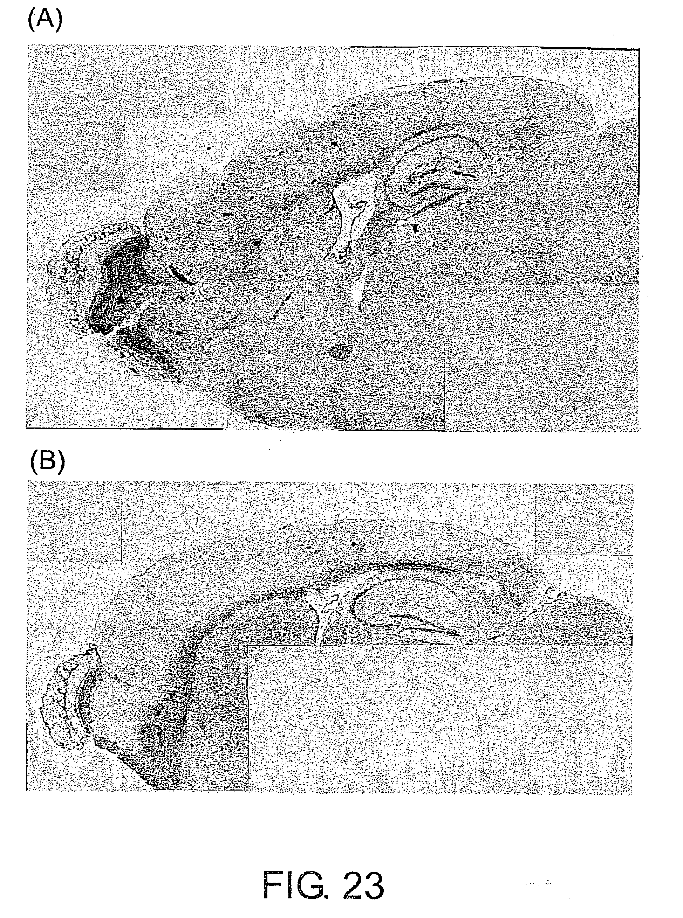

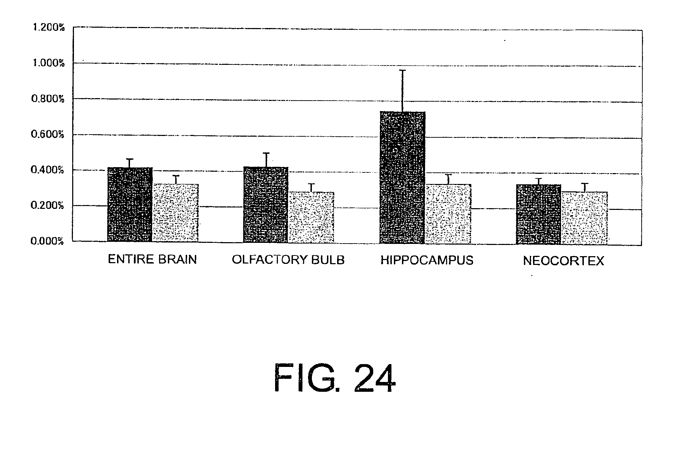

Sendai virus vectors were intramuscularly administered to mice. The mice in each group were dissected eight weeks after administration (15 months old). For histopathological testing, the right brain hemispheres were fixed by immersion in a 10% neutral buffered formalin solution, and after paraffin embedding, longitudinal sections were prepared from the brain tissue located about 2 mm distant from fissura mediana of the brain. The tissue sections were treated with 70% formic acid to detect Aβ protein and senile plaques. The endogenous peroxidase activity was inactivated by 5% H₂O₂. After incubation with an anti-Aβ antibody (antibody 6E10; 1,000-fold dilution), a peroxidase-labeled secondary antibody was added for color development with DAB. Then, images were obtained using a 3CCD camera attached to a microscope. For each sample, 20 to 30 image files were combined (Fig. 23). Using image analysis software NIH image, the area of Aβ accumulation in each region of olfactory bulb, cerebral neocortex, and hippocampus was measured under the same conditions for all samples to calculate the ratio of Aβ-accumulated area in each of the regions tested. In addition, the numbers of senile plaques used in the measurement were compared. As seen in Fig. 24, the result showed that the percent area of senile plaques tended to decrease, especially in the hippocampus.

### (4) Safety assessment of SeV18+CTB-Aβ15x8/ΔF administration

In the same manner as described in (3), samples of HE- and anti-Iba-1 antibody (microglia)-stained paraffin sections derived from the treatment groups and control group eight weeks after administration (15 months old) were obtained, and they were assessed for the infiltration of inflammatory cells and microglial activation in the central nervous system. The result showed that there was no detectable infiltrating inflammatory cell in any region of the brain both in the control and treatment groups. This demonstrates that the vector of the present invention does not induce inflammation in the central nervous system.

Microglial activation was detected around senile plaques in the animals of both groups. However, in the animals of the vector administration group, the number of senile plaques tended to decrease, and in parallel, the proportion of area occupied with microglias also tended to decrease.

### Industrial Applicability

The present invention enables one to increase the expression level of a recombinant protein from RNA viral vectors. Effective gene therapy, gene vaccination, monoclonal antibody preparation, or such can be achieved by using the gene transfer RNA viral vectors of the present invention.

## Claims

1. A minus-strand RNA viral vector encoding a recombinant protein, wherein the protein is encoded as a fusion protein with an AB5 toxin B subunit.

2. A method for producing a minus-strand RNA viral vector with enhanced capacity for expressing a recombinant protein, which comprises the step of producing an RNA viral vector encoding a fusion protein in which the recombinant protein is fused with an AB5 toxin B subunit.

3. A non-therapeutic method for increasing the expression level of a recombinant protein from a minus-strand RNA viral vector, which comprises the step of expressing the recombinant protein as a fusion protein with an AB5 toxin B subunit from an RNA viral vector.

4. The vector of claim 1 or the method of claim 2 or 3, wherein the AB5 toxin B subunit is cholera toxin B (CTB).

5. The vector of claim 1 or 4 or the method of any one of claims 2 to 4, wherein the recombinant protein is a peptide of 120 amino acids or less.

6. The vector of claim 5 or the method of claim 5, wherein the recombinant protein is a peptide of 50 amino acids or less.

7. The vector of any one of claims 1 and 4 to 6 or the method of any one of claims 2 to 6, wherein the recombinant protein is a neuropeptide or endocrine peptide.

8. The vector of any one of claims 1 and 4 to 7 or the method of any one of claims 2 to 7, wherein the recombinant protein is a protein of low solubility.

9. A pharmaceutical composition comprising the vector of any one of claims 1 and 4 to 8 and a pharmaceutically acceptable carrier.

10. An isolated cell introduced with the vector of any one of claims 1 and 4 to 8.

11. A method for producing a recombinant protein, which comprises the step of expressing the vector of any one of claims 1 and 4 to 8.

12. A virus-like particle comprising the vector of any one of claims 1 and 4 to 8.

## Patentansprüche

1. Viraler Minus-Strang-RNA-Vektor, der ein rekombinantes Protein codiert, wobei das Protein als Fusionsprotein mit einer AB5-Toxin-B-Untereinheit codiert wird.

2. Verfahren zur Herstellung eines viralen Minus-Strang-RNA-Vektors mit verstärkter Fähigkeit, ein rekombinantes Protein zu exprimieren, das den Schritt der Herstellung eines viralen RNA-Vektors umfasst, der ein Fusionsprotein codiert, in dem das rekombinante Protein mit einer AB5-Toxin-B-Untereinheit fusioniert ist.

3. Nicht-therapeutisches Verfahren zur Erhöhung des Expressionsspiegels eines rekombinanten Proteins von einem viralen Minus-Strang-RNA-Vektor, das den Schritt der Expression des rekombinanten Proteins als ein Fusionsprotein mit einer AB5-Toxin-B-Untereinheit von einem viralen RNA-Vektor umfasst.

4. Vektor nach Anspruch 1 oder Verfahren nach Anspruch 2 oder 3, wobei die AB5-Toxin-B-Untereinheit Cholera-Toxin-B (CTB) ist.

5. Vektor nach Anspruch 1 oder 4 oder Verfahren nach einem der Ansprüche 2 bis 4, wobei das rekombinante Protein ein Peptid mit 120 oder weniger Aminosäuren ist.

6. Vektor nach Anspruch 5 oder Verfahren nach Anspruch 5, wobei das rekombinante Protein ein Peptid mit 50 oder weniger Aminosäuren ist.

7. Vektor nach einem der Ansprüche 1 und 4 bis 6 oder Verfahren nach einem der Ansprüche 2 bis 6, wobei das rekombinante Protein ein Neuropeptid oder endokrines Peptid ist.

8. Vektor nach einem der Ansprüche 1 und 4 bis 7 oder Verfahren nach einem der Ansprüche 2 bis 7, wobei das rekombinante Protein ein Protein mit geringer Löslichkeit ist.

9. Pharmazeutische Zusammensetzung, die den Vektor nach einem der Ansprüche 1 und 4 bis 8 und einen pharmazeutisch verträglichen Träger umfasst.

10. Isolierte Zelle, in die der Vektor nach einem der Ansprüche 1 und 4 bis 8 eingeführt wurde.

11. Verfahren zur Herstellung eines rekombinanten Proteins, das den Schritt der Expression des Vektors nach einem der Ansprüche 1 und 4 bis 8 umfasst.

12. Virus-ähnliches Partikel, das den Vektor nach einem der Ansprüche 1 und 4 bis 8 umfasst.

## Revendications

1. Vecteur viral à ARN à brin négatif qui code pour une protéine recombinée, dans lequel la protéine est codée en tant que protéine hybride avec une unité secondaire AB5 de la toxine B.

2. Procédé destiné à produire un vecteur viral à ARN à brin négatif qui présente une capacité améliorée destinée à exprimer une protéine recombinée, qui comprend une étape consistant à produire un vecteur viral à ARN qui code pour une protéine hybride dans lequel la protéine recombinée est fusionnée avec une unité secondaire AB5 de la toxine B.

3. Procédé non thérapeutique destiné à améliorer le niveau d'expression d'une protéine recombinée à partir d'un vecteur viral à ARN à brin négatif, qui comprend une étape consistant à exprimer la protéine recombinée en tant que protéine hybride avec une unité secondaire AB5 de la toxine B à partir d'un vecteur viral à ARN.

4. Vecteur selon la revendication 1 ou procédé selon la revendication 2 ou la revendication 3, dans lequel l'unité secondaire AB5 de la toxine B est la toxine B du choléra (CTB).

5. Vecteur selon la revendication 1 ou la revendication 4 ou procédé selon l'une quelconque des revendications 2 à 4, dans lequel la protéine recombinée est un peptide qui comprend 120 acides aminés ou moins.

6. Vecteur selon la revendication 5 ou procédé selon la revendication 5, dans lequel la protéine recombinée est un peptide qui comprend 50 acides aminés ou moins.

7. Vecteur selon l'une quelconque des revendications 1 et 4 à 6 ou procédé selon l'une quelconque des revendications 2 à 6, dans lequel la protéine recombinée est un neuropeptide ou un peptide endocrinien.

8. Vecteur selon l'une quelconque des revendications 1 et 4 à 7 ou procédé selon l'une quelconque des revendications 2 à 7, dans lequel la protéine recombinée est une protéine qui présente une faible solubilité.

9. Composition pharmaceutique qui comprend le vecteur selon l'une quelconque des revendications 1 et 4 à 8, et un véhicule qui peut être accepté de manière pharmaceutique.

10. Cellule isolée introduite avec le vecteur selon l'une quelconque des revendications 1 et 4 à 8.

11. Procédé destiné à produire une protéine recombinée, qui comprend une étape consistant à exprimer le vecteur selon l'une quelconque des revendications 1 et 4 à 8.

12. Particule similaire à un virus qui comprend le vecteur selon l'une quelconque des revendications 1 et 4 à 8.
